# EUROPEAN PATENT SPECIFICATION

(11) **EP 3 061 759 B1**
(45) Date of publication and mention of the grant of the patent: **25.12.2019**
(21) Application number: 15156360.8
(22) Date of filing: 24.02.2015
(51) Int. Cl.: C07D 405/14, C07D 405/04, H01L 51/50

(54) **NITRILE SUBSTITUTED DIBENZOFURANS**
NITRILSUBSTITUIERTE DIBENZOFURANE
DIBENZOFURANES À SUBSTITUANT NITRILE

(43) Date of publication of application: 31.08.2016
(73) Proprietor: Idemitsu Kosan Co., Ltd, Tokyo 100-8321 (JP)
(72) Inventor: WOLLEB, Heinz, 4232 Fehren (CH); NISHIMAE, Yuichi, 4058 Basel (CH); NAGASHIMA, Hideaki, 4057 Basel (CH)
(74) Representative: Hollah, Dorothee

(56) References cited:
- WO-A1-2012/096241

## Description

The present invention relates to compounds of formula I, a process for their production and their use in electronic devices, especially electroluminescent devices. When used as charge transport material and/or host material for phosphorescent emitters in electroluminescent devices, the compounds of formula I may provide improved efficiency and reduced driving voltage of electroluminescent devices.

JP2001043979 relates to a hole transporting material of formula 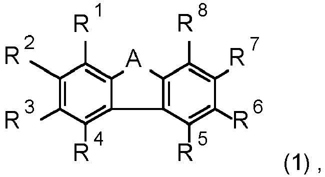 wherein adjacent substitutes may form optionally substituted aliphatic ring, optionally substituted carbon cyclic aromatic ring, optionally substituted heterocyclic aromatic ring, or optionally substituted heterocycle; A is O, S, SO₂, Se, Te, C=C(CN)₂, C=O, C=S, C=Se, or C=Te;
R¹-R⁸ = H, halo, cyano, nitro, amino, ester, mono- or di-substitued amino, acylamino, hydroxyl, alkoxy, mercapto, alkyloxy, alkylthio, aryloxy, arylthio, siloxy, acyl, cycloalkyl, carbamoyl, carboxylic acid, sulfonic acid, optionally substituted aliphatic group, optionally substituted aliphatic cyclic group, optionally substituted carbon cyclic aromatic ring, optionally substituted heterocyclic aromatic ring, optionally substituted heterocycle; at least one of R¹-R⁴ and at least one of R⁵-R⁸ = amino, mono-substituted amino, or di-substituted amino. The following compound is explicitly mentioned: 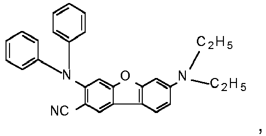 for which no synthesis method is described.

JP2012049518 relates to a material for an organic electroluminescent element, which is a compound represented by general formula 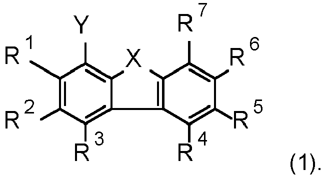 R¹ to R⁷ may among others represent a cyano group. The following compound is explicitly mentioned: 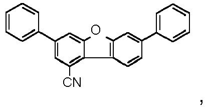 for which no synthesis method is described. US20140001446 relates to a material for an organic electroluminescence device represented by formula 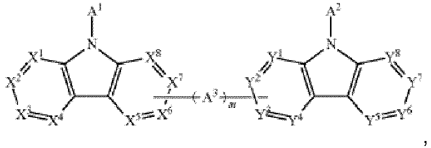 wherein: each of A¹ and A² independently represents a substituted or unsubstituted aromatic hydrocarbon group having 6 to 30 ring carbon atoms or a substituted or unsubstituted heterocyclic group having 5 to 30 ring atoms; A³ represents a substituted or unsubstituted monocyclic hydrocarbon group having 6 or less ring carbon atoms or a substituted or unsubstituted monocyclic heterocyclic group having 6 or less ring atoms; m represents an integer of 0 to 3; each of X¹ to X⁸ and Y¹ to Y⁸ independently represents N or CR^{a}; each of R^{a} independently represents a hydrogen atom, a substituted or unsubstituted aromatic hydrocarbon group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted silyl group, or a halogen atom, provided that one of X⁵ to X⁸ and one of Y¹ to Y⁴ are bonded to each other via A³ or directly; and
the formula (I) satisfies at least one of the following requirements (i) and (ii):
(i) at least one of A¹ and A² represents a cyano-substituted aromatic hydrocarbon group having 6 to 30 ring carbon atoms or a cyano-substituted heterocyclic group having 5 to 30 ring atoms; and
(ii) at least one of X¹ to X⁴ and Y⁵ to Y⁸ represents CR^{a}, and at least one of R^{a} in X¹ to X⁴ and Y⁵ to Y⁸ represents a cyano-substituted aromatic hydrocarbon group having 6 to 30 ring carbon atoms or a cyano-substituted heterocyclic group having 5 to 30 ring atoms, provided that when two or more R^{a} groups exist, the R^{a} groups may be the same or different. The following compound is explicitly mentioned:

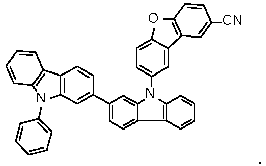

US20130062597 relates to nitrogen-containing heteroaromatic ring compound of formula 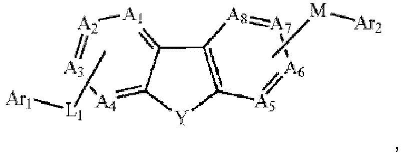
wherein A₁ to A₈ are independently CR₁, R₁ is independently a hydrogen atom, a substituted or unsubstituted alkyl group having 1 to 20 carbon atoms, a substituted or unsubstituted cycloalkyl group having 3 to 20 ring carbon atoms, a substituted or unsubstituted alkoxy group having 1 to 20 carbon atoms, a substituted or unsubstituted cycloalkoxy group having 3 to 20 ring carbon atoms, a substituted or unsubstituted aromatic hydrocarbon group having 6 to 18 ring carbon atoms, a substituted or unsubstituted aryloxy group having 6 to 18 ring carbon atoms, a substituted or unsubstituted heteroaromatic group having 5 to 18 ring atoms, a substituted or unsubstituted amino group, a substituted or unsubstituted silyl group, a fluorine atom, a substituted or unsubstituted fluoroalkyl group, or a cyano group, Y is an oxygen atom or a sulfur atom, Ar₁ is a hydrogen atom, a substituted or unsubstituted aromatic hydrocarbon group having 6 to 18 ring carbon atoms, or a substituted or unsubstituted heteroaromatic group having 5 to 18 ring atoms,
L₁ is a single bond, a substituted or unsubstituted aromatic hydrocarbon group having 6 to 18 ring carbon atoms, or a substituted or unsubstituted heteroaromatic group having 5 to 18 ring atoms,
M is a substituted or unsubstituted nitrogen-containing heteroaromatic group, and Ar₂ is a substituted aromatic hydrocarbon group having 6 to 18 ring carbon atoms, a substituted or unsubstituted monocyclic heteroaromatic group having 5 or 6 ring atoms, a dibenzofuran ring group that may be substituted with a substituent (excluding a 3-carbazolyl group and an N-carbazolyl group), a dibenzothiophene ring group that may be substituted with a substituent (excluding a 3-carbazolyl group and an N-carbazolyl group), or a nitrogen-containing polycyclic group among nitrogen-containing polycyclic groups respectively represented by formulas (1) to (5). M is preferably a substituted or unsubstituted 5-membered or 6-membered monocyclic nitrogen-containing heteroaromatic group. The following compound is explicitly mentioned:

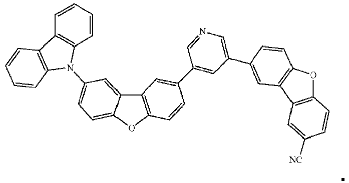

WO2013039073 relates to aromatic amine derivatives of formula 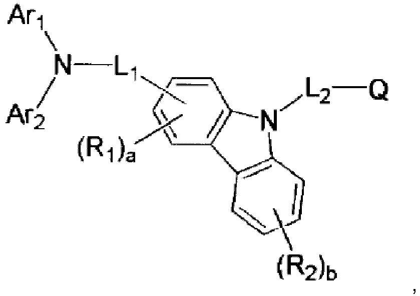 its use as hole-transport material in organic electroluminescent elements. The following compound is explicitly mentioned:

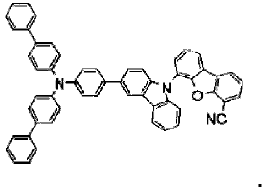

WO2013145923 relates to organic electroluminescent devices which comprise a light emitting layer which is disposed between a cathode and an anode and comprises a first host material, a second host material and a light emitting material, where a specific biscarbazole derivative having a cyano group, 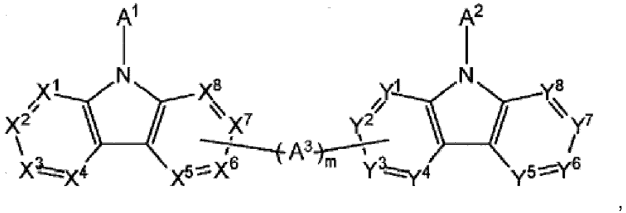 such as, for example, 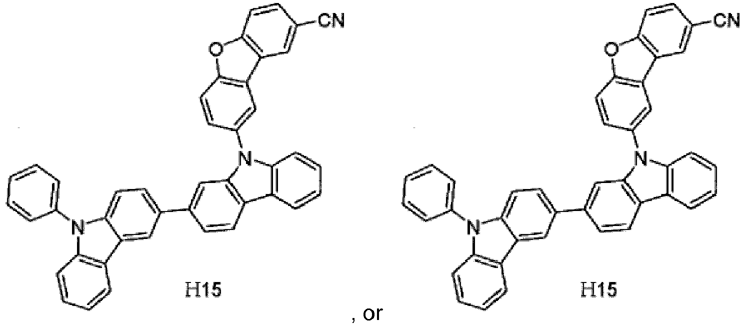 , serves as a first host and a compound having both a carbazole structure and a nitrogen-containing aromatic hetero-ring serves as a second host.

Journal of Materials Chemistry C: Materials for Optical and Electronic Devices 48 (2013) 8140-8145 relates to the synthesis of 6-(3-(9 H-carbazol-9-yl)phenyl)-9-ethyl-9 H-carbazole-3-carbonitrile (m-CzCzCN), 6-(2-(9 H-carbazol-9-yl)phenyl)-9-ethyl-9 H-carbazole-3-carbonitrile (o-CzCzCN), 6-(3,5-di(9 H-carbazol-9-yl)phenyl)-9-ethyl-9 H-carbazole-3-carbonitrile (mCPCzCN), 8-(3-(9 H-carbazol-9-yl)phenyl)dibenzo[b,d]furan-2-carbonitrile (m-CzOCN), 8-(3-(9 H-carbazol-9-yl)phenyl)dibenzo[b,d]thiophene-2- carbonitrile (m-CzSCN) and its use as host materials in blue phosphorescent organic light-emitting diodes (PhOLEDs). These materials are reported to exhibit an excellent performance in their hosted blue phosphorescent OLEDs with iridium(iii)bis(4,6- (difluorophenyl)pyridinato-N,C2')picolinate (Firpic) as the doped emitter.

WO2014013721 discloses nitrogen-containing heteroaromatic ring compounds of formula 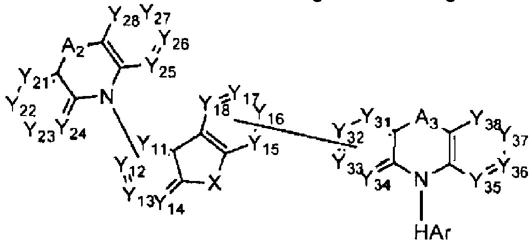 and its use in an organic light emitting element. The following compound is explicitly mentioned:

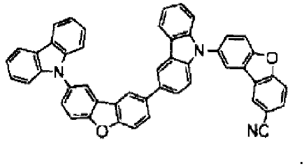

US2013285035 discloses an organic electroluminescent element comprising an anode, a cathode, and an organic compound layer sandwiched by the anode and the cathode, wherein the organic compound layer at least comprises a light-emitting layer and a charge-generating layer; (1) the charge-generating layer is composed of at least one layer and provided between the anode and the light-emitting layer; and (2) at least one layer of the charge-generating layer comprises an organic metal complex. The compound of formula 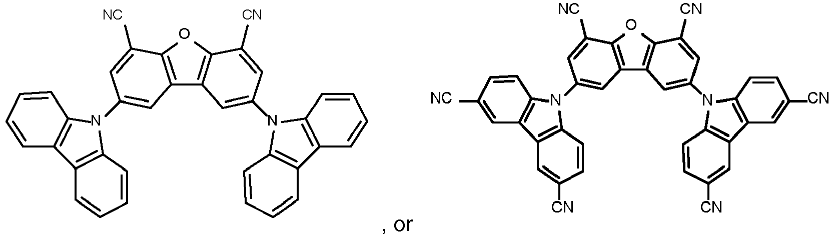 is used as an electron extracting material in the charge generating layer.

KR20140132244 relates to an organic electronic device containing a heterocyclic compound of formula 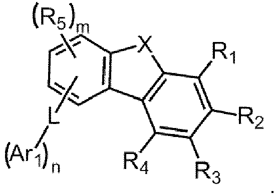 The substituent R₃ represents CN. The substituents R₁, R₂, R₄ and R₅ may represent CN.

Notwithstanding these developments, there remains a need for organic light emitting devices comprising new electron transport materials to provide improved efficiency, stability, manufacturability, and/or spectral characteristics of electroluminescent devices.

Accordingly, it is an object of the present invention, with respect to the aforementioned prior art, to provide further materials suitable for use in OLEDs and further applications in organic electronics. More particularly, it should be possible to provide electron transport materials, hole/exciton blocker materials and matrix materials for use in OLEDs. The materials should be suitable especially for OLEDs which comprise at least one phosphorescence emitter, especially at least one green emitter or at least one blue emitter. Furthermore, the materials should be suitable for providing OLEDs which ensure good efficiencies, good operative lifetimes and a high stability to thermal stress, and a low use and operating voltage of the OLEDs.

Certain dibenzofurans and dibenzothiophenes are found to be suitable for use in organo-electroluminescent devices. In particular, said derivatives are suitable electron transporting materials, hole blocking materials or host materials for phosphorescent emitters with good efficiency and durability.

Accordingly the present invention relates to compounds of formula 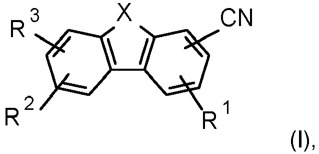 wherein
X is O, or S,
R¹ is H, Si(R²⁴)(R²⁵)(R²⁶), P(O)(R²⁷)(R²⁸), a C₁-C₂₅alkyl group, which can optionally be substituted by E, and or interrupted by D, a C₂-C₂₅alkenyl group, a C₂-C₂₅alkynyl group, -OR³², -SR³³, -COR³⁴, -COOR³⁵, a C₃-C₁₈cycloalkyl group, which can optionally be substituted by G; a group of formula 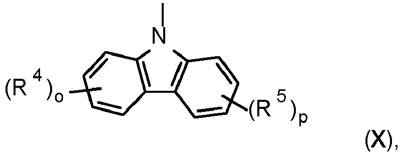 or 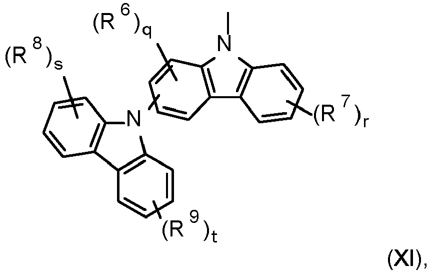
R² is H, Si(R²⁴)(R²⁵)(R²⁶), P(O)(R²⁷)(R²⁸), a C₁-C₂₅alkyl group, which can optionally be substituted by E, and or interrupted by D, a C₂-C₂₅alkenyl group, a C₂-C₂₅alkynyl group, -OR³², -SR³³, -COR³⁴, -COOR³⁵, a C₃-C₁₈cycloalkyl group, which can optionally be substituted by G; or a group of formula (**X**), or (**XI**),
R³ is H, Si(R²⁴)(R²⁵)(R²⁶), P(O)(R²⁷)(R²⁸), a C₁-C₂₅alkyl group, which can optionally be substituted by E, and or interrupted by D, a C₂-C₂₅alkenyl group, a C₂-C₂₅alkynyl group, -OR³², -SR³³, -COR³⁴, -COOR³⁵, a C₃-C₁₈cycloalkyl group, which can optionally be substituted by G; or a group of formula (**X**), or (**XI**),
o, p, r, s and t are independently of each other 0, or an integer 1 to 4,
q is 0, or an integer 1 to 3,
R⁴ and R⁵ may be the same or different in each occurence and are independently of each other H, CN, Si(R²⁴)(R²⁵)(R²⁶), P(O)(R²⁷)(R²⁸), N(R³⁰)(R³¹), a C₁-C₂₅alkyl group, which can optionally be substituted by E, and or interrupted by D, a C₂-C₂₅alkenyl group, a C₂-C₂₅alkynyl group, -OR³², -SR³³, -COR³⁴, -COOR³⁵, a C₃-C₁₈cycloalkyl group, or a group of formula 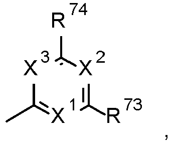 wherein
X¹, X² and X³ are independently of each other N, or CH,
R⁶, R⁷, R⁸ and R⁹ may be the same or different in each occurence and are independently of each other H, CN, Si(R²⁴)(R²⁵)(R²⁶), P(O)(R²⁷)(R²⁸), N(R³⁰)(R³¹), a C₁-C₂₅alkyl group, which can optionally be substituted by E, and or interrupted by D, a C₂-C₂₅alkenyl group, a C₂-C₂₅alkynyl group, -OR³², -SR³³, -COR³⁴, -COOR³⁵, a C₃-C₁₈cycloalkyl group, which can optionally be substituted by G; a C₆-C₂₄aryl group, which can optionally be substituted by G, or a C₂-C₃₀heteroaryl group, which can optionally be substituted by G;
R²⁴, R²⁵, R²⁶, R²⁷ and R²⁸ are independently of each other a C₁-C₂₅alkyl group, a C₆-C₂₄aryl group, which can optionally be substituted by G, or a C₂-C₃₀heteroaryl group, which can optionally be substituted by G;
R³⁰ and R³¹ are independently of each other a C₆-C₂₄aryl group, which can optionally be substituted by G, or a C₂-C₃₀heteroaryl group, which can optionally be substituted by G;
R³² and R³³ are independently of each other a C₆-C₁₈aryl group; a C₆-C₁₈aryl group, which is substituted by C₁-C₁₈alkyl group, or C₁-C₁₈alkoxy group; a C₁-C₁₈alkyl group; or a C₁-C₁₈alkyl group, which is interrupted by -O-;
R³⁴ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-;
R³⁵ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or -C≡C-,
E is -OR⁶⁹, -SR⁶⁹, -NR⁶⁵R⁶⁶, -COR⁶⁸, -COOR⁶⁷, -CONR⁶⁵R⁶⁶, -CN, F, or NO₂;
G is E, or a C₁-C₁₈alkyl group, a C₃-C₁₈cycloalkyl group, a C₆-C₂₄aryl group, a C₆-C₂₄aryl group, which is substituted by F, -CN, C₁-C₁₈alkyl, or C₁-C₁₈alkyl which is interrupted by O;
a C₂-C₅₀heteroaryl group, or a C₂-C₅₀heteroaryl group, which is substituted by F, -CN, C₁-C₁₈alkyl, C₁-C₁₈alkyl which is interrupted by O;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ together form a five or six membered ring;
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-;
R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-;
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-;
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl; and
R⁷³ and R⁷⁴ are independently of each other H, or a phenyl group.
with the proviso that one of R¹, R² and R³ is a group of formula (**X**), or (**XI**).

Certain compounds of the present invention show, when used as host and/or hole blocker in combination with phosphorescent emitters, excellent power efficiencies, in particular electroluminescent (EL) devices comprising the compounds of the present invention exhibit reduced drive voltage and excellent operative lifetime while maintaining excellent luminance properties. Furthermore, the colour and external quantum efficiency (EQE) can be improved and the roll-off may be reduced by use of the inventive compounds.

The compounds of the present invention may be used for electrophotographic photoreceptors, photoelectric converters, organic solar cells (organic photovoltaics), switching elements, such as organic transistors, for example, organic FETs and organic TFTs, organic light emitting field effect transistors (OLEFETs), image sensors, dye lasers and electroluminescent devices, such as, for example, organic light-emitting diodes (OLEDs).

Accordingly, a further subject of the present invention is directed to an electronic device, comprising a compound according to the present invention. The electronic device is preferably an electroluminescent device.

The compounds of formula I can in principal be used in any layer of an EL device, but are preferably used as host, electron transport and/or hole blocking material.

Hence, a further subject of the present invention is directed to an electron transport layer and/or a hole blocking layer comprising a compound of formula I according to the present invention.

A further subject of the present invention is directed to an emitting layer, comprising a compound of formula I according to the present invention. In said embodiment a compound of formula I is preferably used as host material in combination with a phosphorescent emitter.

D is preferably -CO-, -COO-, -S-, -SO-, -SO₂-, -O-, -NR⁶⁵-, wherein R⁶⁵ is C₁-C₁₈alkyl, such as methyl, ethyl, n-propyl, iso-propyl, n-butyl, isobutyl, tert-butyl, or sec-butyl, or C₆-C₁₄aryl, such as phenyl, tolyl, naphthyl, or biphenylyl.

E is preferably -OR⁶⁹; -SR⁶⁹; -NR⁶⁵R⁶⁵; -COR⁶⁸; -COOR⁶⁷; -CONR⁶⁵R⁶⁵; or -CN; wherein R⁶⁵, R⁶⁷, R⁶⁸ and R⁶⁹ are independently of each other C₁-C₁₈alkyl, such as methyl, ethyl, n-propyl, iso-propyl, n-butyl, isobutyl, tert-butyl, sec-butyl, hexyl, octyl, or 2-ethyl-hexyl, or C₆-C₁₄aryl, such as phenyl, tolyl, naphthyl, or biphenylyl.

The C₁-C₂₅alkyl groups, R¹, R², R³, R⁴, R⁵, R⁶, R⁷, R⁸ and R⁹, are typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl.

A C₁-C₂₅alkyl group substituted by one or more E and/or interrupted by one or more units D is, for example, (CH₂CH₂O)₁₋₉-R^{x}, where R^{x} is H or C₁-C₁₀alkyl or C₂-C₁₀alkanoyl (e.g. CO-CH(C₂H₅)C₄H₉), CH₂-CH(OR^{y'})-CH₂-O-R^{y}, where R^{y} is C₁-C₁₈alkyl, C₅-C₁₂cycloalkyl, phenyl, C₇-C₁₅phenylalkyl, and R^{y'} embraces the same definitions as R^{y} or is H; C₁-C₈alkylene-COO-R^{z}, e.g. CH₂COOR_{z}, CH(CH₃)COOR^{z}, C(CH₃)₂COOR^{z}, where R^{z} is H, C₁-C₁₈alkyl, (CH₂CH₂O)₁₋₉-R^{x}, and R^{x} embraces the definitions indicated above; CH₂CH₂-O-CO-CH=CH₂; CH₂CH(OH)CH₂-O-CO-C(CH₃)=CH₂.

An alkyl group substituted by E is, for example, an alkyl group where at least one of the hydrogen atoms is replaced by F. Examples are -CF₃, -CF₂CF₃, -CF₂CF₂CF₃, -CF(CF₃)₂, - (CF₂)₃CF₃, and -C(CF₃)₃.

The C₂-C₂₅alkenyl group, R¹, R², R³, R⁴, R⁵, R⁶, R⁷, R⁸ and R⁹, is a straight-chain, or branched alkenyl group, such as, for example, vinyl, allyl, methallyl, isopropenyl, 2-butenyl, 3-butenyl, isobutenyl, n-penta-2,4-dienyl, 3-methyl-but-2-enyl, n-oct-2-enyl, n-dodec-2-enyl, isododecenyl, n-dodec-2-enyl or n-octadec-4-enyl.

The C₂-C₂₅alkynyl group, R¹, R², R³, R⁴, R⁵, R⁶, R⁷, R⁸ and R⁹, is straight-chain or branched and preferably a C₂-C₈alkynyl group, such as, for example, ethynyl, 1-propyn-3-yl, 1-butyn-4-yl, 1-pentyn-5-yl, 2-methyl-3-butyn-2-yl, 1,4-pentadiyn-3-yl, 1,3-pentadiyn-5-yl, 1-hexyn-6-yl, cis-3-methyl-2-penten-4-yn-1-yl, trans-3-methyl-2-penten-4-yn-1-yl, 1,3-hexadiyn-5-yl, 1-octyn-8-yl, 1-nonyn-9-yl, 1-decyn-10-yl, or 1-tetracosyn-24-yl.

Examples of the C₃-C₁₈cycloalkyl group are cyclopentyl, cyclohexyl, cycloheptyl, cyclooctyl, cyclononyl, cyclodecyl, cycloundecyl, cyclododecyl, preferably cyclopentyl, cyclohexyl, cycloheptyl and cyclooctyl, which may be unsubstituted or substituted by G.

The C₆-C₂₄aryl groups, R⁶, R⁷, R⁸, R⁹, R²⁴, R²⁵, R²⁶, R²⁷ and R²⁸, which optionally can be substituted by G, are typically phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, which may be unsubstituted or substituted by G.

The C₂-C₃₀heteroaryl group groups R⁶, R⁷, R⁸, R⁹, R²⁴, R²⁵, R²⁶, R²⁷ and R²⁸, which optionally can be substituted by G, represent a ring with five to seven ring atoms or a condensed ring system, wherein nitrogen, oxygen or sulfur are the possible hetero atoms, and is typically a heterocyclic group with five to 30 atoms having at least six conjugated π-electrons such as thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, benzimidazo[1,2-a]benzimidazo-5-yl, carbazolyl, or phenoxazinyl, which can be unsubstituted or substituted by G.

The wording "substituted by G" means that one, or more, especially one to three substituents G might be present. G has the same preferences as E, or is C₁-C₁₈alkyl, especially C₁-C₈alkyl, such as methyl, ethyl, n-propyl, iso-propyl, n-butyl, isobutyl, tert-butyl, sec-butyl, hexyl, octyl, or 2-ethyl-hexyl.

Prefered C₂-C₃₀heteroaryl groups are pyridyl, pyrimidyl, triazinyl, benzimidazo[1,2-a]benzimidazo-5-yl 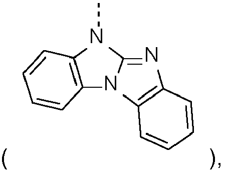 carbazolyl, dibenzofuranyl, which can be unsubstituted or substituted especially by C₆-C₁₀aryl, or C₆-C₁₀aryl, which is substituted by C₁-C₄alkyl; or C₂-C₁₄heteroaryl, especially

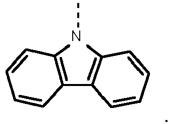

Examples of a group of formula Si(R²⁴)(R²⁵)(R²⁶) are a trimethylsilyl group, a triethylsilyl group, a tert-butyldimethylsilyl group, a propyldimethylsilyl group, a triisopropylsilyl group, a triphenylsilyl group, a phenyldimethylsilyl group, a t-butyldiphenylsilyl group, a tritolylsilyl group, a trixylylsilyl group, or a trinaphthylsilyl group.

Examples of the group P(O)(R²⁷)(R²⁸) include diphenylphosphoryl group and a dimethylphosphoryl group.

Examples of the group N(R³⁰)(R³¹) include diphenylamino and a phenylnaphthylamino group.

In a preferred embodiment of the present invention the compounds of formula (I) comprise 1, or 2 groups of formula (**X**) and/or (**XI**).

Preferred embodiments of the present invention are directed to compounds of formula 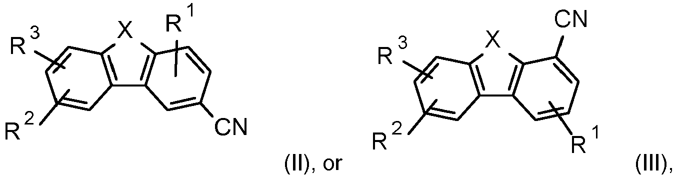 wherein X, R¹, R² and R³ are defined above, or below.

In said embodiment compounds of formula 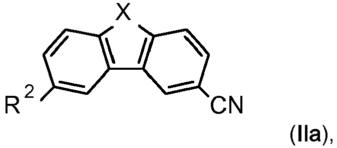 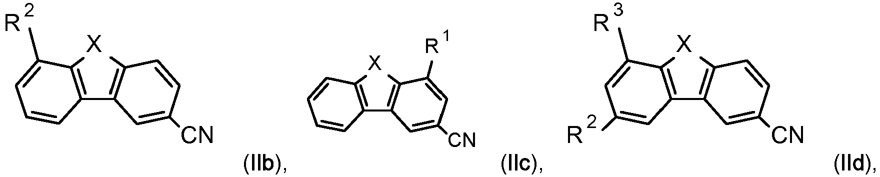 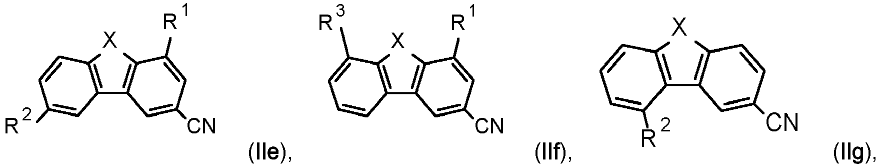 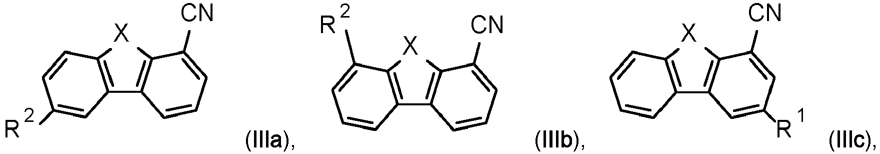 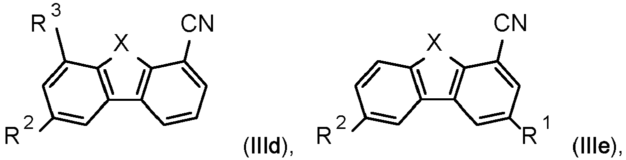 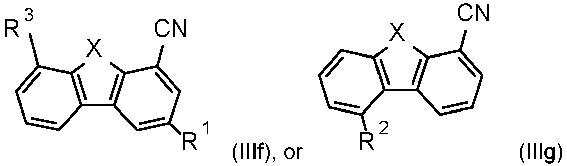 are more preferred, wherein **X**, R¹, R² and R³ are defined above, or below.
R¹ is preferably H, a group of formula (**X**), or (**XI**),
R² is preferably H, a group of formula (**X**), or (**XI**), and
R³ is preferably H, a group of formula (**X**), or (**XI**).

The group of formula (**X**) is preferably a group of formula 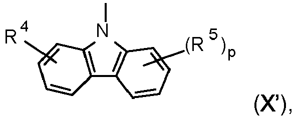 wherein p is 0, 1, or 2, R⁴ and R⁵ are independently of each other H, CN, a C₁-C₂₅alkyl group, or a group of formula 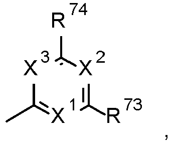 wherein
X¹, X² and X³ are independently of each other N, or CH, and
R⁷³ and R⁷⁴ are independently of each other H, or a phenyl group.

The group of formula (**X'**) is more preferably a group of formula 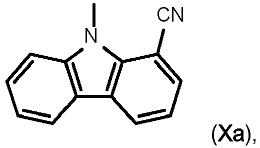 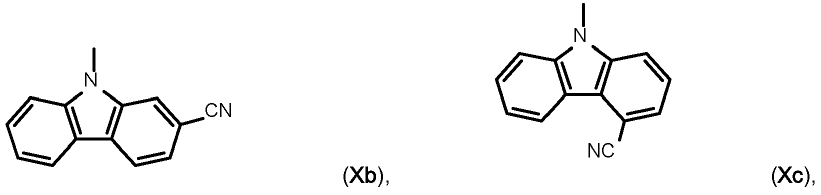 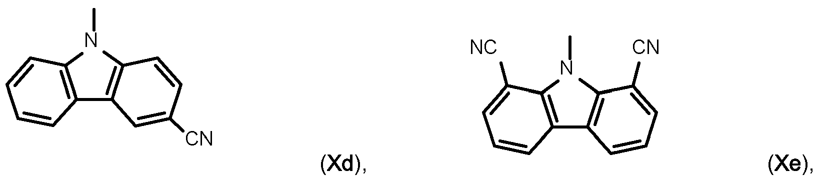 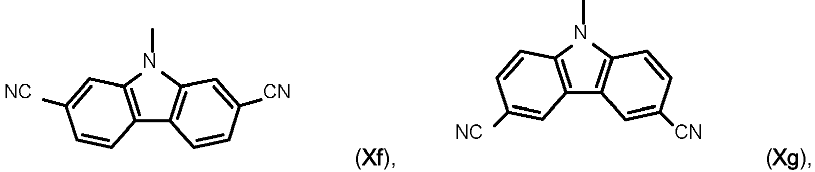 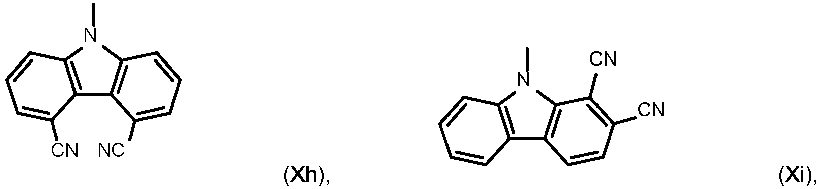 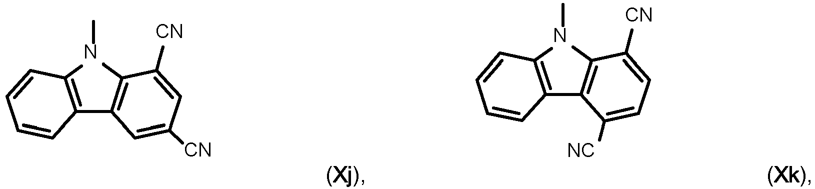 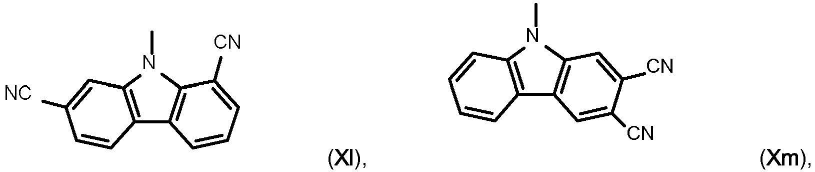 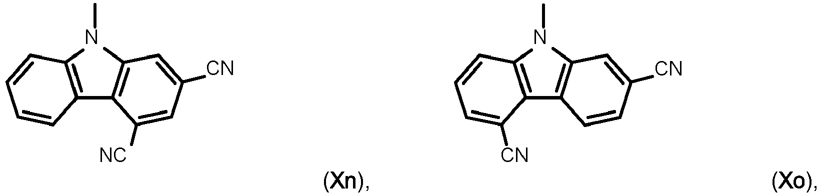 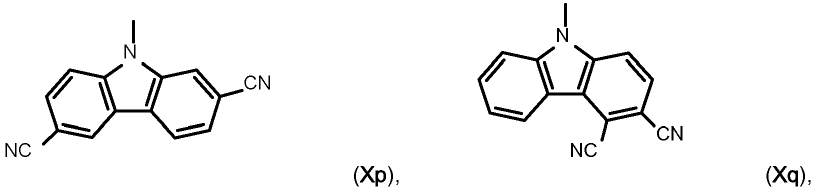 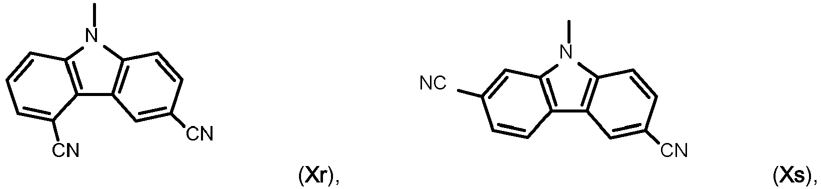 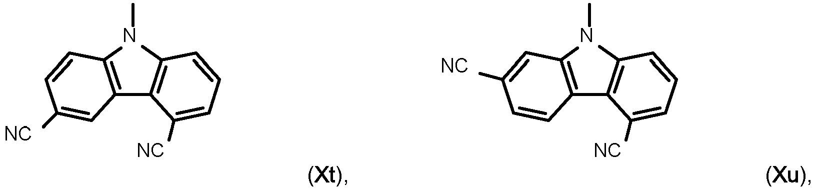 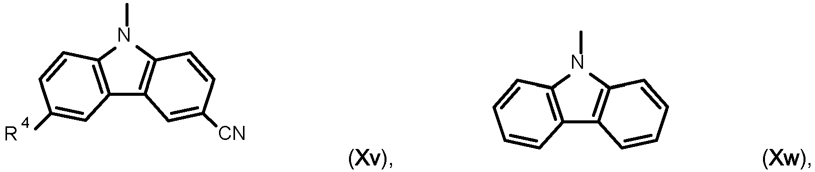 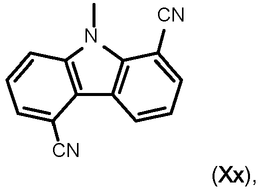 or 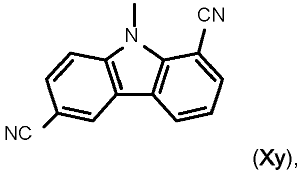 wherein R⁴ is a C₁-C₂₅alkyl group.

The group of formula (**XI**) is preferably a group of formula 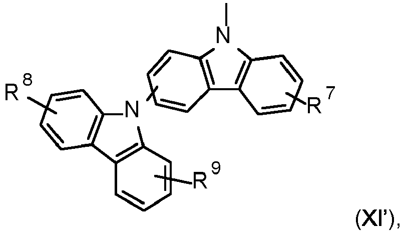 wherein
R⁷ is H, CN, or a C₁-C₂₅alkyl group;
R³ is H, CN, or a C₁-C₂₅alkyl group; and
R⁹ is H, CN, or a C₁-C₂₅alkyl group.

The group of formula (**XI**') is more preferably a group of formula 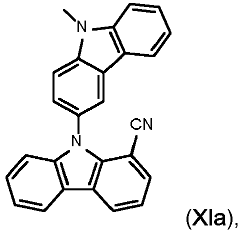 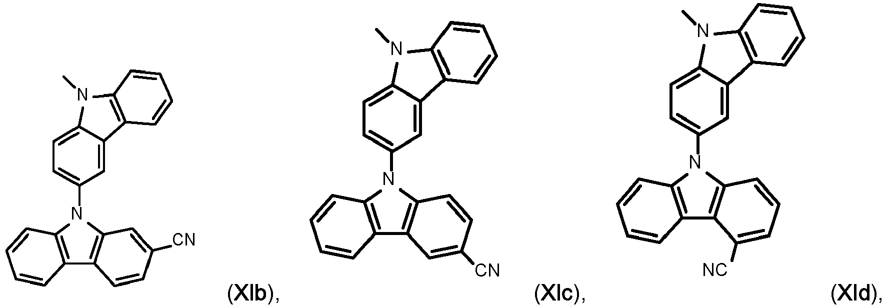 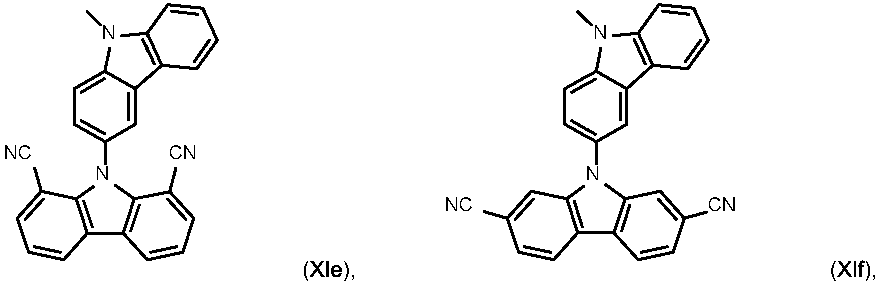 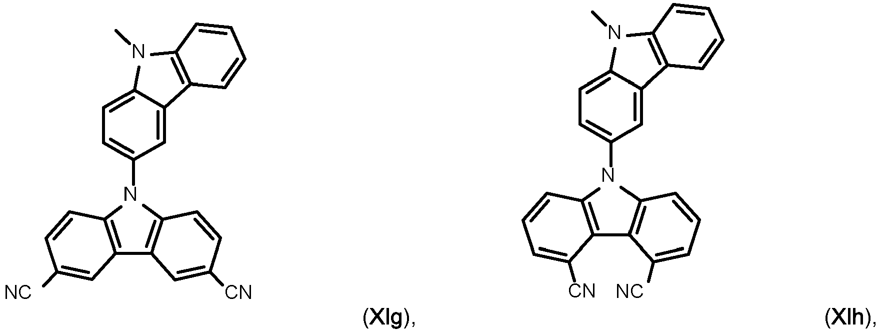 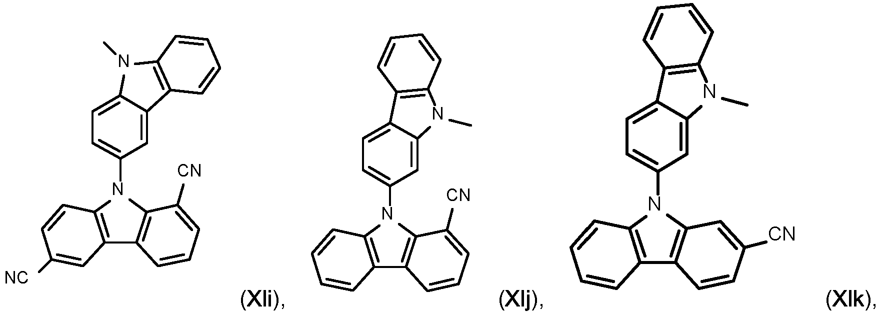 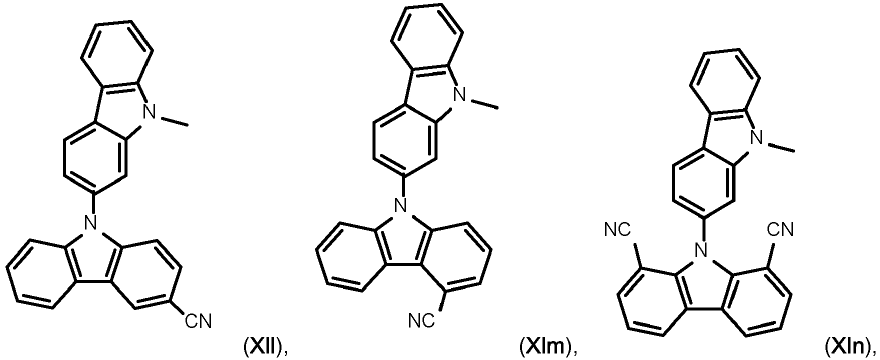 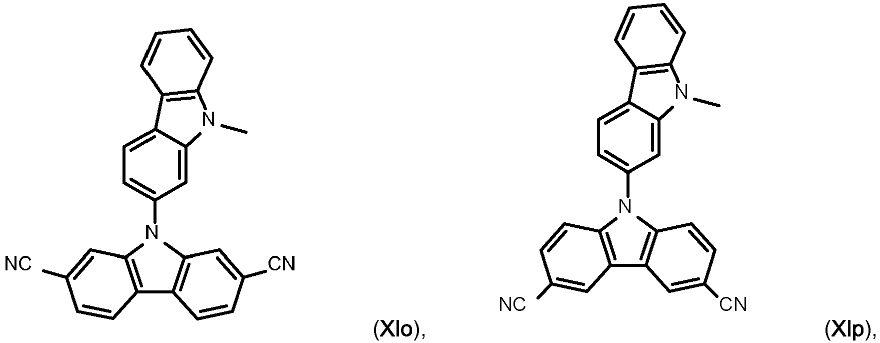 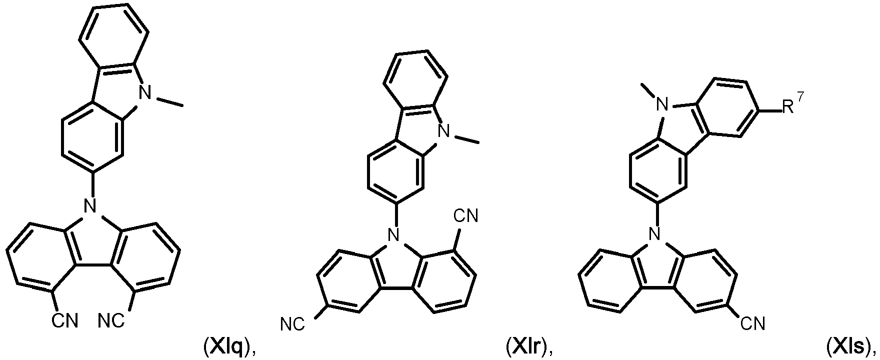 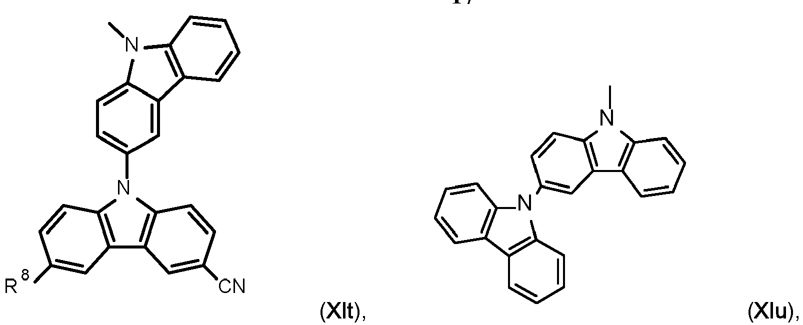 or 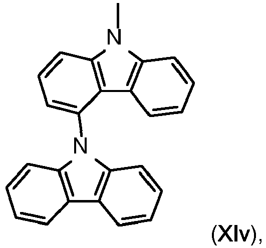
wherein R⁷ is a C₁-C₂₅alkyl group and R⁸ is a C₁-C₂₅alkyl group.
X is preferably O.

In a particularly preferred embodiment the present invention is directed to compounds of formula 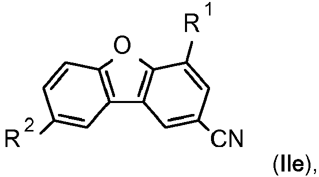 wherein
R¹ and R² are independently of each other a group of formula (**Xa**), (**Xb**), (**Xc**), (**Xd**), (**Xf**), (**Xg**), (**Xv**), or (**Xw**) as defined above; or a group of formula (**XIa**), (**XIb**), (**XIc**), (**XId**), (**XIf**), (**XIg**), (**XIs**), (**XIt**), (**XIu**), or (**XIv**) as defined above; or
R¹ is H, and R² is a group of formula (**Xa**), (**Xb**), (**Xc**), (**Xd**), (**Xf**), (**Xg**), (**Xv**), or (**Xw**) as defined above; or a group of formula (**XIa**), (**XIb**), (**XIc**), (**XId**), (**XIf**), (**XIg**), (**XIs**), (**XIt**), (**XIu**), or (**XIv**) as defined above; or
R² is H, and R¹ is a group of formula (**Xa**), (**Xb**), (**Xc**), (**Xd**), (**Xf**), (**Xg**), (**Xv**), or (**Xw**) as defined above; or a group of formula (**XIa**), (**XIb**), (**XIc**), (**XId**), (**XIf**), (**XIg**), (**XIs**), (**XIt**), (**XIu**), or (**XIv**) as defined above.

Examples of particularly preferred compounds of formula (**I**) are shown below, it is however noted that compounds A25-A32, A44-A47, B25-B32 and B44-B47 do no longer fall within the scope of the present invention: 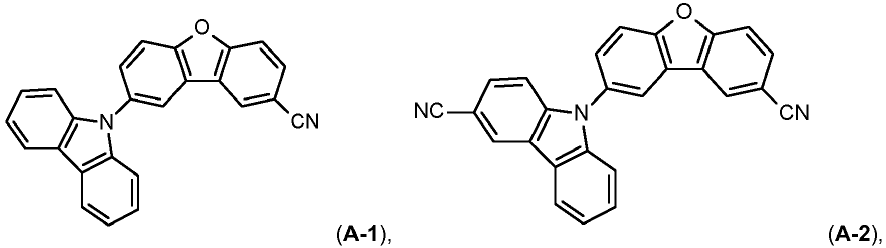 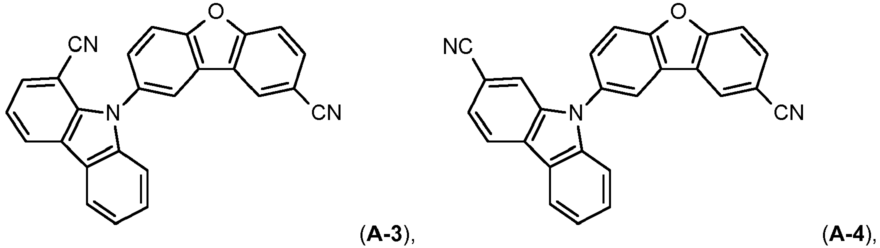 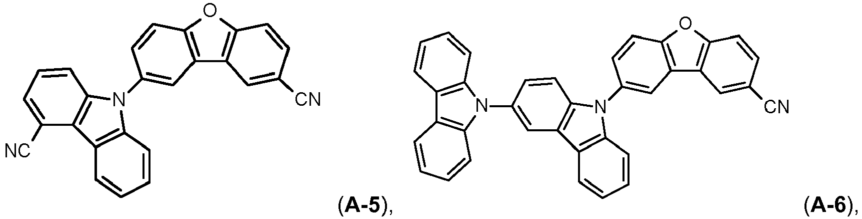 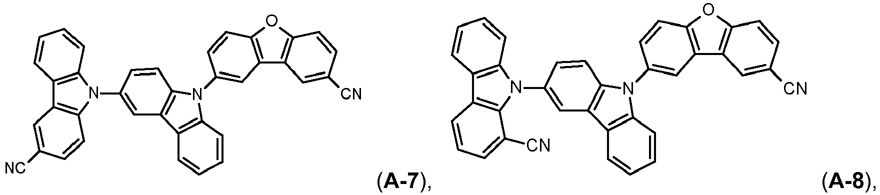 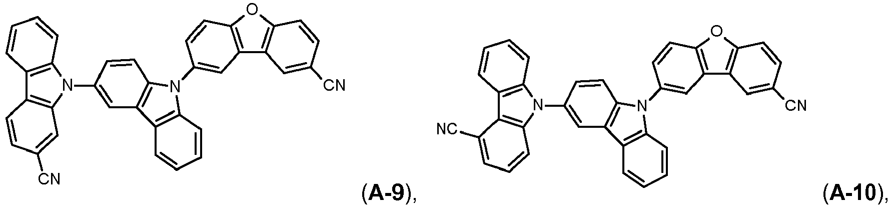 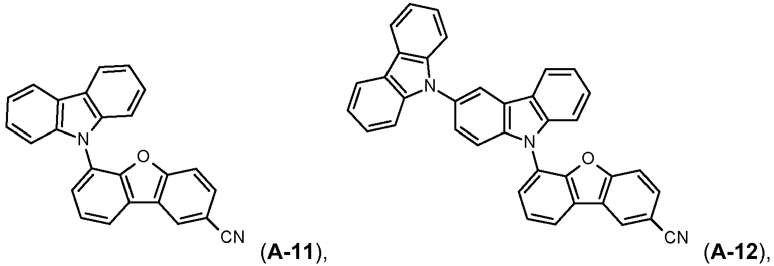 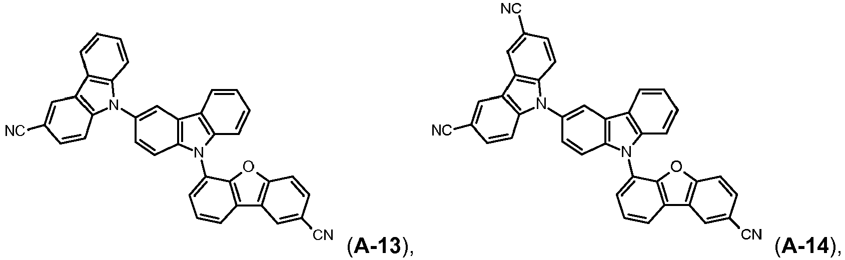 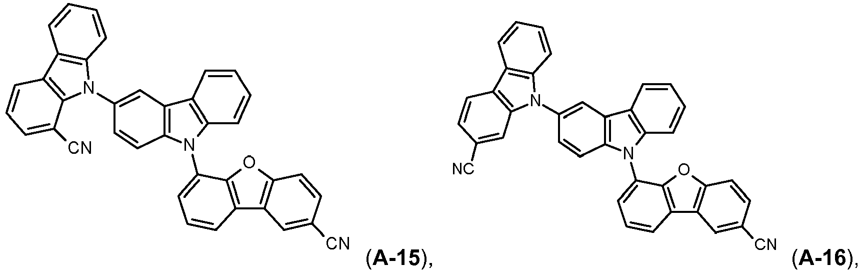 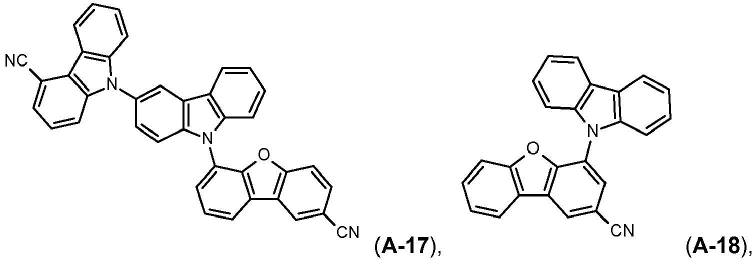 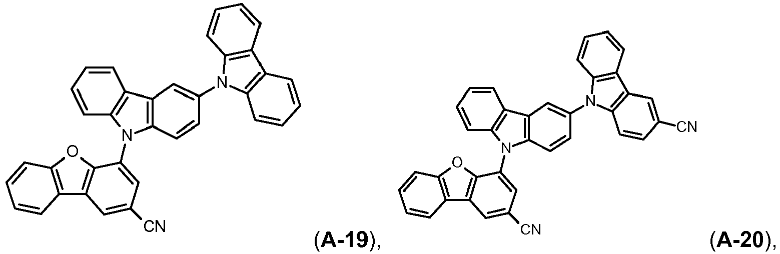 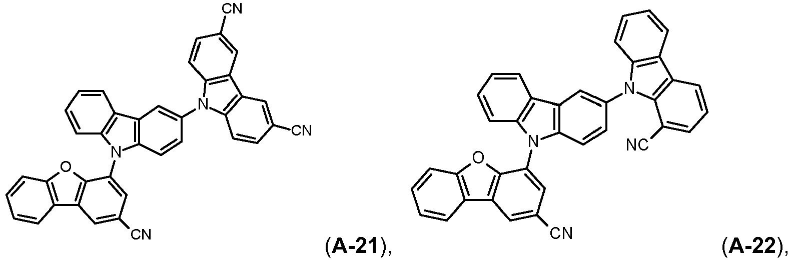 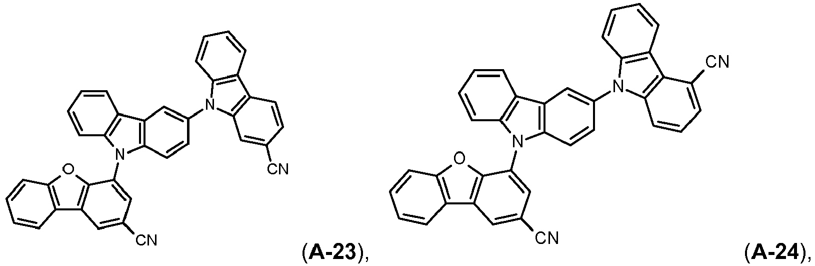 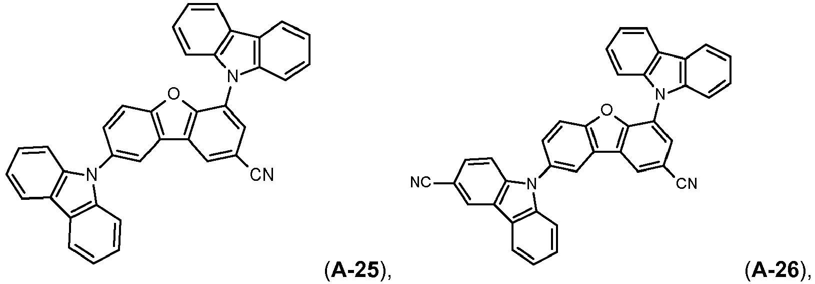 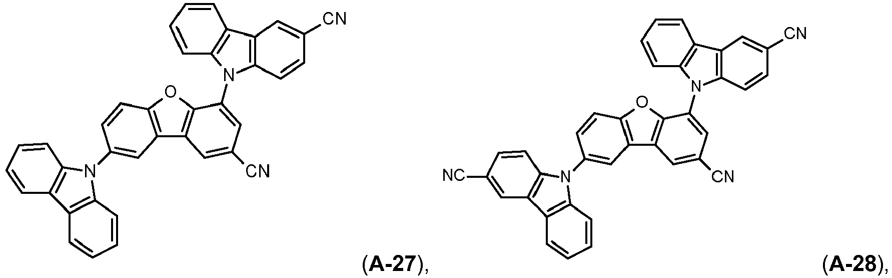 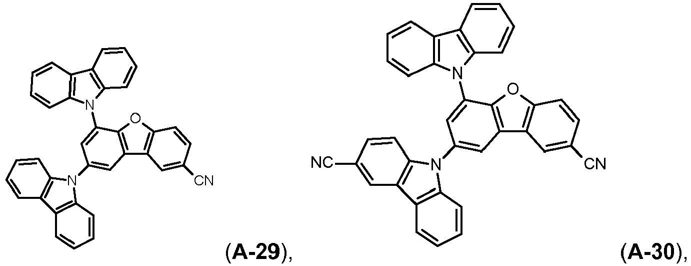 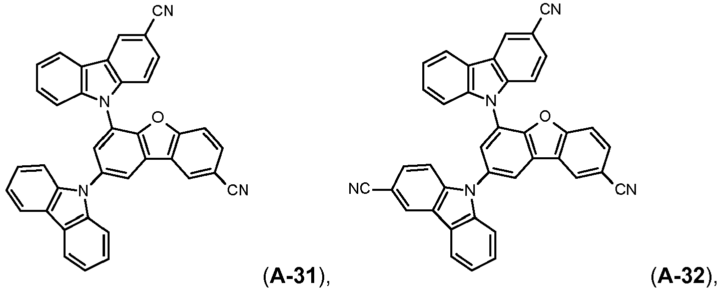 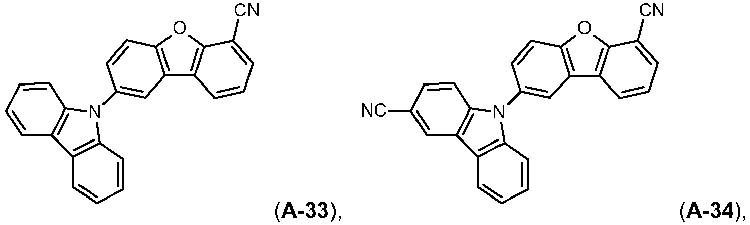 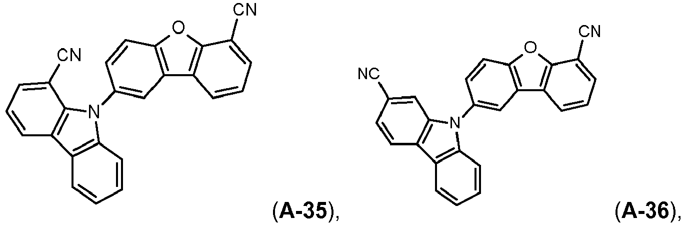 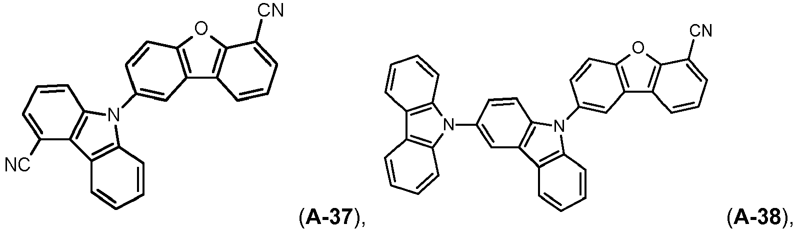 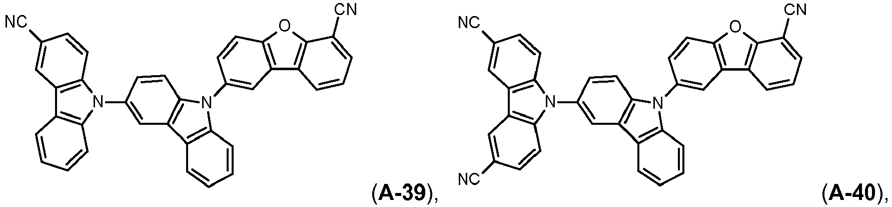 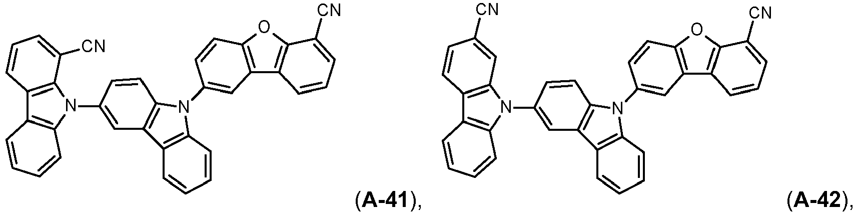 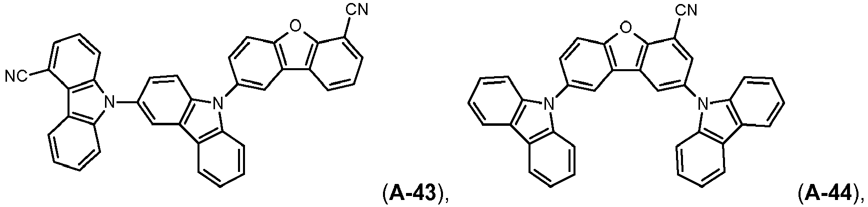 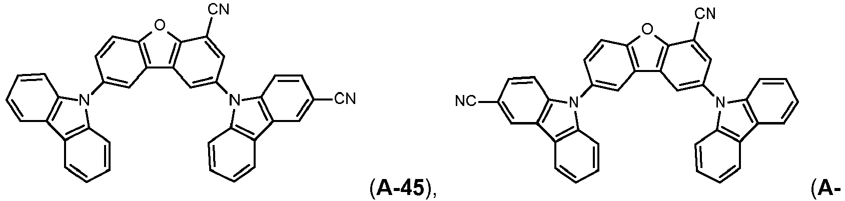 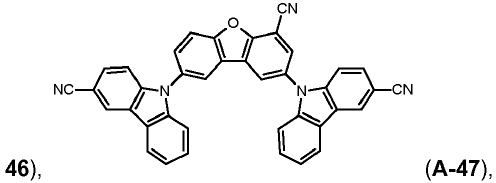 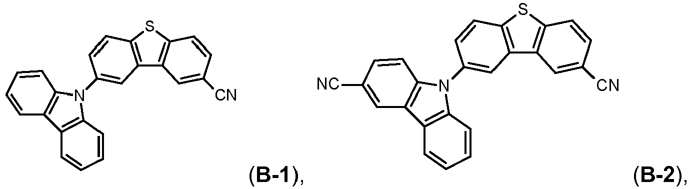 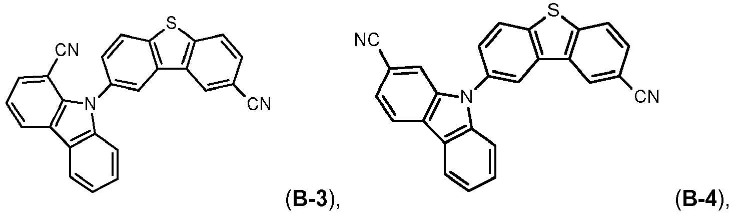 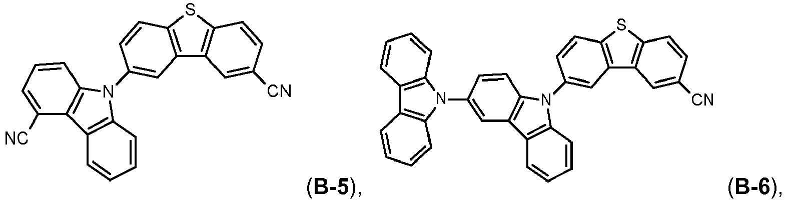 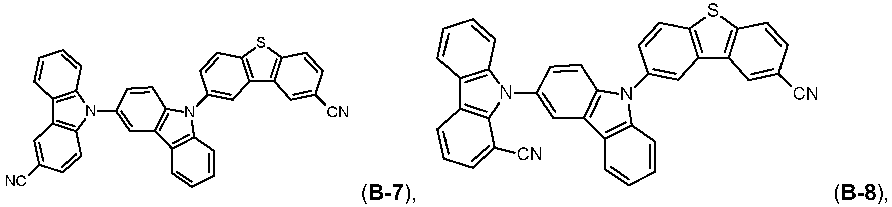 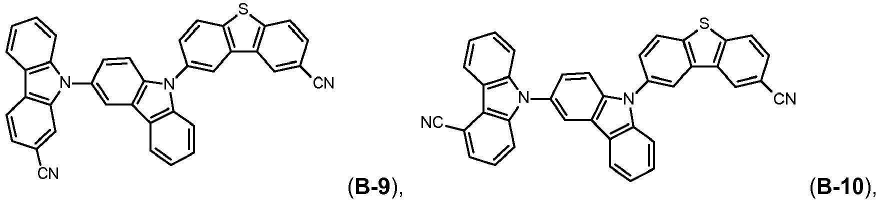 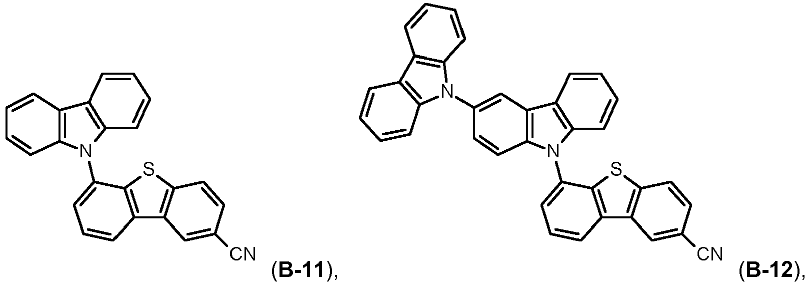 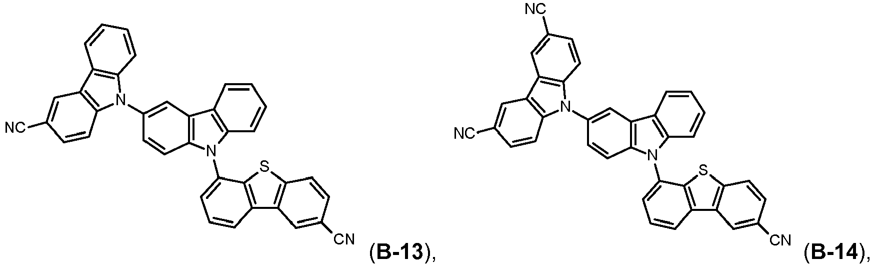 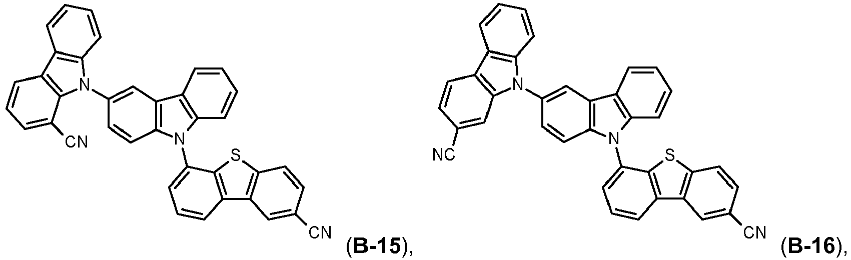 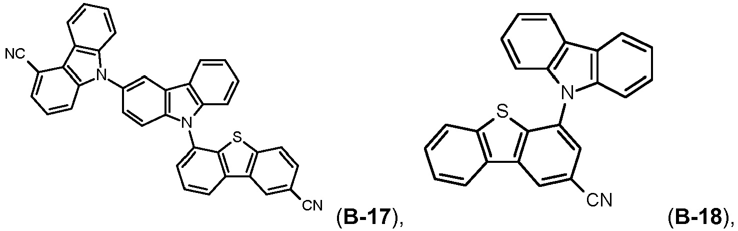 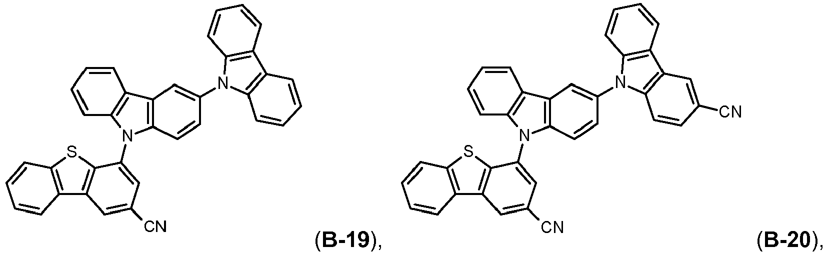 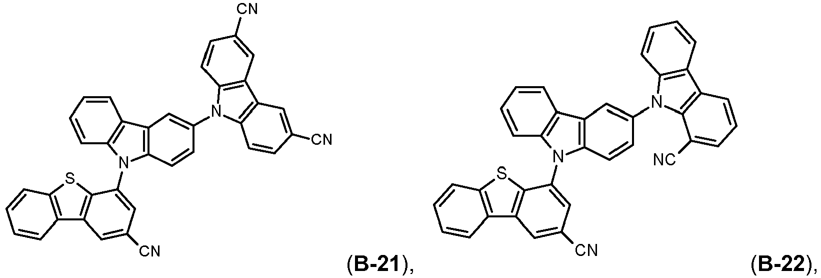 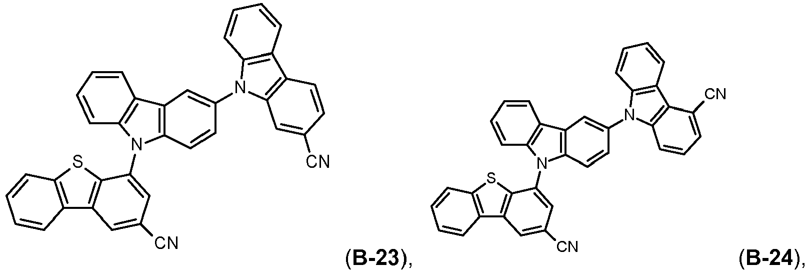 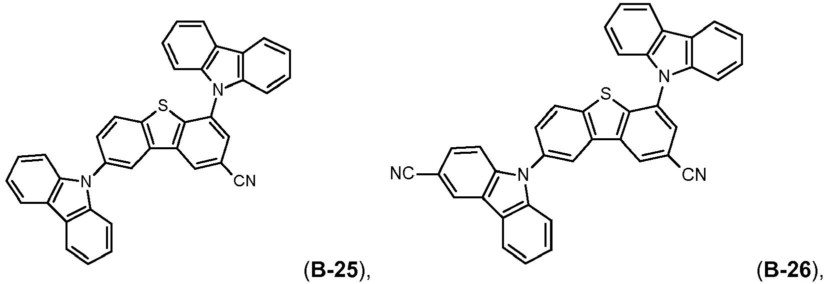 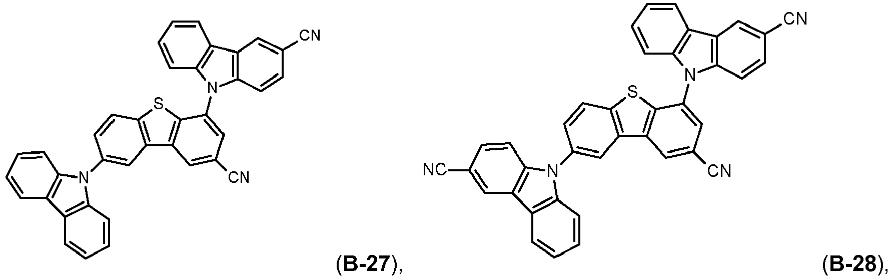 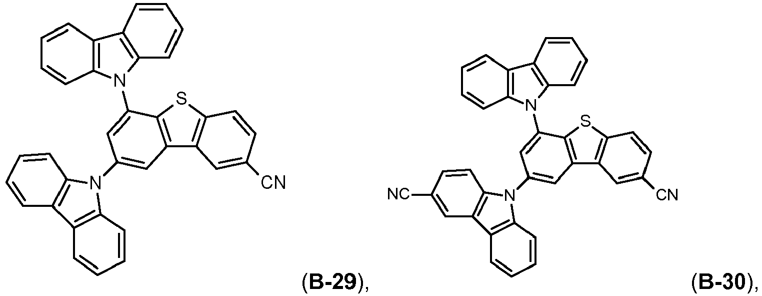 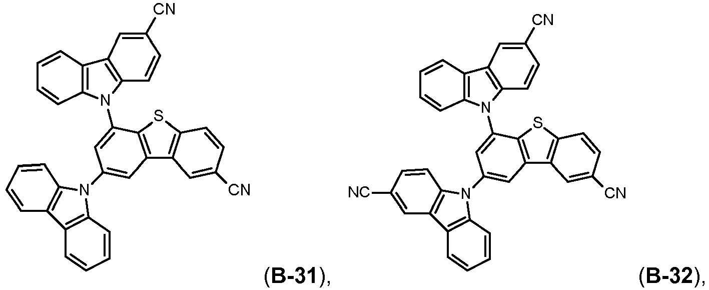 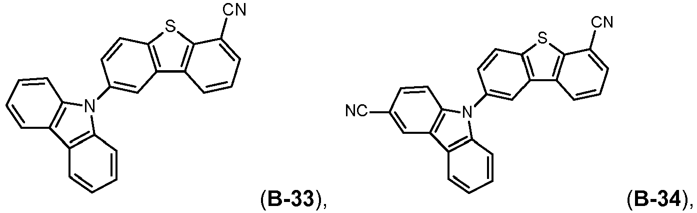 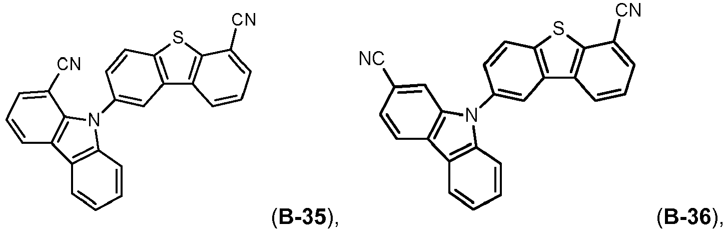 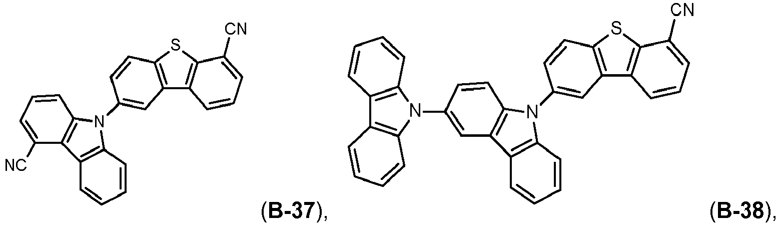 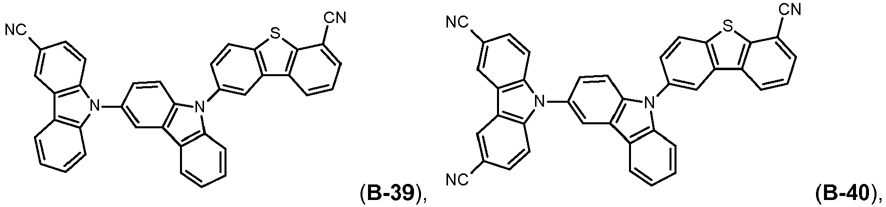 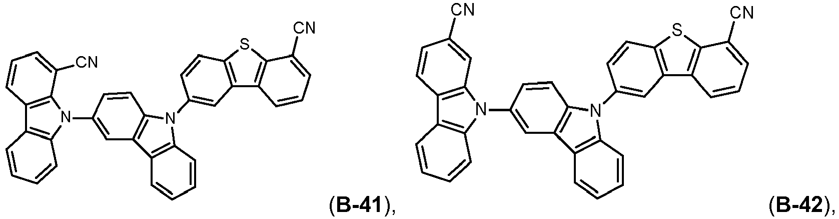 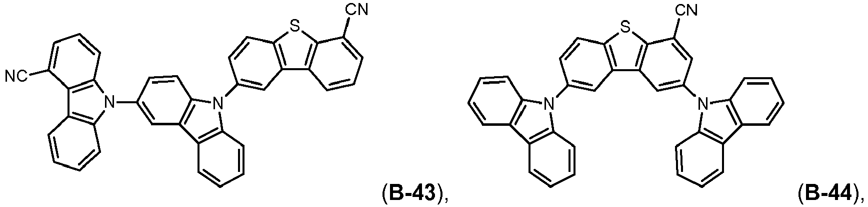 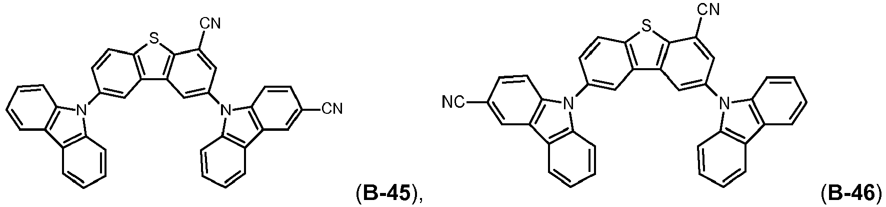 and

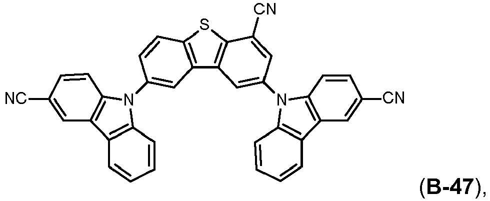

C₁-C₂₅alkyl (C₁-C₁₈alkyl) is typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. C₁-C₄alkyl is typically methyl, ethyl, n-propyl, isopropyl, n-butyl, sec.-butyl, isobutyl, tert.-butyl.

C₁-C₂₅alkoxy groups (C₁-C₁₈alkoxy groups) are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, un-decyloxy, dodecyloxy, tetradecyloxy, pentadecyloxy, hexadecyloxy, heptadecyloxy and octadecyloxy. Examples of C₁-C₈alkoxy are methoxy, ethoxy, n-propoxy, isopropoxy, n-butoxy, sec.-butoxy, isobutoxy, tert.-butoxy, n-pentyloxy, 2-pentyloxy, 3-pentyloxy, 2,2-dimethylpropoxy, n-hexyloxy, n-heptyloxy, n-octyloxy, 1,1,3,3-tetramethylbutoxy and 2-ethylhexyloxy, preferably C₁-C₄alkoxy such as typically methoxy, ethoxy, n-propoxy, isopropoxy, n-butoxy, sec.-butoxy, isobutoxy, tert.-butoxy.

C₆-C₂₄aryl (C₆-C₁₈aryl), which optionally can be substituted, is typically phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, which may be unsubstituted or substituted by G. Phenyl, 1-naphthyl and 2-naphthyl are examples of a C₆-C₁₀aryl group.

C₂-C₃₀heteroaryl represents a ring with five to seven ring atoms or a condensed ring system, wherein nitrogen, oxygen or sulfur are the possible hetero atoms, and is typically a heterocyclic group with five to 30 atoms having at least six conjugated π-electrons such as thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, 4-imidazo[1,2-a]benzimidazoyl, 5-benzimidazo[1,2-a]benzimidazoyl, carbazolyl, or phenoxazinyl, which can be unsubstituted or substituted by G. Benzimidazo[1,2-a]benzimidazo-5-yl, benzimidazo[1,2-a]benzimidazo-2-yl, carbazolyl and dibenzofuranyl are examples of a C₂-C₁₄heteroaryl group.

The term "cycloalkyl group" is typically C₃-C₁₈cycloalkyl, especially C₅-C₁₂cycloalkyl, such as cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, or cyclooctyl, which may be unsubstituted or substituted by G.

G is as defined above and is especially C₁-C₁₈alkyl, very especially C₁-C₈alkyl, such as methyl, ethyl, n-propyl, iso-propyl, n-butyl, isobutyl, tert-butyl, sec-butyl, hexyl, octyl, or 2-ethyl-hexyl.

The wording "substituted by G" means that one, or more, especially one to three substituents G might be present. If a substituent occurs more than one time in a group, it can be different in each occurrence.

As described above, the aforementioned groups may be interrupted by D. Interruptions are of course possible only in the case of groups containing at least 2 carbon atoms connected to one another by single bonds. C₁-C₁₈alkyl interrupted by one or more units D is, for example, (CH₂CH₂O)₁₋₉-R^{x}, where R^{x} is H or C₁-C₁₀alkyl or C₂-C₁₀alkanoyl (e.g. CO-CH(C₂H₅)C₄H₉), CH₂-CH(OR^{y'})-CH₂-O-R^{y}, where R^{y} is C₁-C₁₈alkyl, C₅-C₁₂cycloalkyl, phenyl, C₇-C₁₅phenylalkyl, and R^{y'} embraces the same definitions as R^{y} or is H; C₁-C₈alkylene-COO-R^{z}, e.g. CH₂COOR_{z}, CH(CH₃)COOR^{z}, C(CH₃)₂COOR^{z}, where R^{z} is H, C₁-C₁₈alkyl, (CH₂CH₂O)₁₋₉-R^{x}, and R^{x} embraces the definitions indicated above; CH₂CH₂-O-CO-CH=CH₂; CH₂CH(OH)CH₂-O-CO-C(CH₃)=CH₂.

Nitrile substituted carbazoles can be synthesized starting from the corresponding bromo or iodo substituted carbazoles using Rosenmund-von Braun reactions in DMF as described, for example, by D. A. Patrick, European Journal of Medicinal Chemistry (1997) 32. 781-793. Alternatively nitrile substituted phenyl hydrazine can be reacted with cyclohexanone to yield nitrile substituted 2,3,4,9-tetrahydro-1H-carbazole that can be oxidized to nitrile substituted carbazoles by using chloranil as described, for example, by B. M. Barclay, Journal of the Chemical Society, 1945, 530.

Alternatively carbazole can be reacted to the corresponding aldehyde using Vilsmeier-Haack conditions followed by transforming the aldehyde to the nitrile as described by K. Moriyama, Tetrahedron, 201, 68, 4588. Bromo or iodo substituted dibenzofurans can be reacted with CuCN in DMF to yield nitrile substituted dibenzofurans. Alternatively, dibenzofuran carbaldehydes can be reacted with hydroxylamine followed by dehydration to yield nitrile substituted dibenzofurans, or dibenzofuran carboxylic acids can be transformed to the corresponding dibenzofuran carboxylic amides followed by dehydration to yield nitrile substituted dibenzofurans.

The carbazole substituted dibenzofurans can be synthesized by reacting halogenated dibenzofuran derivatives with nitrile substituted carbazoles using copper catalyzed Ullmann conditions as described, for example, in EP2301921, H. Gilman and D. A. Shirley, J. Am. Chem. Soc. 66 (1944) 888; D. Li et al., Dyes and Pigments 49 (2001) 181 - 186 and Eur. J. Org. Chem. (2007) 2147-2151. The reaction can be performed in solvent or in a melt. Suitable solvents are, for example, (polar) aprotic solvents such as dimethyl sulfoxide, dimethylformamide, NMP, tridecane or alcohols.

Alternatively, the carbazole substituted dibenzofurans can be synthesized by reacting halogenated dibenzofuran derivatives with nitrile substituted carbazoles using palladium catalyzed Buchwald-Hartwig conditions as described, for example, in Björn Schlummer, Ulrich Scholz, Adv. Synth. Catal. 2004, 346, 1599 - 1626.

Suitable base skeletons of the formula 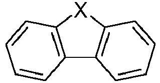 are either commercially available (especially in the cases when X is S, O, NH), or can be obtained by processes known to those skilled in the art. Reference is made to WO2010079051 and EP1885818.

Optionally substituted dibenzofurans, dibenzothiophenes and carbazoles can be dibrominated in the 2,8 positions (dibenzofuran and dibenzothiophene) or 3,6 positions (carbazole) with bromine or NBS in glacial acetic acid or in chloroform. For example, the bromination with Br₂ can be effected in glacial acetic acid or chloroform at low temperatures, e.g. 0°C. Suitable processes are described, for example, in M. Park, J.R. Buck, C.J. Rizzo, Tetrahedron, 54 (1998) 12707-12714 for X= NPh, and in W. Yang et al., J. Mater. Chem. 13 (2003) 1351 for X= S. In addition, 3,6-dibromocarbazole, 3,6-dibromo-9-phenylcarbazole, 2,8-dibromodibenzothiophene, 2,8-dibromodibenzofuran, 2-bromocarbazole, 3-bromodibenzothiophene, 3-bromodibenzofuran, 3-bromocarbazole, 2-bromodibenzothiophene and 2-bromodibenzofuran are commercially available.

Monobromination in the 4 position of dibenzofuran (and analogously for dibenzothiophene) is described, for example, in J. Am. Chem. Soc. 1984, 106, 7150. Dibenzofuran (dibenzothiophene) can be monobrominated in the 3 position by a sequence known to those skilled in the art, comprising a nitration, reduction and subsequent Sandmeyer reaction.

Monobromination in the 2 position of dibenzofuran or dibenzothiophene and monobromination in the 3 position of carbazole are effected analogously to the dibromination, with the exception that only one equivalent of bromine or NBS is added.

Alternatively, it is also possible to utilize iodinated dibenzofurans, dibenzothiophenes and carbazoles. The preparation is described, inter alia, in Tetrahedron. Lett. 47 (2006) 6957-6960, Eur. J. Inorg. Chem. 24 (2005) 4976-4984, J. Heterocyclic Chem. 39 (2002) 933-941, J. Am. Chem. Soc. 124 (2002) 11900-11907, J. Heterocyclic Chem, 38 (2001) 77-87.

For the nucleophilic substitution, Cl- or F-substituted dibenzofurans, dibenzothiophenes and carbazoles are required. The chlorination is described, inter alia, in J. Heterocyclic Chemistry, 34 (1997) 891-900, Org. Lett., 6 (2004) 3501-3504; J. Chem. Soc. [Section] C: Organic, 16 (1971) 2775-7, Tetrahedron Lett. 25 (1984) 5363-6, J. Org. Chem. 69 (2004) 8177-8182. The fluorination is described in J. Org. Chem. 63 (1998) 878-880 and J. Chem. Soc., Perkin Trans. 2, 5 (2002) 953-957. 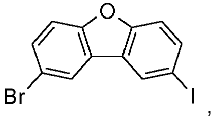 is described in EP1885818. It has been found that the compounds of the formula I are particularly suitable for use in applications in which charge carrier conductivity is required, especially for use in organic electronics applications, for example selected from switching elements such as organic transistors, e.g. organic FETs and organic TFTs, organic solar cells and organic light-emitting diodes (OLEDs), the compounds of the formula I being particularly suitable in OLEDs for use as host material in a light-emitting layer and/or as hole and/or exciton blocker material and/or as electron transport material, especially in combination with a phosphorescence emitter, very especially blue, or green emitting phosphorescence emitters. In the case of use of the inventive compounds of the formula I in OLEDs, OLEDs which have good efficiencies and a long lifetime and which can be operated especially at a low use and operating voltage are obtained. Furthermore, the compounds of the formula I can be used as conductor/complementary materials in organic electronics applications selected from switching elements and organic solar cells.

In the emission layer or one of the emission layers of an OLED, it is also possible to combine an emitter material with a matrix material of the compound of the formula I and a further matrix material which has, for example, a good hole transport property. This achieves a high quantum efficiency of this emission layer.

Suitable structures of organic electronic devices are known to those skilled in the art and are specified below.

The organic transistor generally includes a semiconductor layer formed from an organic layer with hole transport capacity and/or electron transport capacity; a gate electrode formed from a conductive layer; and an insulating layer introduced between the semiconductor layer and the conductive layer. A source electrode and a drain electrode are mounted on this arrangement in order thus to produce the transistor element. In addition, further layers known to those skilled in the art may be present in the organic transistor.

The organic solar cell (photoelectric conversion element) generally comprises an organic layer present between two plate-type electrodes arranged in parallel. The organic layer may be configured on a comb-type electrode. There is no particular restriction regarding the site of the organic layer and there is no particular restriction regarding the material of the electrodes. When, however, plate-type electrodes arranged in parallel are used, at least one electrode is preferably formed from a transparent electrode, for example an ITO electrode or a fluorine-doped tin oxide electrode. The organic layer is formed from two sublayers, i.e. a layer with p-type semiconductor properties or hole transport capacity, and a layer formed with n-type semiconductor properties or electron transport capacity. In addition, it is possible for further layers known to those skilled in the art to be present in the organic solar cell. The layer with electron transport capacity may comprise the compounds of formula I.

### Structure of the inventive OLED

The inventive organic light-emitting diode (OLED) generally has the following structure: an anode (a) and a cathode (i) and a light-emitting layer (e) arranged between the anode (a) and the cathode (i).

The inventive OLED may, for example - in a preferred embodiment - be formed from the following layers:
1. Anode (a)
2. Hole transport layer (c)
3. Light-emitting layer (e)
4. Blocking layer for holes/excitons (f)
5. Electron transport layer (g)
6. Cathode (i)

Layer sequences different from the aforementioned structure are also possible, and are known to those skilled in the art. For example, it is possible that the OLED does not have all of the layers mentioned; for example, an OLED with layers (a) (anode), (e) (light-emitting layer) and (i) (cathode) is likewise suitable, in which case the functions of the layers (c) (hole transport layer) and (f) (blocking layer for holes/excitons) and (g) (electron transport layer) are assumed by the adjacent layers. OLEDs which have layers (a), (c), (e) and (i), or layers (a), (e), (f), (g) and (i), are likewise suitable. In addition, the OLEDs may have a blocking layer for electrons/excitons (d) between the hole transport layer (c) and the Light-emitting layer (e).

It is additionally possible that a plurality of the aforementioned functions (electron/exciton blocker, hole/exciton blocker, hole injection, hole conduction, electron injection, electron conduction) are combined in one layer and are assumed, for example, by a single material present in this layer. For example, a material used in the hole transport layer, in one embodiment, may simultaneously block excitons and/or electrons.

Furthermore, the individual layers of the OLED among those specified above may in turn be formed from two or more layers. For example, the hole transport layer may be formed from a layer into which holes are injected from the electrode, and a layer which transports the holes away from the hole-injecting layer into the light-emitting layer. The electron transport layer may likewise consist of a plurality of layers, for example a layer in which electrons are injected by the electrode, and a layer which receives electrons from the electron injection layer and transports them into the light-emitting layer. These layers mentioned are each selected according to factors such as energy level, thermal resistance and charge carrier mobility, and also energy difference of the layers specified with the organic layers or the metal electrodes. The person skilled in the art is capable of selecting the structure of the OLEDs such that it is matched optimally to the organic compounds used in accordance with the invention.

In a preferred embodiment the OLED according to the present invention comprises in this order:
(a) an anode,
(b) optionally a hole injection layer,
(c) optionally a hole transport layer,
(d) optionally an electron/exciton blocking layer
(e) an emitting layer,
(f) optionally a hole/ exciton blocking layer
(g) optionally an electron transport layer,
(h) optionally an electron injection layer, and
(i) a cathode.

In a particularly preferred embodiment the OLED according to the present invention comprises in this order:
(a) an anode,
(b) optionally a hole injection layer,
(c) a hole transport layer,
(d) an electron/exciton blocking layer
(e) an emitting layer,
(f) a hole/ exciton blocking layer
(g) an electron transport layer, and
(h) optionally an electron injection layer, and
(i) a cathode.

The properties and functions of these various layers, as well as example materials are known from the prior art and are described in more detail below on basis of preferred embodiments.

### Anode (a):

The anode is an electrode which provides positive charge carriers. It may be composed, for example, of materials which comprise a metal, a mixture of different metals, a metal alloy, a metal oxide or a mixture of different metal oxides. Alternatively, the anode may be a conductive polymer. Suitable metals comprise the metals of groups 11, 4, 5 and 6 of the Periodic Table of the Elements, and also the transition metals of groups 8 to 10. When the anode is to be transparent, mixed metal oxides of groups 12, 13 and 14 of the Periodic Table of the Elements are generally used, for example indium tin oxide (ITO). It is likewise possible that the anode (a) comprises an organic material, for example polyaniline, as described, for example, in Nature, Vol. 357, pages 477 to 479 (June 11, 1992). Preferred anode materials include conductive metal oxides, such as indium tin oxide (ITO) and indium zinc oxide (IZO), aluminum zinc oxide (AlZnO), and metals. Anode (and substrate) may be sufficiently transparent to create a bottom-emitting device. A preferred transparent substrate and anode combination is commercially available ITO (anode) deposited on glass or plastic (substrate). A reflective anode may be preferred for some top-emitting devices, to increase the amount of light emitted from the top of the device. At least either the anode or the cathode should be at least partly transparent in order to be able to emit the light formed. Other anode materials and structures may be used.

### Hole injection layer (b):

Generally, injection layers are comprised of a material that may improve the injection of charge carriers from one layer, such as an electrode or a charge generating layer, into an adjacent organic layer. Injection layers may also perform a charge transport function. The hole injection layer may be any layer that improves the injection of holes from anode into an adjacent organic layer. A hole injection layer may comprise a solution deposited material, such as a spin-coated polymer, or it may be a vapor deposited small molecule material, such as, for example, CuPc, MTDATA, or dipyrazino[2,3-f:2',3'-h]quinoxaline-2,3,6,7,10,11-hexacarbonitrile (HAT-CN). Polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PEDOT/PSS.

### Hole transport layer (c):

Either hole-transporting molecules or polymers may be used as the hole transport material. Suitable hole transport materials for layer (c) of the inventive OLED are disclosed, for example, in Kirk-Othmer Encyclopedia of Chemical Technology, 4th Edition, Vol. 18, pages 837 to 860, 1996, US20070278938, US2008/0106190, US2011/0163302 (triarylamines with (di)benzothiophen/(di)benzofuran; Nan-Xing Hu et al. Synth. Met. 111 (2000) 421 (indolocarbazoles), WO2010002850 (substituted phenylamine compounds) and WO2012/16601 (in particular the hole transport materials mentioned on pages 16 and 17 of WO2012/16601). Combination of different hole transport material may be used. Reference is made, for example, to WO2013/022419, wherein 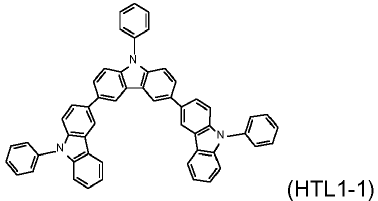 and 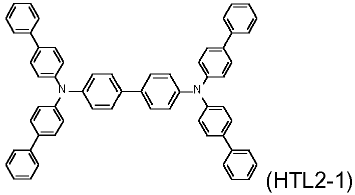 constitute the hole transport layer. Customarily used hole-transporting molecules are selected from the group consisting of 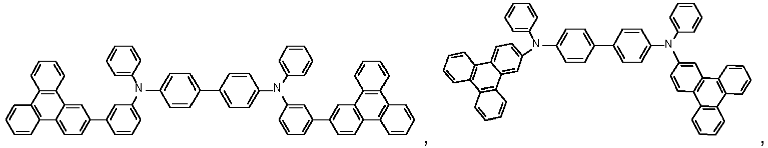 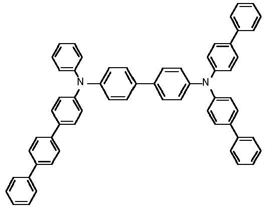 (4-phenyl-N-(4-phenylphenyl)-N-[4-[4-(N-[4-(4-phenyl-phenyl)phenyl]anilino)phenyl]phenyl]aniline), 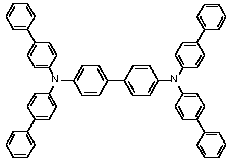 (4-phenyl-N-(4-phenylphenyl)-N-[4-[4-(4-phenyl-N-(4-phenylphenyl)anilino)phenyl]phenyl]aniline), 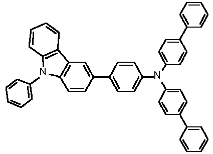 (4-phenyl-N-[4-(9-phenylcarbazol-3-yl)phenyl]-N-(4-phenylphenyl)aniline), 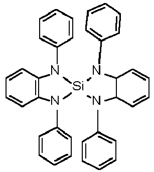 (1,1',3,3'-tetraphenylspiro[1,3,2-benzodiazasilole-2,2'-3a,7a-dihydro-1,3,2-benzodiazasilole]), 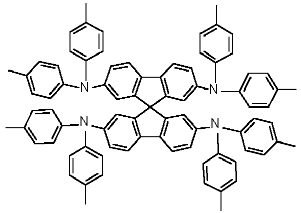 (N2,N2,N2',N2',N7,N7,N7',N7'-octakis(p-tolyl)-9,9'-spirobi[fluorene]-2,2',7,7'-tetramine), 4,4'-bis[N-(1-naphthyl)-N-phenylamino]biphenyl (α-NPD), N,N'-diphenyl-N,N'-bis(3-methylphenyl)-[1,1'-biphenyl]-4,4'-diamine (TPD), 1,1-bis[(di-4-tolylamino)phenyl]-cyclohexane (TAPC), N,N'-bis(4-methylphenyl)-N,N'-bis(4-ethylphenyl)-[1,1'-(3,3'-dimethyl)-biphenyl]-4,4'-diamine (ETPD), tetrakis(3-methylphenyl)-N,N,N',N'-2,5-phenylenediamine (PDA), α-phenyl-4-N,N-diphenylaminostyrene (TPS), p-(diethylamino)benzaldehyde diphenylhydrazone (DEH), triphenylamine (TPA), bis[4-(N,N-diethylamino)2-methylphenyl](4-methylphenyl)methane (MPMP), 1-phenyl-3-[p-(diethylamino)styryl]5-[p-(diethylamino)phenyl]pyrazoline (PPR or DEASP), 1,2-trans-bis(9H-carbazol9-yl)-cyclobutane (DCZB), N,N,N',N'-tetrakis(4-methylphenyl)-(1,1'-biphenyl)-4,4'-diamine (TTB), fluorine compounds such as 2,2',7,7'-tetra(N,N-di-tolyl)amino9,9-spirobifluorene (spiro-TTB), N,N'-bis(naphthalen-1-yl)-N,N'-bis(phenyl)9,9-spirobifluorene (spiro-NPB) and 9,9-bis(4-(N,N-bis-biphenyl-4-yl-amino)phenyl-9Hfluorene, benzidine compounds such as N,N'-bis(naphthalen-1-yl)-N,N'-bis(phenyl)benzidine and porphyrin compounds such as copper phthalocyanines. In addition, polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PE-DOT/PSS. Preferred examples of a material of the hole injecting layer are a porphyrin compound, an aromatic tertiary amine compound, or a styrylamine compound. Particularly preferable examples include an aromatic tertiary amine compound such as hexacyanohexaazatriphenylene (HAT). In addition, dibenzofurane and dibenzothiophene compounds mentioned as host below, such as, for example, 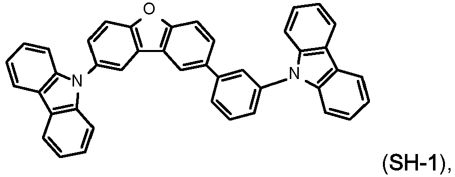 may be used as hole transport material and exciton blocker material.

In a preferred embodiment it is possible to use metal carbene complexes as hole transport materials. Suitable carbene complexes are, for example, carbene complexes as described in WO2005/019373A2, WO2006/056418 A2, WO2005/113704, WO2007/115970, WO2007/115981, WO2008/000727 and WO2014147134. One example of a suitable carbene complex is Ir(DPBIC)₃ with the formula: 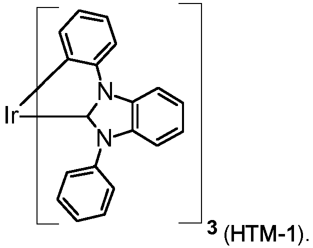 Another example of a suitable carbene complex is Ir(ABIC)₃ with the Ia:

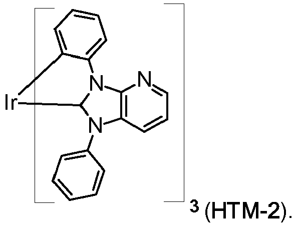

The hole-transporting layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, 2003, 359 (p-doped organic layers); A. G. Werner, F. Li, K. Harada, M. Pfeiffer, T. Fritz, K. Leo, Appl. Phys. Lett., Vol. 82, No. 25, 2003, 4495 and Pfeiffer et al., Organic Electronics 2003, 4, 89 - 103 and K. Walzer, B. Maennig, M. Pfeiffer, K. Leo, Chem. Soc. Rev. 2007, 107, 1233. For example it is possible to use mixtures in the hole-transporting layer, in particular mixtures which lead to electrical p-doping of the hole-transporting layer. p-Doping is achieved by the addition of oxidizing materials. These mixtures may, for example, be the following mixtures: mixtures of the abovementioned hole transport materials with at least one metal oxide, for example MoO₂, MoO₃, WOₓ, ReO₃ and/or V₂O₅, preferably MoO₃ and/or ReO₃, or mixtures comprising the aforementioned hole transport materials and one or more compounds selected from 7,7,8,8-tetracyanoquinodimethane (TCNQ), 2,3,5,6-tetrafluoro-7,7,8,8-tetracyanoquinodimethane (F₄-TCNQ), 2,5-bis(2-hydroxyethoxy)-7,7,8,8-tetracyanoquinodimethane, bis(tetra-n-butylammonium)tetracyanodiphenoquinodimethane, 2,5-dimethyl-7,7,8,8-tetracyanoquinodimethane, tetracyanoethylene, 11,11,12,12-tetracyanonaphtho2,6-quinodimethane, 2-fluoro-7,7,8,8-tetracyanoquino-dimethane, 2,5-difluoro-7,7,8,8etracyanoquinodimethane, dicyanomethylene-1,3,4,5,7,8-hexafluoro-6H-naphthalen-2-ylidene)malononitrile (F₆-TNAP), Mo(tfd)₃ (from Kahn et al., J. Am. Chem. Soc. 2009, 131 (35), 12530-12531), compounds as described in EP1988587,
US2008265216, EP2180029, US20100102709, WO2010132236, EP2180029 and quinone compounds as mentioned in EP2401254. Preferred mixtures comprise the aforementioned carbene complexes, such as, for example, the carbene complexes **HTM-1** and **HTM-2**, and MoO₃ and/or ReO₃. In a particularly preferred embodiment the hole transport layer comprises from 0.1 to 10 wt % of MoO₃ and 90 to 99.9 wt % carbene complex, especially of a carbene complex **HTM-1** and **HTM-2**, wherein the total amount of the MoO₃ and the carbene complex is 100 wt %. In another particularly preferred embodiment the hole transport layer comprises from 0.1 to 10 wt % of MoO₃ and 90 to 99.9 wt % dibenzofurane compound, especially compound (**SH-1**), wherein the total amount of the MoO₃ and the dibenzofurane compound is 100 wt %.

### Exciton blocking layer (d):

Blocking layers may be used to reduce the number of charge carriers (electrons or holes) and/or excitons that leave the emissive layer. An electron/exciton blocking layer (d) may be disposed between the first emitting layer (e) and the hole transport layer (c), to block electrons from emitting layer (e) in the direction of hole transport layer (c). Blocking layers may also be used to block excitons from diffusing out of the emissive layer. Suitable metal complexes for use as electron/exciton blocker material are, for example, carbene complexes as described in WO2005/019373A2, WO2006/056418A2, WO2005/113704, WO2007/115970, WO2007/115981, WO2008/000727 and WO2014147134. Explicit reference is made here to the disclosure of the WO applications cited, and these disclosures shall be considered to be incorporated into the content of the present application. One example of a suitable carbene complex is compound **HTM-1.** Another example of a suitable carbene complex is compound **HTM-2.** In addition, all hole transport materials having sufficient high triplet energy are suitable exciton blocking materials. Additional examples of hole transporting materials having sufficient high triplet energy are **(SH-1) and (SH-11)**.

### Emitting layer (e)

The light-emitting layer (e) comprises at least one emitter material. In principle, it may be a fluorescence or phosphorescence emitter, suitable emitter materials being known to those skilled in the art. The at least one emitter material is preferably a phosphorescence emitter. The phosphorescence emitter compounds used with preference are based on metal complexes, and especially the complexes of the metals Ru, Rh, Ir, Pd and Pt, in particular the complexes of Ir, have gained significance. The compounds of the formula I can be used as the matrix in the light-emitting layer.

Suitable metal complexes for use in the inventive OLEDs are described, for example, in documents WO 02/60910 A1, US 2001/0015432 A1, US 2001/0019782 A1,
US 2002/0055014 A1, US 2002/0024293 A1, US 2002/0048689 A1, EP 1 191 612 A2,
EP 1 191 613 A2, EP 1 211 257 A2, US 2002/0094453 A1, WO 02/02714 A2,
WO 00/70655 A2, WO 01/41512 A1, WO 02/15645 A1, WO 2005/019373 A2,
WO 2005/113704 A2, WO 2006/115301 A1, WO 2006/067074 A1, WO 2006/056418,
WO 2006121811 A1, WO 2007095118 A2, WO 2007/115970, WO 2007/115981,
WO 2008/000727, WO2010129323, WO2010056669, WO10086089, US2011/0057559,
WO2011/106344, US2011/0233528, WO2012/048266, WO2012/172482, WO2015000955 and PCT/EP2014/066272.

Further suitable metal complexes are the commercially available metal complexes tris(2-phenylpyridine)iridium(III), iridium(III) tris(2-(4-tolyl)pyridinato-N,C^{2'}), bis(2-phenylpyridine)(acetylacetonato)iridium(III), iridium(III) tris(1-phenylisoquinoline), iridium(III) bis(2,2'-benzothienyl)pyridinato-N,C^{3'})(acetylacetonate), tris(2-phenylquinoline)iridium(III), iridium(III) bis(2-(4,6-difluorophenyl)pyridinato-N,C²)picolinate, iridium(III) bis(1-phenylisoquinoline)(acetylacetonate), bis(2-phenylquinoline)(acetylacetonato)iridium(III), iridium(III) bis(di-benzo[f,h]quinoxaline)(acetylacetonate), iridium(III) bis(2-methyldibenzo[f,h]quinoxaline)(acetylacetonate) and tris(3-methyl-1-phenyl-4-trimethylacetyl-5-pyrazolino)terbium(III), bis[1-(9,9-dimethyl-9H-fluoren-2-yl)isoquinoline](acetylacetonato)iridium(III), bis(2-phenylbenzothiazolato)(acetylacetonato)iridium(III), bis(2-(9,9-dihexylfluorenyl)-1-pyridine)(acetylacetonato)iridium(III), bis(2-benzo[b]thiophen-2-yl-pyridine)(acetylacetonato)iridium(III).

In addition, the following commercially available materials are suitable: tris(dibenzoylacetonato)mono(phenanthroline)europium(III), tris(dibenzoylmethane)-mono(phenanthroline)europium(III), tris(dibenzoylmethane)mono(5-aminophenanthroline)-europium(III), tris(di-2-naphthoylmethane)mono(phenanthroline)europium(III), tris(4-bromobenzoylmethane)mono(phenanthroline)europium(III), tris(di(biphenyl)methane)-mono(phenanthroline)europium(III), tris(dibenzoylmethane)mono(4,7-diphenylphenanthroline)europium(III), tris(dibenzoylmethane)mono(4,7-di-methylphenanthroline)europium(III), tris(dibenzoylmethane)mono(4,7-dimethylphenanthrolinedisulfonic acid)europium(III) disodium salt, tris[di(4-(2-(2-ethoxyethoxy)ethoxy)-benzoylmethane)]mono(phenanthroline)europium(III) and tris[di[4-(2-(2-ethoxy-ethoxy)ethoxy)benzoylmethane)]mono(5-aminophenanthroline)europium(III), osmium(II) bis(3-(trifluoromethyl)-5-(4-tert-butylpyridyl)-1,2,4-triazolato)diphenylmethylphosphine, osmium(II) bis(3-(trifluoromethyl)-5-(2-pyridyl)-1,2,4-triazole)dimethylphenylphosphine, osmium(II) bis(3-(trifluoromethyl)-5-(4-tert-butylpyridyl)-1,2,4-triazolato)dimethylphenylphosphine, osmium(II) bis(3-(trifluoromethyl)-5-(2-pyridyl)-pyrazolato)dimethylphenylphosphine, tris[4,4'-di-tert-butyl(2,2')-bipyridine]ruthenium(III), osmium(II) bis(2-(9,9-dibutylfluorenyl)-1-isoquinoline(acetylacetonate).

Preferred phosphorescence emitters are carbene complexes. Suitable phosphorescent blue emitters are specified in the following publications: WO2006/056418A2, WO2005/113704, WO2007/115970, WO2007/115981, WO2008/000727, WO2009050281, WO2009050290, WO2011051404, US2011/057559 WO2011/073149, WO2012/121936A2, US2012/0305894A1, WO2012/170571, WO2012/170461, WO2012/170463, WO2006/121811, WO2007/095118, WO2008/156879, WO2008/156879, WO2010/068876, US2011/0057559, WO2011/106344, US2011/0233528, WO2012/048266, WO2012/172482, WO2015000955 and PCT/EP2014/066272.

Preferably, the light emitting layer (e) comprises at least one carbine complex as phosphorescence emitter. Suitable carbine complexes are, for example, compounds of the formula 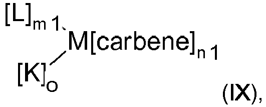 which are described in WO 2005/019373 A2, wherein the symbols have the following meanings:
M is a metal atom selected from the group consisting of Co, Rh, Ir, Nb, Pd, Pt, Fe, Ru, Os, Cr, Mo, W, Mn, Tc, Re, Cu, Ag and Au in any oxidation state possible for the respective metal atom;
Carbene is a carbene ligand which may be uncharged or monoanionic and monodentate, bidentate or tridentate, with the carbene ligand also being able to be a biscarbene or triscarbene ligand;
L is a monoanionic or dianionic ligand, which may be monodentate or bidentate;
K is an uncharged monodentate or bidentate ligand selected from the group consisting of phosphines; phosphonates and derivatives thereof, arsenates and derivatives thereof;
phosphites; CO; pyridines; nitriles and conjugated dienes which form a π complex with M¹; n1 is the number of carbene ligands, where n1 is at least 1 and when n1 > 1 the carbene ligands in the complex of the formula I can be identical or different;
m1 is the number of ligands L, where m1 can be 0 or ≥ 1 and when m1 > 1 the ligands L can be identical or different;
o is the number of ligands K, where o can be 0 or ≥ 1 and when o > 1 the ligands K can be identical or different;
where the sum n1 + m1 + o is dependent on the oxidation state and coordination number of the metal atom and on the denticity of the ligands carbene, L and K and also on the charge on the ligands, carbene and L, with the proviso that n1 is at least 1.

More preferred are metal-carbene complexes of the general formula 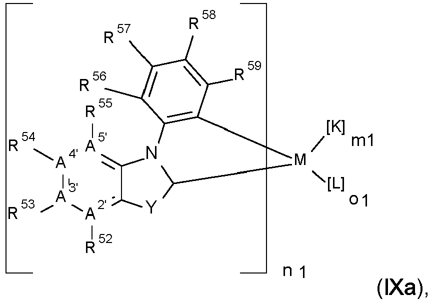 which are described in WO2011/073149, where M, n1, Y, A^{2'}, A^{3'}, A^{4'}, A^{5'}, R⁵¹, R⁵², R⁵³, R⁵⁴, R⁵⁵, R⁵⁶, R⁵⁷, R⁵⁸, R⁵⁹, K, L, m1 and o1 are each defined as follows:
M is Ir, or Pt,
n1 is an integer selected from 1, 2 and 3,
Y is NR⁵¹, O, S or C(R²⁵)₂,
A^{2'}, A^{3'}, A^{4'}, and A^{5'} are each independently N or C, where 2 A' = nitrogen atoms and at least one carbon atom is present between two nitrogen atoms in the ring,
R⁵¹ is a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
R⁵², R⁵³, R⁵⁴ and R⁵⁵ are each, if A^{2'}, A^{3'}, A^{4'} and/or A^{5'} is N, a free electron pair, or, if A^{2'}, A^{3'}, A^{4'} and/or A^{5'} is C, each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or R⁵³ and R⁵⁴ together with A^{3'} and A^{4'} form an optionally substituted, unsaturated ring optionally interrupted by at least one further heteroatom and having a total of 5 to 18 carbon atoms and/or heteroatoms,
R⁵⁶, R⁵⁷, R⁵⁸ and R⁵⁹ are each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, cycloheteroalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or
R⁵⁶ and R⁵⁷, R⁵⁷ and R⁵⁸ or R⁵⁸ and R⁵⁹, together with the carbon atoms to which they are bonded, form a saturated, unsaturated or aromatic, optionally substituted ring optionally interrupted by at least one heteroatom and having a total of 5 to 18 carbon atoms and/or heteroatoms, and/or
if A^{5'} is C, R⁵⁵ and R⁵⁶ together form a saturated or unsaturated, linear or branched bridge optionally comprising heteroatoms, an aromatic unit, heteroaromatic unit and/or functional groups and having a total of 1 to 30 carbon atoms and/or heteroatoms, to which is optionally fused a substituted or unsubstituted, five- to eight-membered ring comprising carbon atoms and/or heteroatoms,
R²⁵ is independently a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
K is an uncharged mono- or bidentate ligand,
L is a mono- or dianionic ligand, preferably monoanionic ligand, which may be mono- or bidentate,
m1 is 0, 1 or 2, where, when m1 is 2, the K ligands may be the same or different,
o1 is 0, 1 or 2, where, when o1 is 2, the L ligands may be the same or different.

The compound of formula **IX** is preferably a compound of the formula:

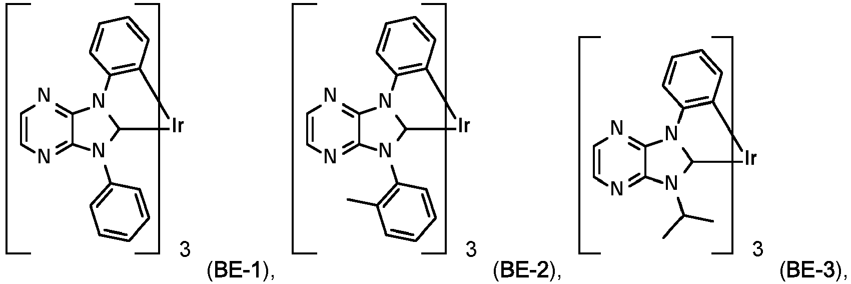

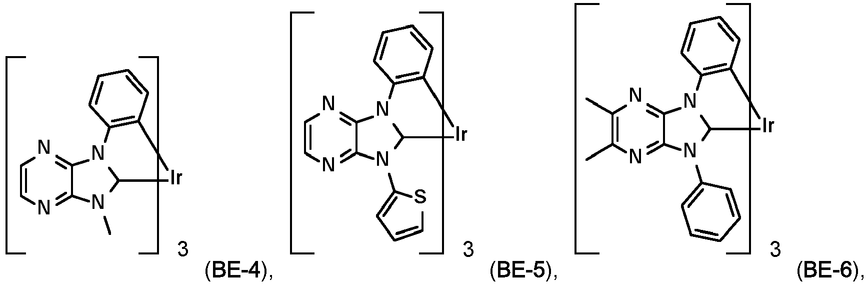

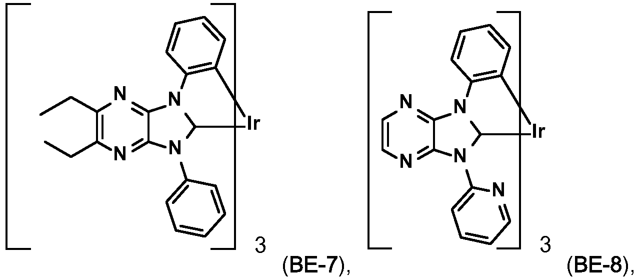

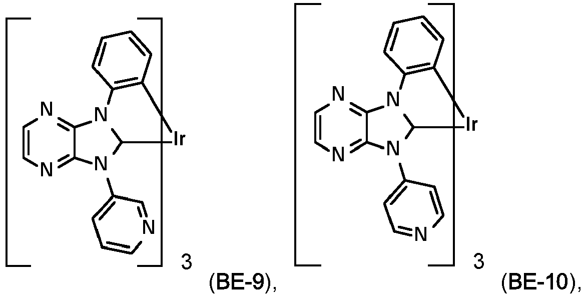

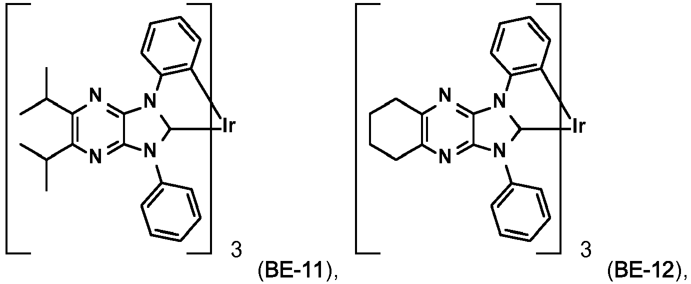

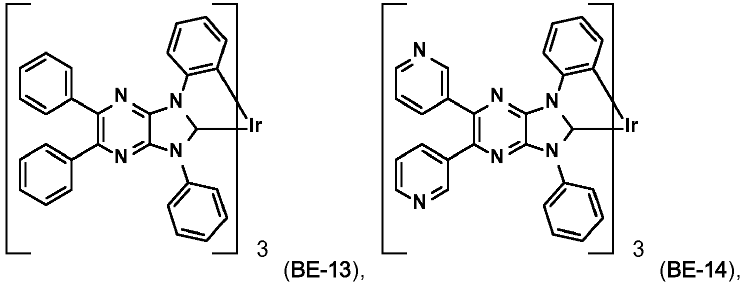

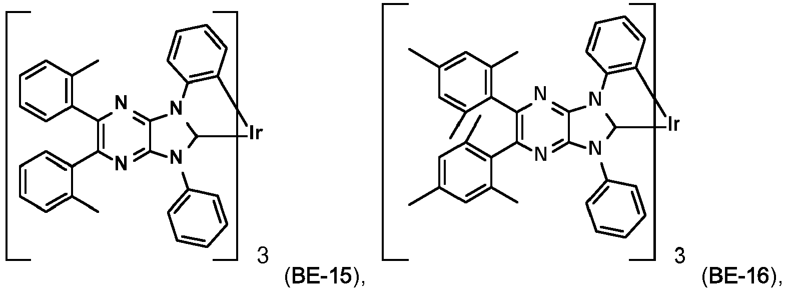

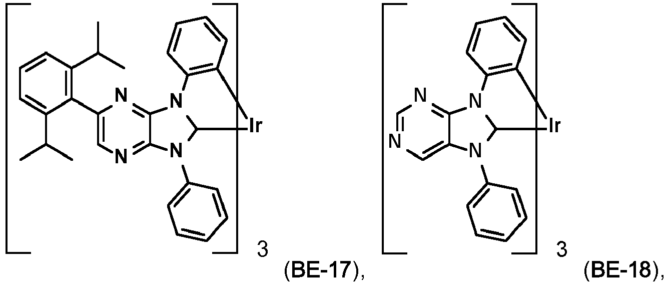

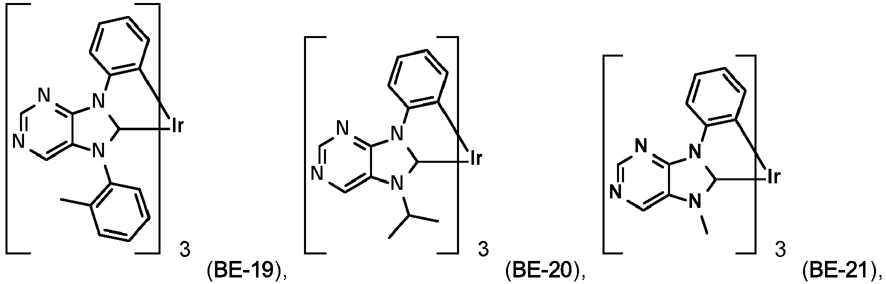

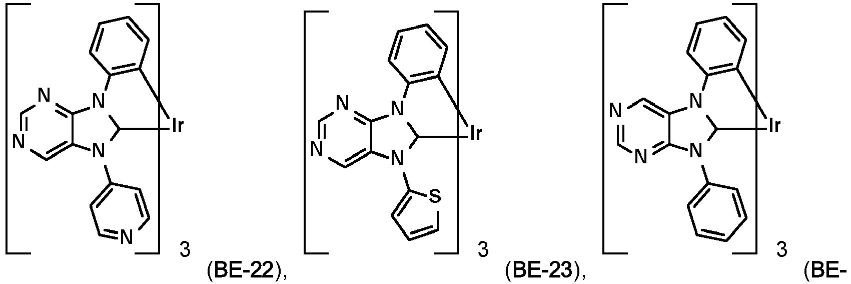

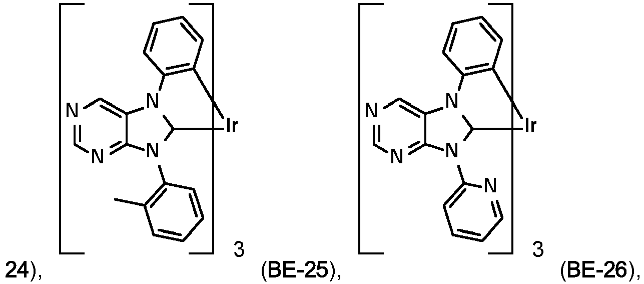

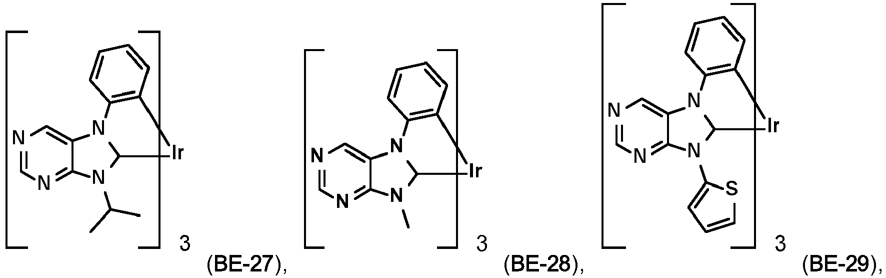

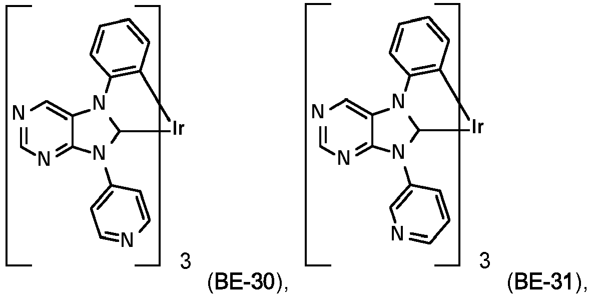

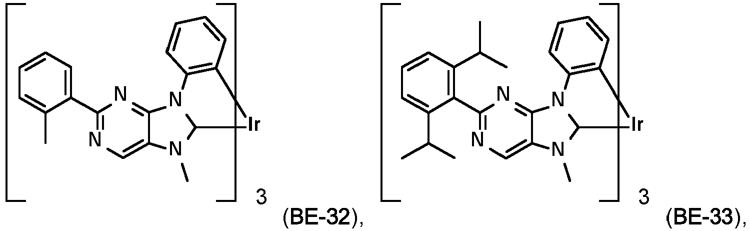

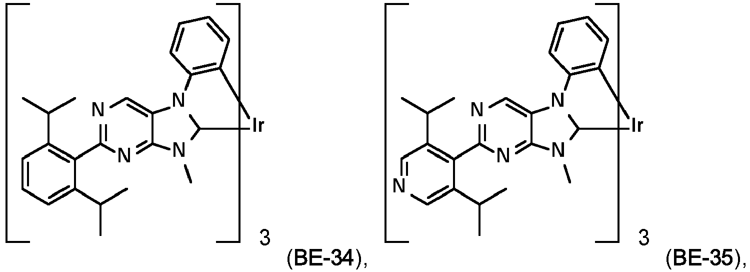

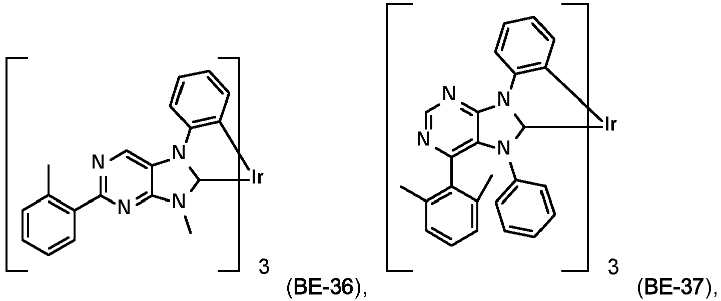

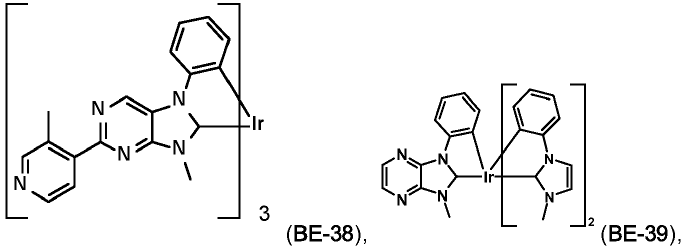

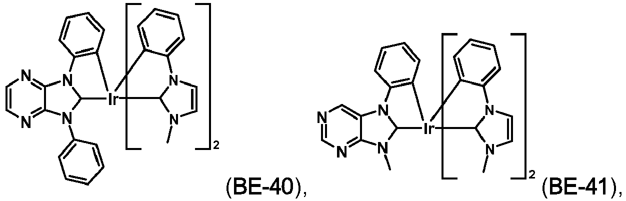

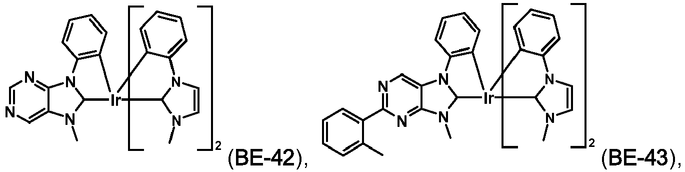

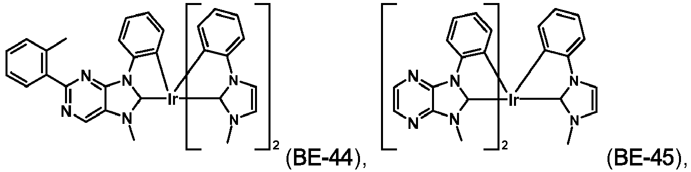

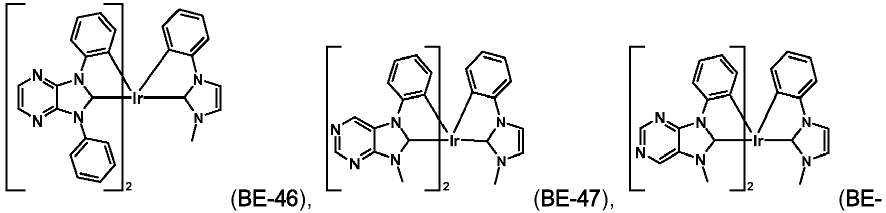

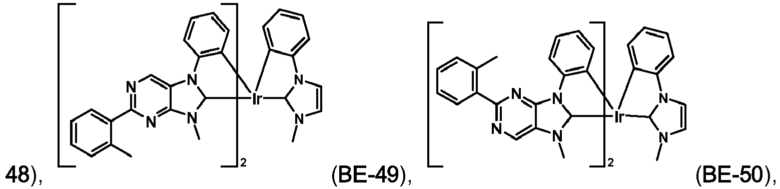

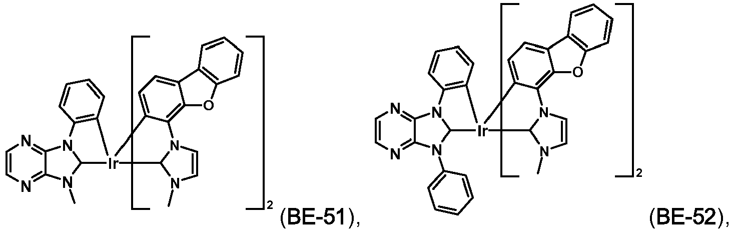

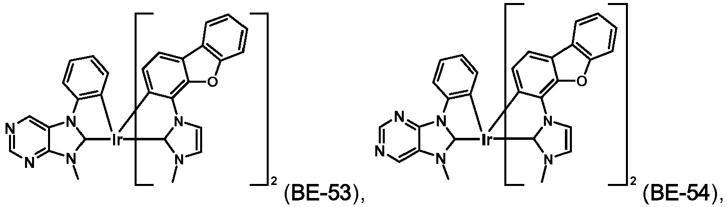

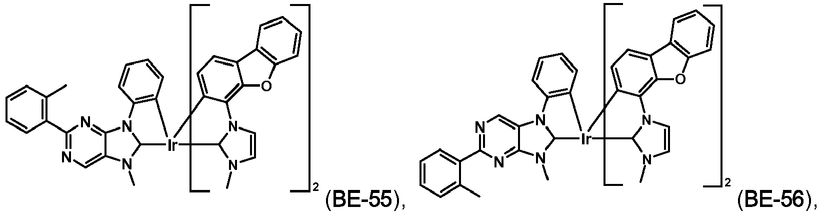

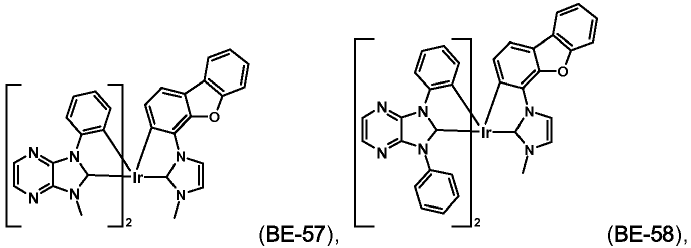

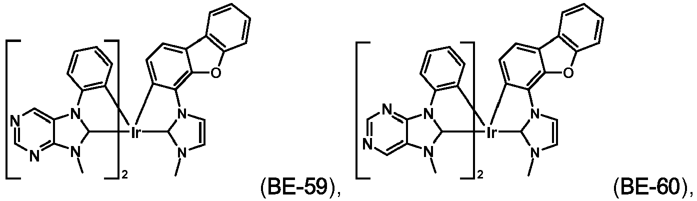

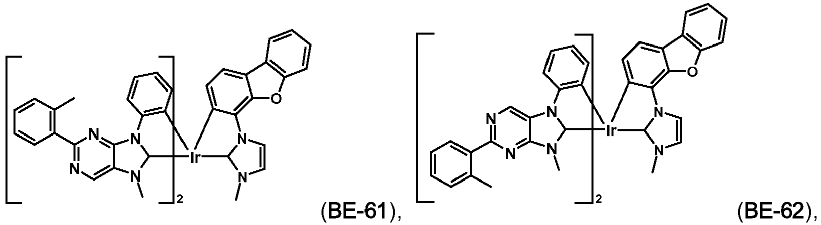

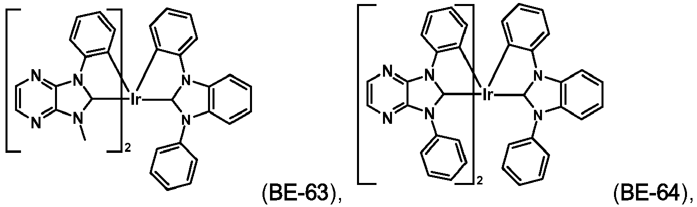

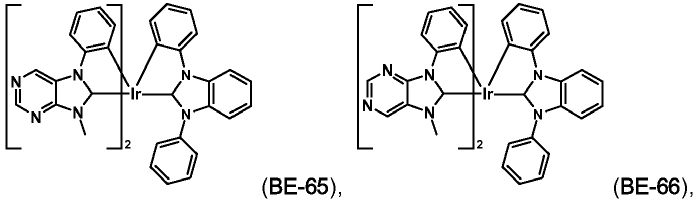

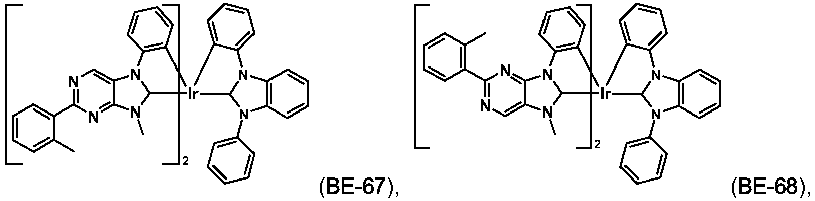

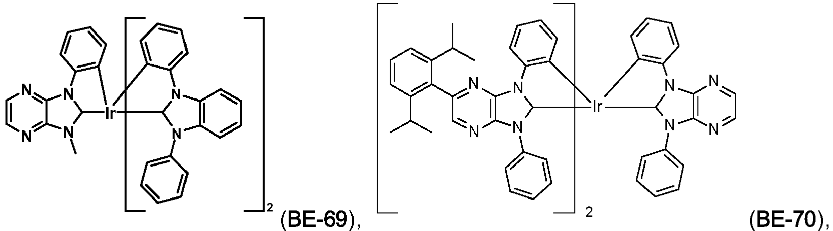

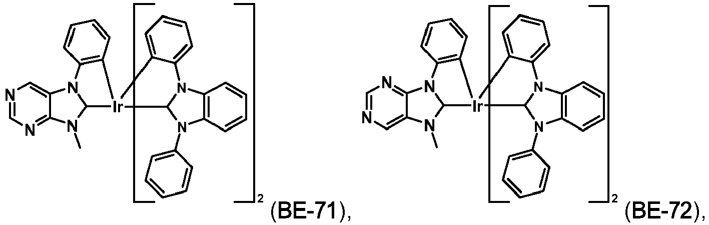

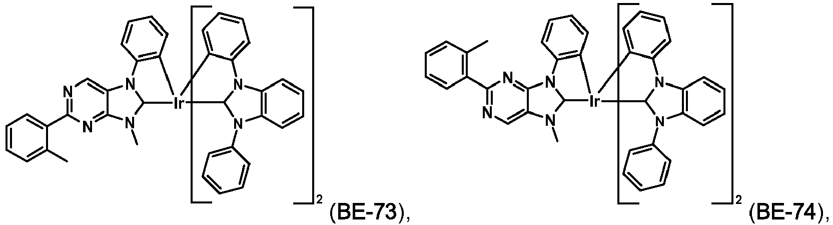

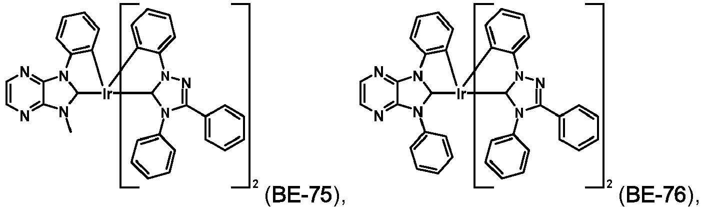

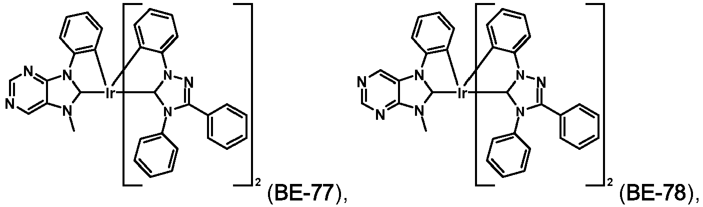

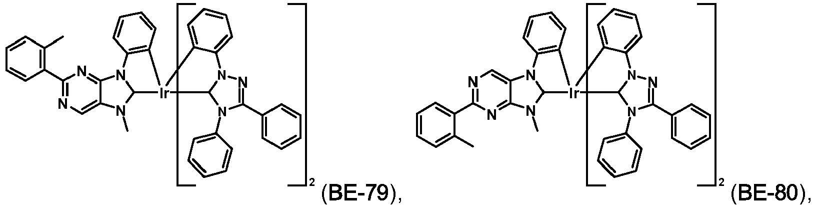

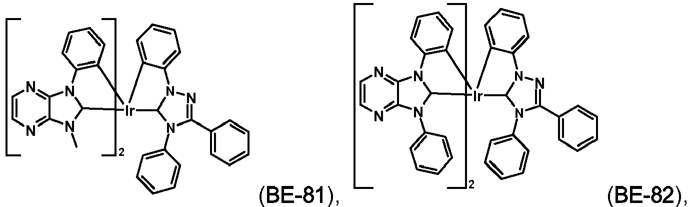

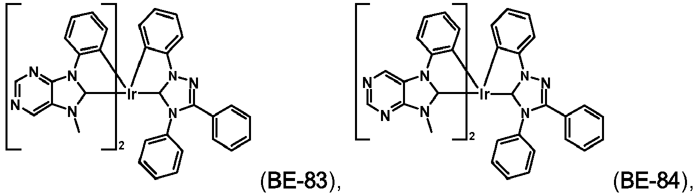

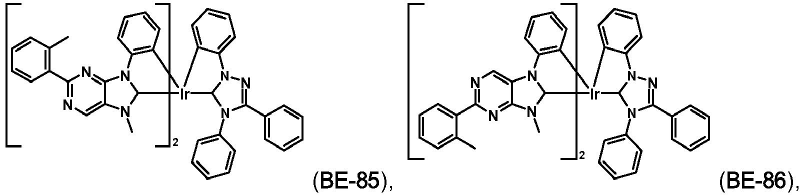

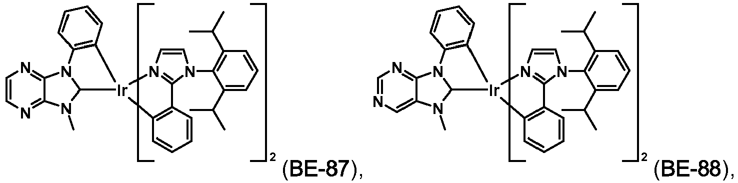

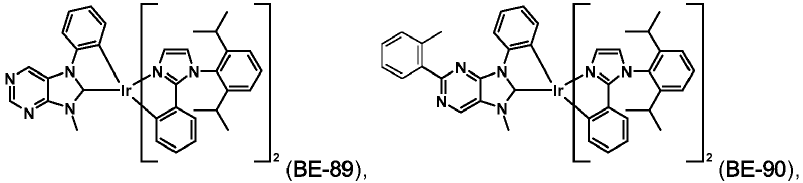

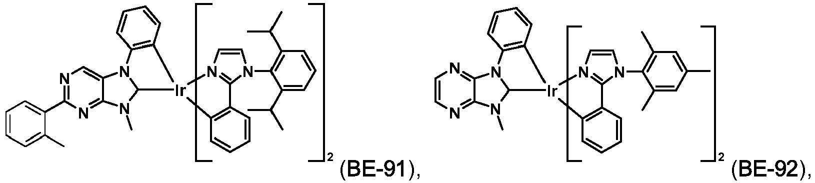

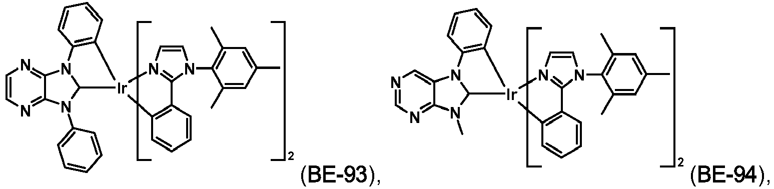

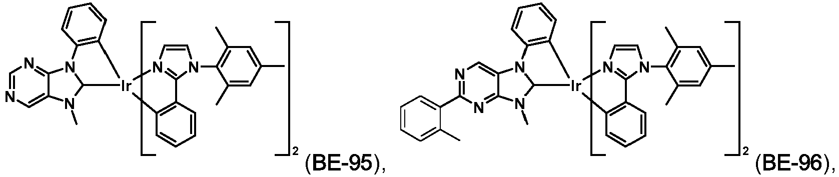

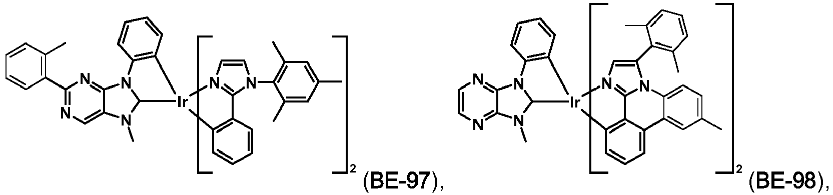

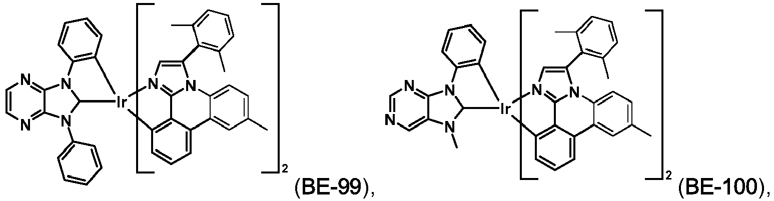

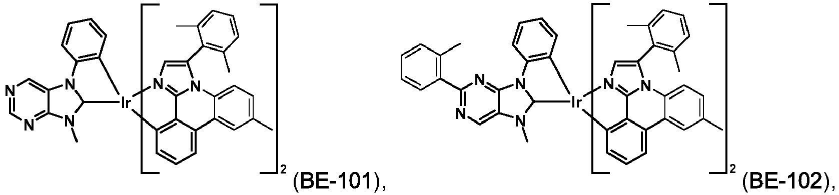

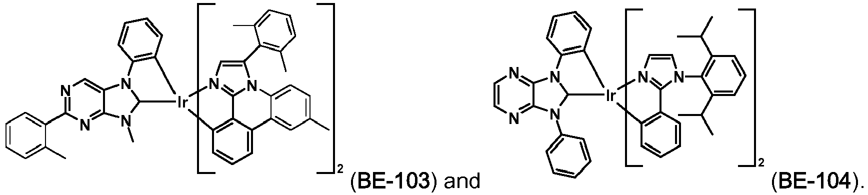

Further suitable non-carbene emitter materials are mentioned below: 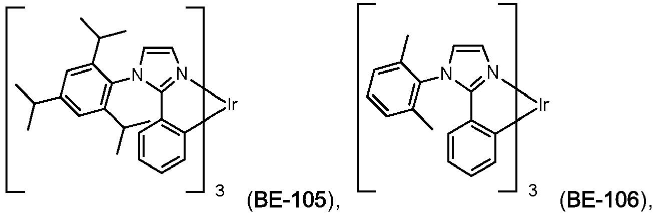 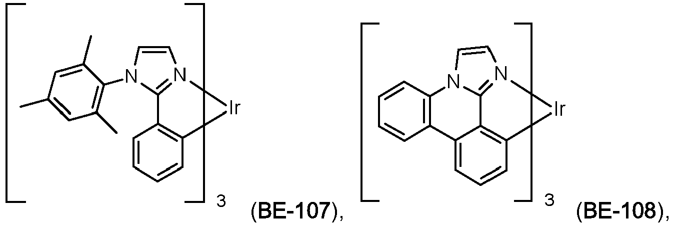 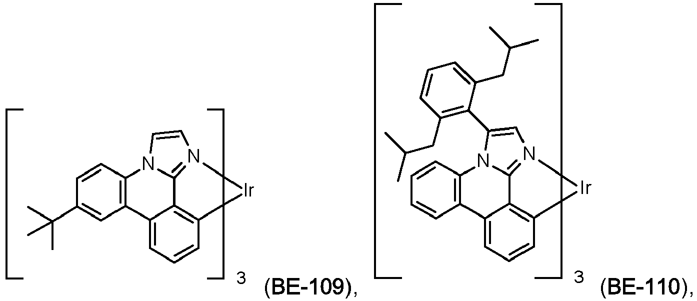 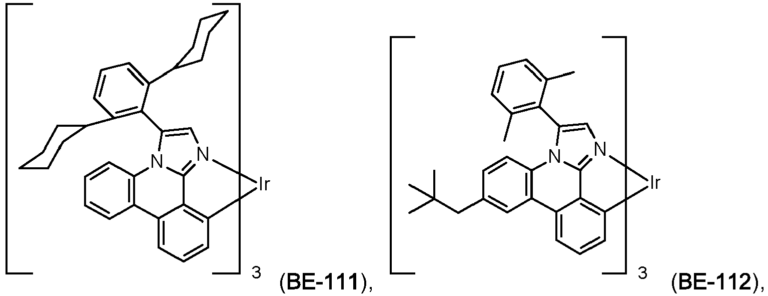 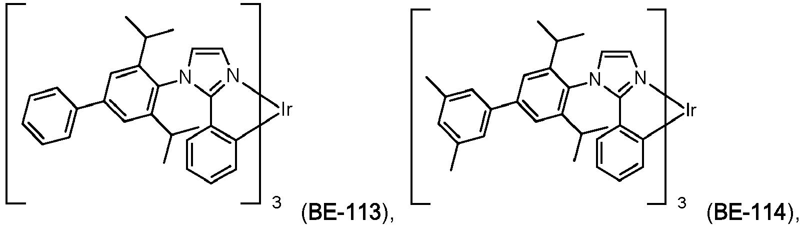 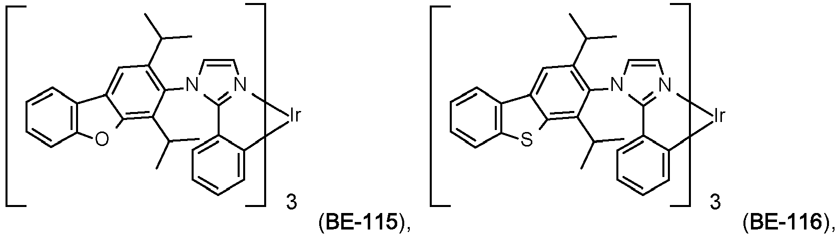 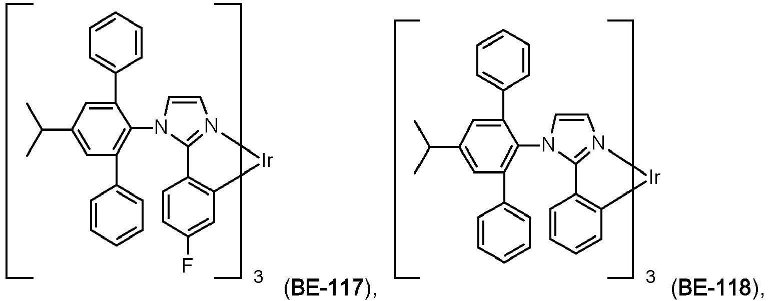 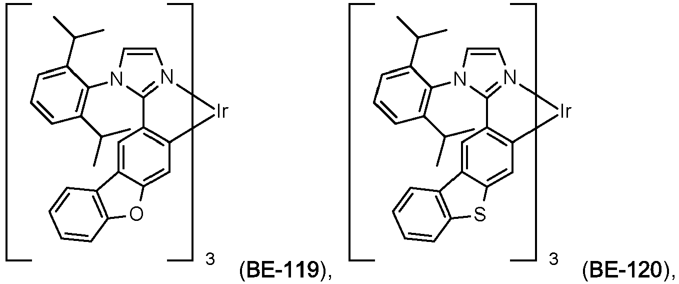 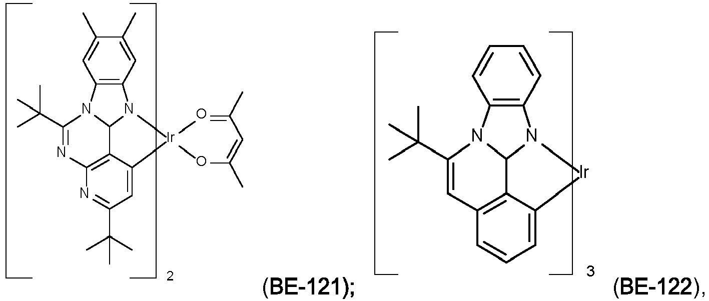 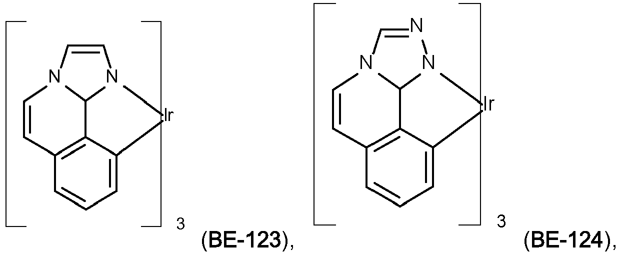 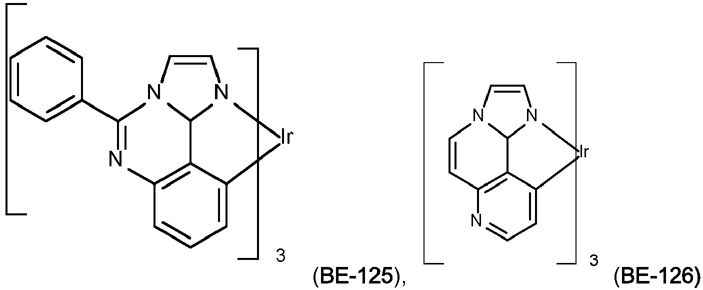 and

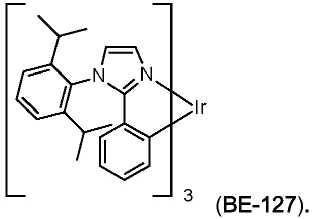

The compound of formula **IX** is more preferably a compound (**BE-1**), (**BE-2**), (**BE-7**), (**BE-12**), (**BE-16**), (**BE-64**), or (**BE-70**). The most preferred phosphorescent blue emitters are compounds (**BE-1**) and (**BE-12**).

The homoleptic metal-carbene complexes may be present in the form of facial or meridional isomers, preference being given to the facial isomers.

Suitable carbene complexes of formula (**IX**) and their preparation process are, for example, described in WO2011/073149.

The compounds of the present invention can also be used as host for phosphorescent green emitters. Suitable phosphorescent green emitters are, for example, specified in the following publications: WO2006014599, WO20080220265, WO2009073245, WO2010027583, WO2010028151, US20110227049, WO2011090535, WO2012/08881, WO20100056669, WO20100118029, WO20100244004, WO2011109042, WO2012166608, US20120292600, EP2551933A1; US6687266, US20070190359, US20070190359, US20060008670; WO2006098460, US20110210316, WO 2012053627; US6921915, US20090039776; JP2007123392 and European patent application no. 14180422.9.

Examples of suitable phosphorescent green emitters are shown below:

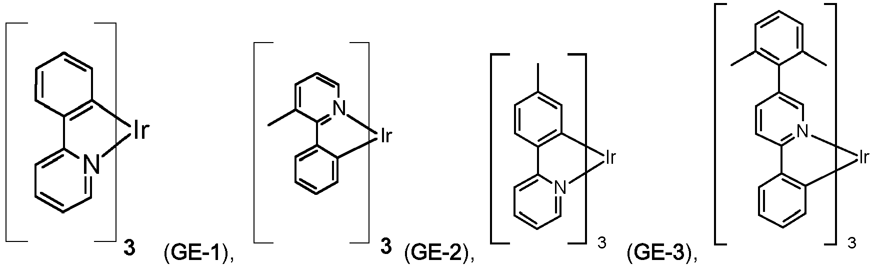

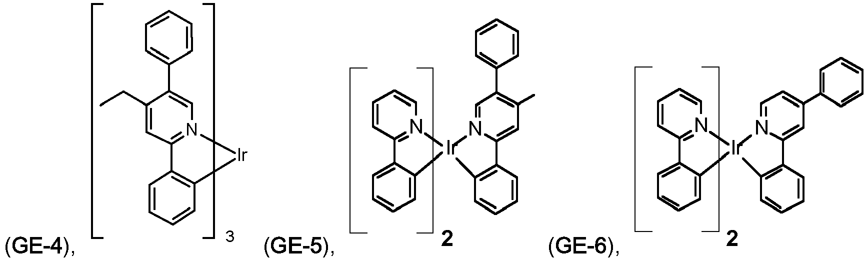

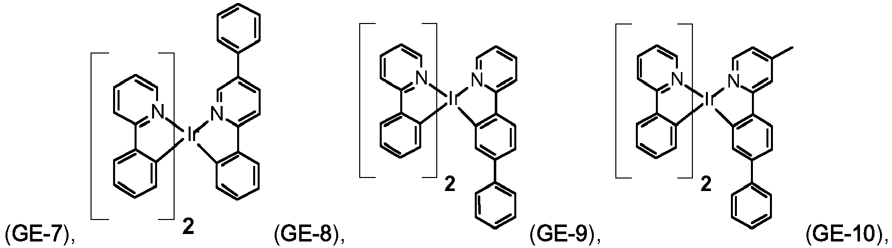

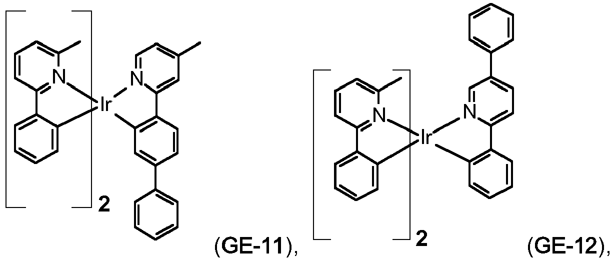

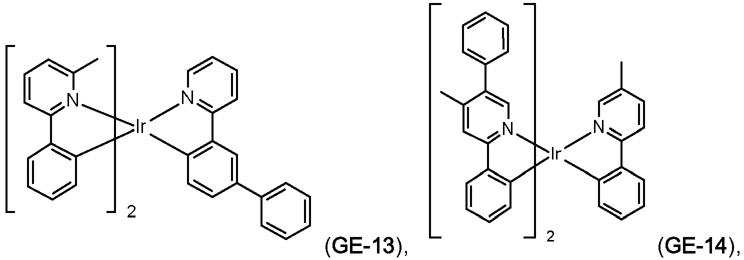

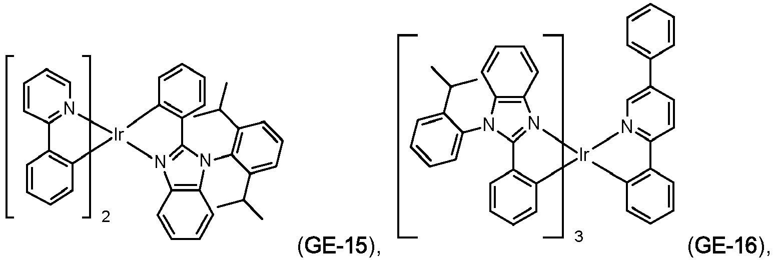

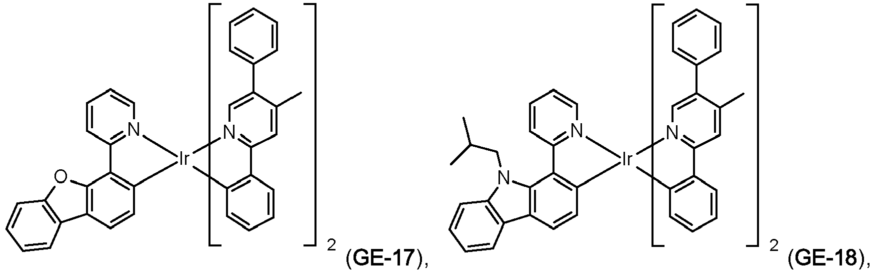

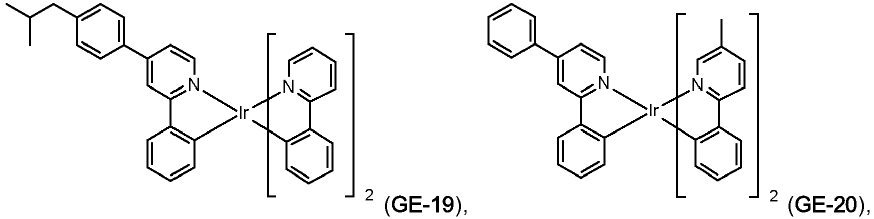

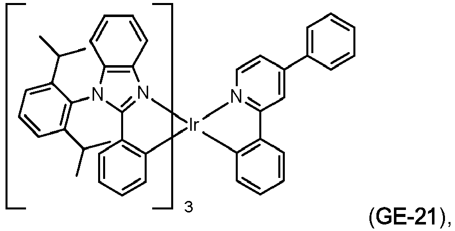

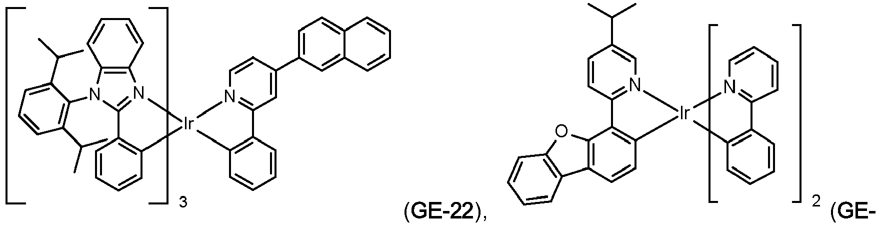

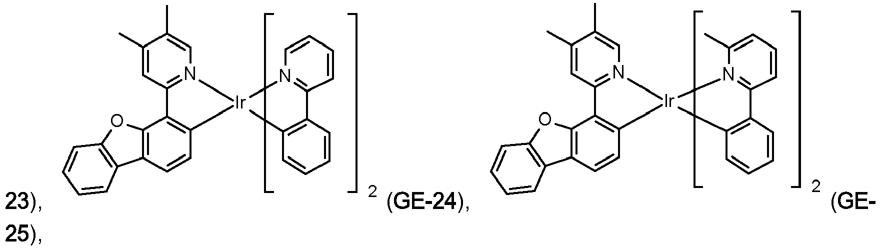

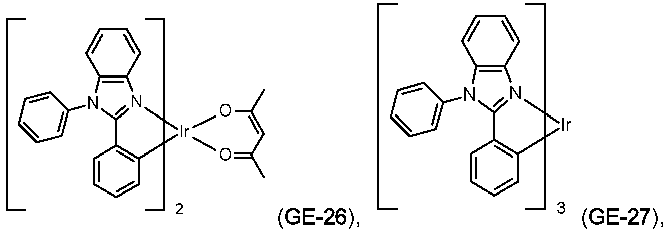

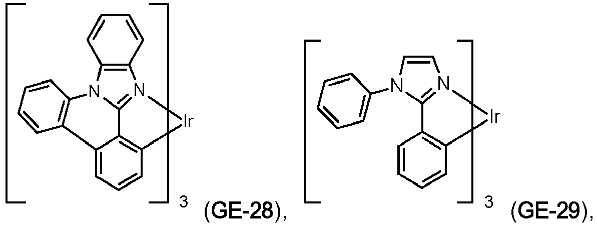

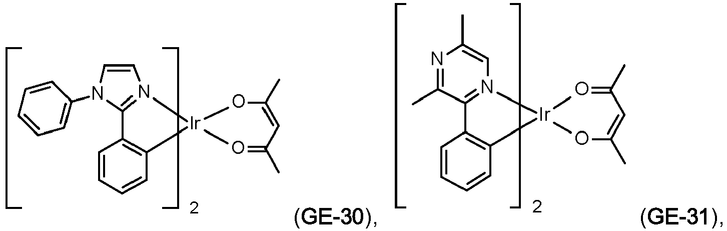

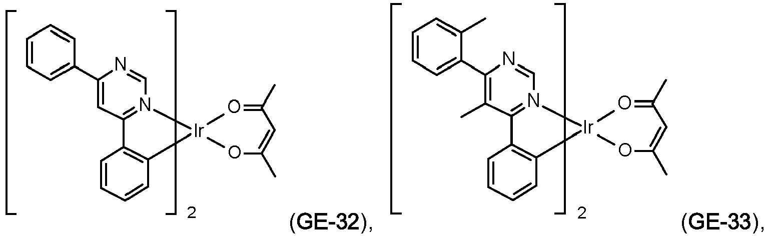

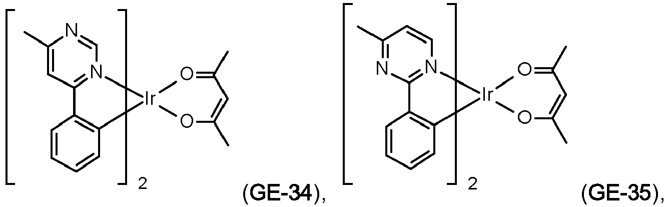

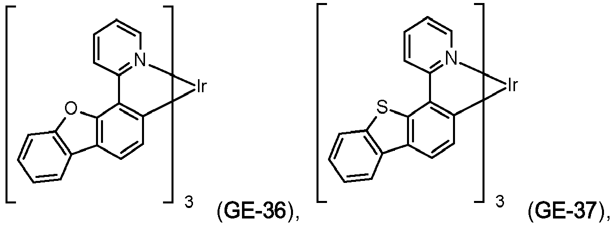

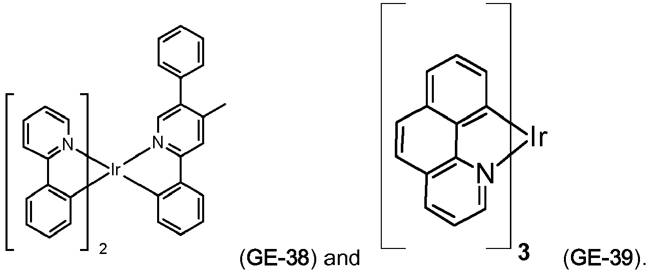

### Host (matrix) materials

The light-emitting layer may comprise further components in addition to the emitter material. For example, a fluroescent dye may be present in the light-emitting layer in order to alter the emission color of the emitter material. In addition - in a preferred embodiment - a matrix material can be used. This matrix material may be a polymer, for example poly(N-vinylcarbazole) or polysilane. The matrix material may, however, be a small molecule, for example 4,4'-N,N'-dicarbazolebiphenyl (CDP=CBP) or tertiary aromatic amines, for example TCTA.

In a preferred embodiment of the present invention, at least one compound of the formula I, especially a compound of the formula **II, or III** is used as matrix material. Examples of preferred compounds of formula I are compounds **A-1** to **A-47 as well as B-1** to **B-47.**

In a preferred embodiment, the light-emitting layer is formed from 2 to 40% by weight, preferably 5 to 35% by weight, of at least one of the aforementioned emitter materials and 60 to 98% by weight, preferably 75 to 95% by weight, of at least one of the aforementioned, or belowmentioned matrix materials - in one embodiment at least one compound of the formula I - where the sum total of the emitter material and of the matrix material adds up to 100% by weight.

Suitable metal complexes for use together with the compounds of the formula I as matrix material in OLEDs are, for example, also carbene complexes as described in WO 2005/019373 A2, WO 2006/056418 A2, WO 2005/113704, WO 2007/115970, WO 2007/115981, WO 2008/000727 and WO2014147134.

Further suitable host materials, which may be small molecules or (co)polymers of the small molecules mentioned, are specified in the following publications: WO2007108459 (H-1 to H-37), preferably H-20 to H-22 and H-32 to H-37, most preferably H-20, H-32, H-36, H-37, WO2008035571 A1 (Host 1 to Host 6), JP2010135467 (compounds 1 to 46 and Host-1 to Host-39 and Host-43), WO2009008100 compounds No.1 to No.67, preferably No.3, No.4, No.7 to No. 12, No.55, No.59, No. 63 to No.67, more preferably No. 4, No. 8 to No. 12, No. 55, No. 59, No.64, No.65, and No. 67, WO2009008099 compounds No. 1 to No. 110, WO2008140114 compounds 1-1 to 1-50, WO2008090912 compounds OC-7 to OC-36 and the polymers of Mo-42 to Mo-51, JP2008084913 H-1 to H-70, WO2007077810 compounds 1 to 44, preferably 1, 2, 4-6, 8, 19-22, 26, 28-30, 32, 36, 39-44, WO201001830 the polymers of monomers 1-1 to 1-9, preferably of 1-3, 1-7, and 1-9, WO2008029729 the (polymers of) compounds 1-1 to 1-36, WO20100443342 HS-1 to HS-101 and BH-1 to BH-17, preferably BH-1 to BH-17, JP2009182298 the (co)polymers based on the monomers 1 to 75, JP2009170764, JP2009135183 the (co)polymers based on the monomers 1-14, WO2009063757 preferably the (co)polymers based on the monomers 1-1 to 1-26, WO2008146838 the compounds a-1 to a-43 and 1-1 to 1-46, JP2008207520 the (co)polymers based on the monomers 1-1 to 1-26, JP2008066569 the (co)polymers based on the monomers 1-1 to 1-16, WO2008029652 the (co)polymers based on the monomers 1-1 to 1-52, WO2007114244 the (co)polymers based on the monomers 1-1 to 1-18, JP2010040830 the compounds HA-1 to HA-20, HB-1 to HB-16, HC-1 to HC-23 and the (co)polymers based on the monomers HD-1 to HD-12, JP2009021336, WO2010090077 the compounds 1 to 55, WO2010079678 the compounds H1 to H42, WO2010067746, WO2010044342 the compounds HS-1 to HS-101 and Poly-1 to Poly-4, JP2010114180 the compounds PH-1 to PH-36, US2009284138 the compounds 1 to 111 and H1 to H71, WO2008072596 the compounds 1 to 45, JP2010021336 the compounds H-1 to H-38, preferably H-1, WO2010004877 the compounds H-1 to H-60, JP2009267255 the compounds 1-1 to 1-105, WO2009104488 the compounds 1-1 to 1-38, WO2009086028, US2009153034, US2009134784, WO2009084413 the compounds 2-1 to 2-56, JP2009114369 the compounds 2-1 to 2-40, JP2009114370 the compounds 1 to 67, WO2009060742 the compounds 2-1 to 2-56, WO2009060757 the compounds 1-1 to 1-76, WO2009060780 the compounds 1-1 to 1-70, WO2009060779 the compounds 1-1 to 1-42, WO2008156105 the compounds 1 to 54, JP2009059767 the compounds 1 to 20, JP2008074939 the compounds 1 to 256, JP2008021687 the compounds 1 to 50, WO2007119816 the compounds 1 to 37, WO2010087222 the compounds H-1 to H-31, WO2010095564 the compounds HOST-1 to HOST-61, WO2007108362, WO2009003898, WO2009003919, WO2010040777, US2007224446, WO06128800, WO2012014621, WO2012105310, WO2012/130709, WO2014/009317 (in particular compound A-24), WO2014/044722 and WO2014/072320 (in particular page 25 to 29 of WO2014/072320).

The above-mentioned small molecules are more preferred than the above-mentioned (co)polymers of the small molecules.

Further suitable host materials, are described in WO2011137072 (for example, 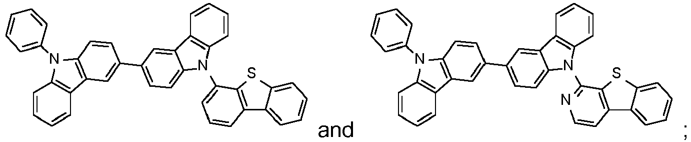 best results are achieved if said compounds are combined with 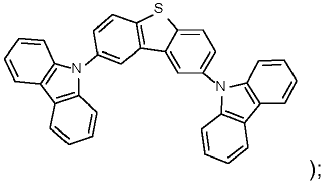 WO2012048266 (for example, 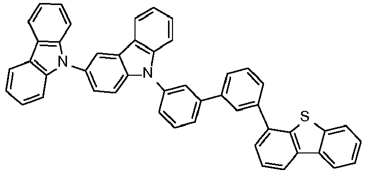 and 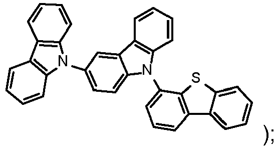 WO2012162325 (for example, 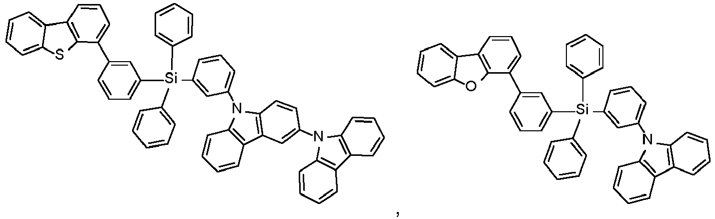 and 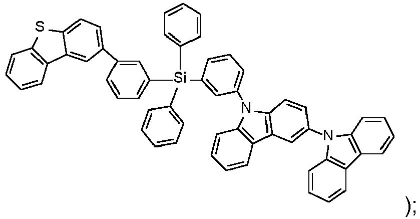 and EP2551932 (for example,

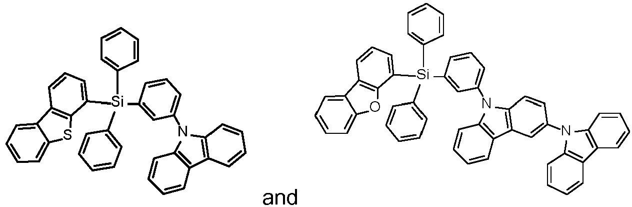

In a particularly preferred embodiment, one or more compounds of the general formula (XII) specified hereinafter are used as second host material. 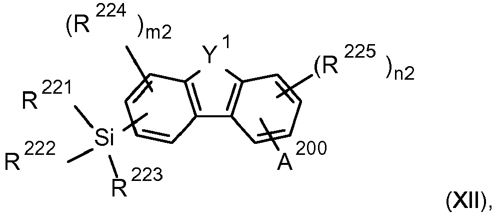 wherein
Y¹ is NR, S, O or PR;
R is aryl, heteroaryl, alkyl, cycloalkyl, or heterocycloalkyl;
A²⁰⁰ is -NR²⁰⁶R²⁰⁷, -P(O)R²⁰⁸R²⁰⁹, -PR²¹⁰R²¹¹, -S(O)₂R²¹², -S(O)R²¹³, -SR²¹⁴, or -OR²¹⁵; R²²¹, R²²² and R²²³ are independently of each other aryl, heteroaryl, alkyl, cycloalkyl, or heterocycloalkyl, wherein at least on of the groups R²²¹, R²²², or R²²³ is aryl, or heteroaryl; R²²⁴ and R²²⁵ are independently of each other alkyl, cycloalkyl, heterocycloalkyl, aryl, heteroaryl, a group A²⁰⁰, or a group having donor, or acceptor characteristics;
n2 and m2 are independently of each other 0, 1, 2, or 3;
R²⁰⁶ and R²⁰⁷ form together with the nitrogen atom a cyclic residue having 3 to 10 ring atoms, which can be unsubstituted, or which can be substituted with one, or more substituents selected from alkyl, cycloalkyl, heterocycloalkyl, aryl, heteroaryl and a group having donor, or acceptor characteristics; and/or which can be annulated with one, or more further cyclic residues having 3 to 10 ring atoms, wherein the annulated residues can be unsubstituted, or can be substituted with one, or more substituents selected from alkyl, cycloalkyl, heterocycloalkyl, aryl, heteroaryl and a group having donor, or acceptor characteristics; and R²⁰⁸, R²⁰⁹, R²¹⁰, R²¹¹, R²¹², R²¹³ R²¹⁴ und R²¹⁵ are independently of each other aryl, heteroaryl, alkyl, cycloalkyl, or heterocycloalkyl. Compounds of formula **XII**, such as, for example, 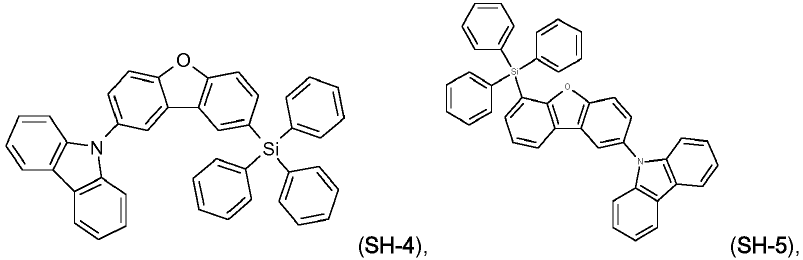 or 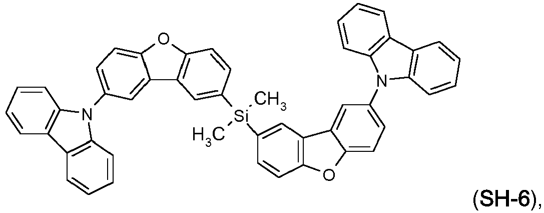 are described in WO2010079051 (in particular pages on 19 to 26 and in tables on pages 27 to 34, pages 35 to 37 and pages 42 to 43).

Additional host materials on basis of dibenzofurane are, for example, described in US2009066226, EP1885818B1, EP1970976, EP1998388 and EP2034538. Examples of particularly preferred host materials are shown below:

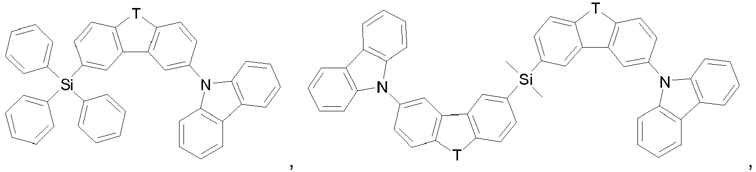

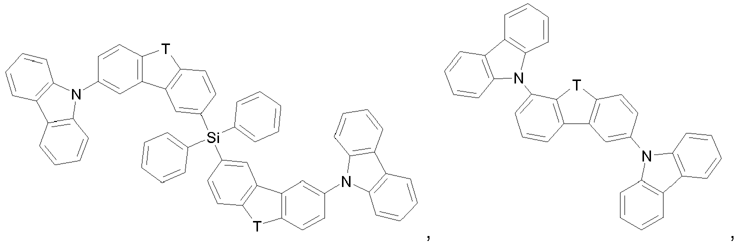

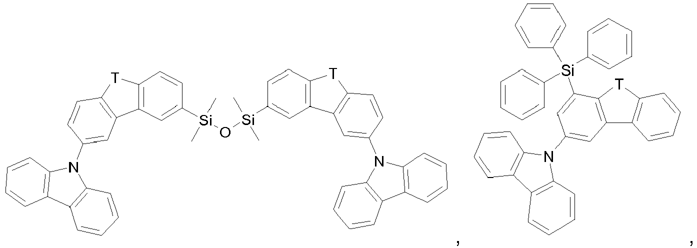

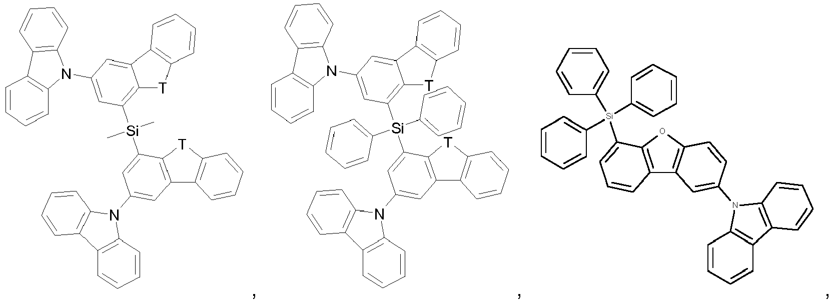

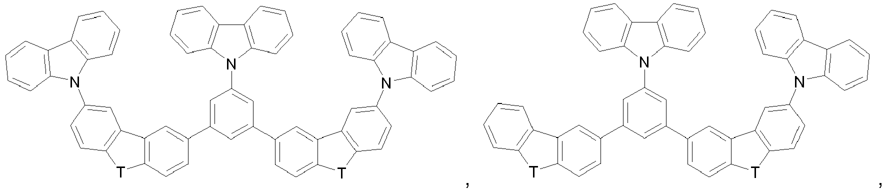

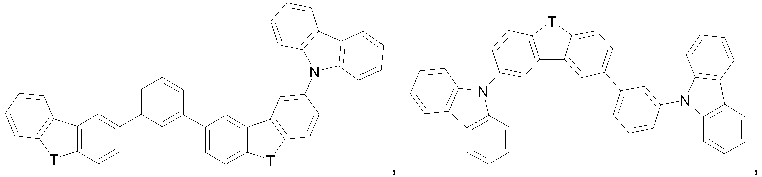

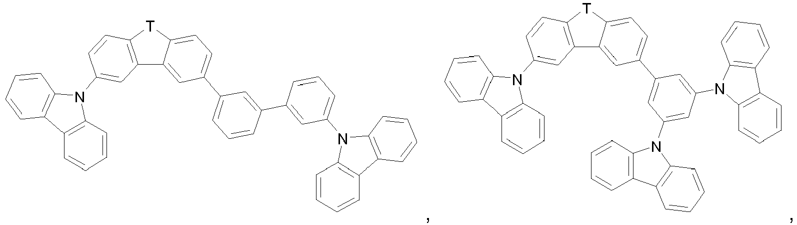

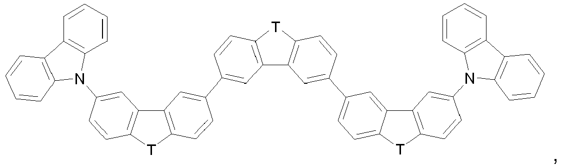

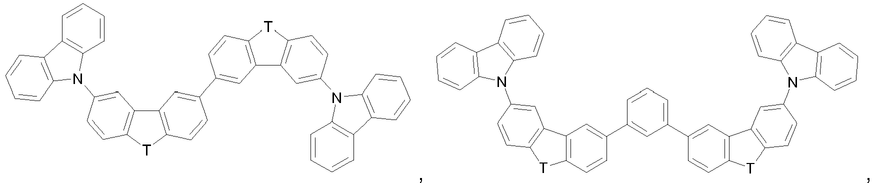

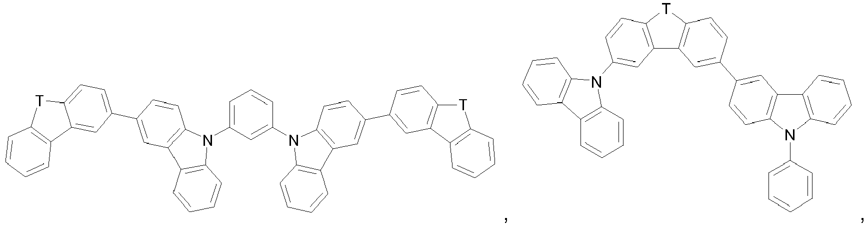

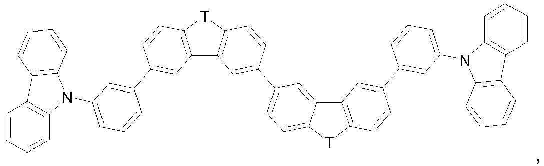

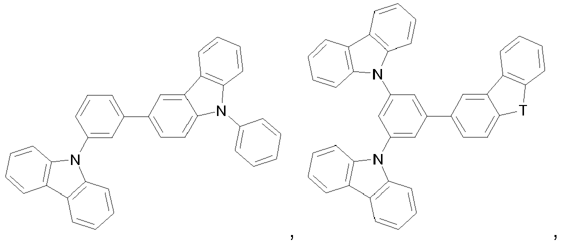

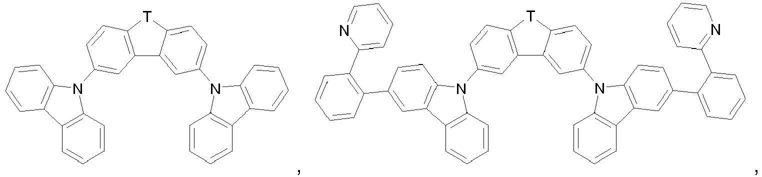

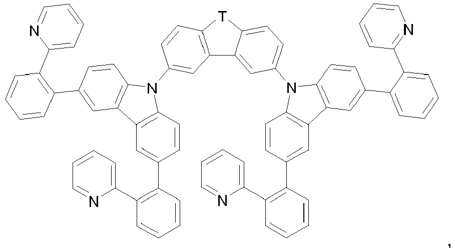

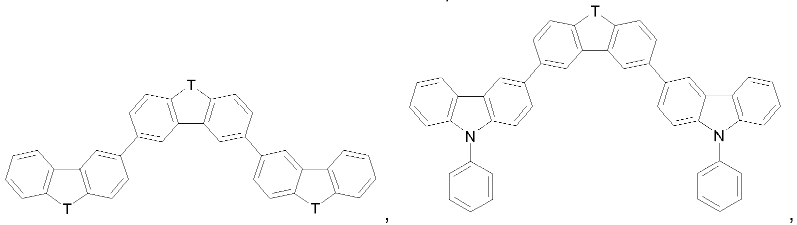

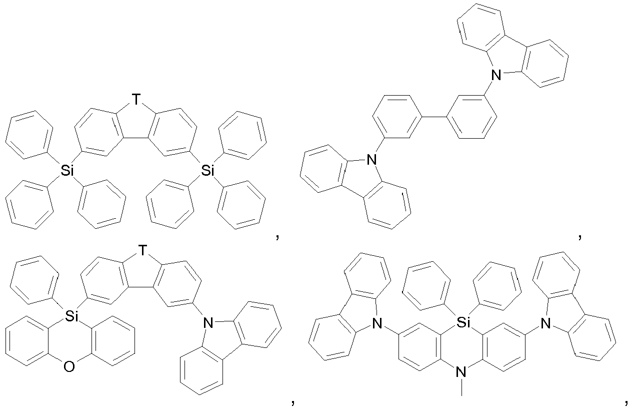

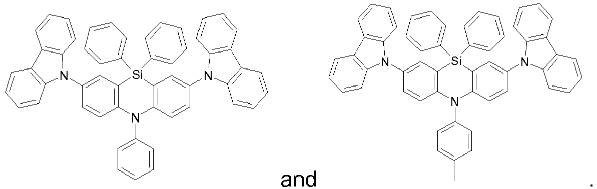

In the above-mentioned compounds T is O, or S, preferably O. If T occurs more than one time in a molecule, all groups T have the same meaning. Compounds (SH-1), 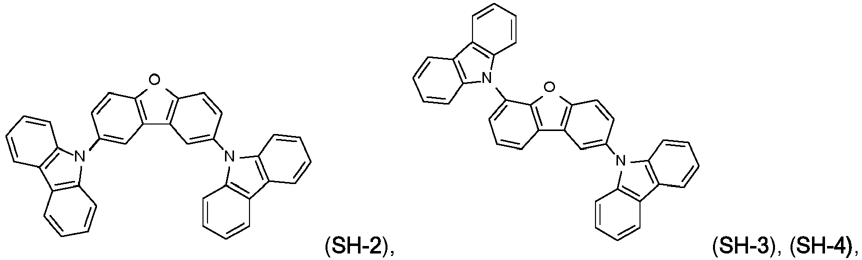 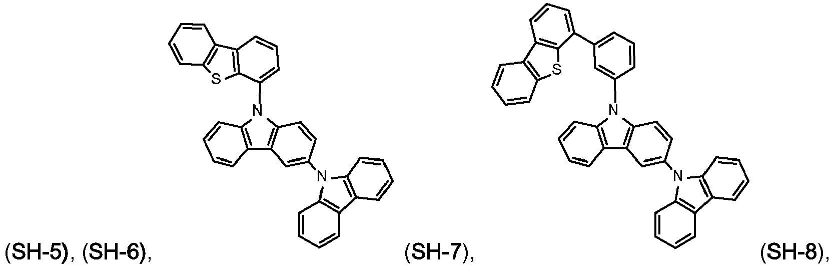 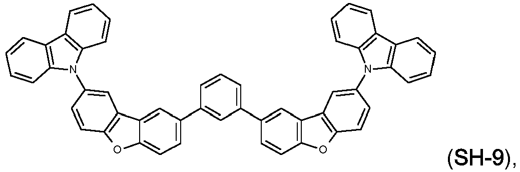 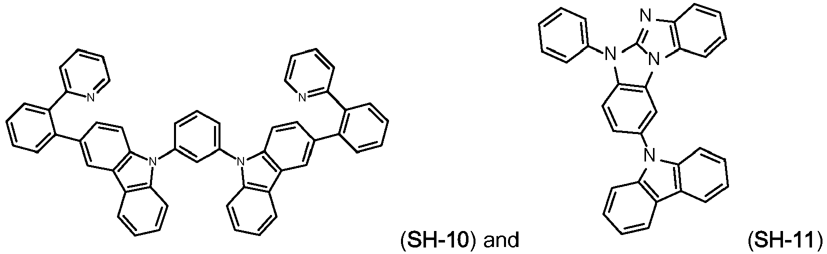 are most preferred.

### Hole/exciton blocking layer (f):

Blocking layers may be used to reduce the number of charge carriers (electrons or holes) and/or excitons that leave the emissive layer. The hole blocking layer may be disposed between the emitting layer (e) and electron transport layer (g), to block holes from leaving layer (e) in the direction of electron transport layer (g). Blocking layers may also be used to block excitons from diffusing out of the emissive layer.

In a preferred embodiment of the present invention, at least one compound of the formula I, especially a compound of the formula **II, or III** is used as hole/exciton blocking material. Examples of preferred compounds of formula I are compounds **A-1** to **A-47 as well as B-1** to **B-47.**

Additional hole blocker materials typically used in OLEDs are 2,6-bis(N-carbazolyl)pyridine (mCPy), 2,9-dimethyl-4,7-diphenyl-1,10-phenanthroline (bathocuproin, (BCP)), bis(2-methyl-8-quinolinato)-4-phenylphenylato)aluminum(III) (BAlq), phenothiazine S,S-dioxide derivates and 1,3,5-tris(N-phenyl-2-benzylimidazolyl)benzene) (TPBI), TPBI also being suitable as electron-transport material. Further suitable hole blockers and/or electron conductor materials are 2,2',2"-(1,3,5-benzenetriyl)tris(1-phenyl-1-H-benzimidazole), 2-(4-biphenylyl)-5-(4-tert-butylphenyl)-1,3,4-oxadiazole, 8-hydroxyquinolinolatolithium, 4-(naphthalen-1-yl)-3,5-diphenyl-4H-1,2,4-triazole, 1,3-bis[2-(2,2'-bipyridin-6-yl)-1,3,4-oxadiazo-5-yl]benzene, 4,7-diphenyl-1,10-phenanthroline, 3-(4-biphenylyl)-4-phenyl-5-tert-butylphenyl-1,2,4-triazole, 6,6'-bis[5-(biphenyl-4-yl)-1,3,4-oxadiazo-2-yl]-2,2'-bipyridyl, 2-phenyl-9, 10-di(naphthalene-2-yl)anthracene, 2,7-bis[2-(2,2'-bipyridin-6-yl)-1,3,4-oxadiazo-5-yl]-9,9-dimethylfluorene, 1,3-bis[2-(4-tert-butylphenyl)-1,3,4-oxadiazo-5-yl]benzene, 2-(naphthalene-2-yl)-4,7-diphenyl-1,10-phenanthroline, tris(2,4,6-trimethyl-3-(pyridin-3-yl)phenyl)borane, 2,9-bis(naphthalene-2-yl)-4,7-diphenyl-1,10-phenanthroline, 1-methyl-2-(4-(naphthalene-2-yl)phenyl)-1H-imidazo[4,5-f][1,10]phenanthroline. In a further embodiment, it is possible to use compounds which comprise aromatic or heteroaromatic rings joined via groups comprising carbonyl groups, as disclosed in WO2006/100298, disilyl compounds selected from the group consisting of disilylcarbazoles, disilylbenzofurans, dis-ilylbenzothiophenes, disilylbenzophospholes, disilylbenzothiophene S-oxides and disilylbenzothiophene S,S-dioxides, as specified, for example, in PCT applications WO2009/003919 and WO2009003898 and disilyl compounds as disclosed in WO2008/034758, as a blocking layer for holes/excitons (f).

In another preferred embodiment compounds (**SH-1**), (**SH-2**), (**SH-3**), (**SH-4**), (**SH-5**), (**SH-6**), (**SH-7**), (**SH-8**), (**SH-9**) and (**SH-10**) may be used as hole/exciton blocking materials.

### Electron transport layer (g):

Electron transport layer may include a material capable of transporting electrons. Electron transport layer may be intrinsic (undoped), or doped. Doping may be used to enhance conductivity. Suitable electron-transporting materials for layer (g) of the inventive OLEDs comprise metals chelated with oxinoid compounds, such as tris(8-hydroxyquinolato)aluminum (Alq₃), compounds based on phenanthroline such as 2,9-dimethyl-4,7-diphenyl-1,10-phenanthroline (DDPA = BCP), 4,7-diphenyl-1,10-phenanthroline (Bphen), 2,4,7,9-tetraphenyl-1,10-phenanthroline, 4,7-diphenyl-1,10-phenanthroline (DPA) or phenanthroline derivatives disclosed in EP1786050, in EP1970371, or in EP1097981, and azole compounds such as 2-(4-biphenylyl)-5-(4-t-butylphenyl)-1,3,4-oxadiazole (PBD) and 3-(4-biphenylyl)-4phenyl-5-(4-t-butylphenyl)-1,2,4-triazole (TAZ).

In a preferred embodiment of the present invention, at least one compound of the formula I, especially a compound of the formula **II, or III** is used as electron transport material. Examples of preferred compounds of formula I are compounds **A-1** to **A-47 as well as B-1** to **B-47.**

It is likewise possible to use mixtures of at least two materials in the electron-transporting layer, in which case at least one material is electron-conducting. Preferably, in such mixed electron-transport layers, at least one phenanthroline compound is used, preferably BCP, or at least one pyridine compound according to the formula **(VIII)** below, preferably a compound of the formula **(VIIIaa)** below. More preferably, in mixed electron-transport layers, in addition to at least one phenanthroline compound, alkaline earth metal or alkali metal hydroxyquinolate complexes, for example Liq, are used. Suitable alkaline earth metal or alkali metal hydroxyquinolate complexes are specified below **(formula VII).** Reference is made to WO2011/157779.

The electron-transport layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, No. 1, 1 July 2003 (p-doped organic layers); A. G. Werner, F. Li, K. Harada, M. Pfeiffer, T. Fritz, K. Leo, Appl. Phys. Lett., Vol. 82, No. 25, 23 June 2003 and Pfeiffer et al., Organic Electronics 2003, 4, 89 - 103 and K. Walzer, B. Maennig, M. Pfeiffer, K. Leo, Chem. Soc. Rev. 2007, 107, 1233. For example, it is possible to use mixtures which lead to electrical n-doping of the electron-transport layer. n-Doping is achieved by the addition of reducing materials. These mixtures may, for example, be mixtures of the abovementioned electron transport materials with alkali/alkaline earth metals or alkali/alkaline earth metal salts, for example Li, Cs, Ca, Sr, Cs₂CO₃, with alkali metal complexes and with Y, Ce, Sm, Gd, Tb, Er, Tm, Yb, Li₃N, Rb₂CO₃, dipotassium phthalate, W(hpp)₄ from EP1786050, or with compounds described in EP1837926B1, EP1837927, EP2246862 and WO2010132236.

In a preferred embodiment, the electron-transport layer comprises at least one compound of the general formula (**VII**) 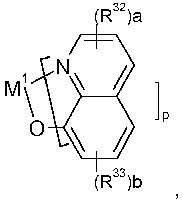 in which
R³² and R³³ are each independently F, C₁-C₈-alkyl, or C₆-C₁₄-aryl, which is optionally substituted by one or more C₁-C₈-alkyl groups, or two R³² and/or R³³ substituents together form a fused benzene ring which is optionally substituted by one or more C₁-C₈-alkyl groups;
a and b are each independently 0, or 1, 2 or 3,
M¹ is an alkaline metal atom or alkaline earth metal atom,
p is 1 when M¹ is an alkali metal atom, p is 2 when M¹ is an earth alkali metal atom. A very particularly preferred compound of the formula (VII) is 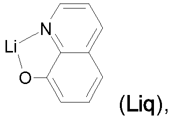 which may be present as a single species, or in other forms such as Li_{g}Q_{g} in which g is an integer, for example Li₆Q₆. Q is an 8-hydroxyquinolate ligand or an 8-hydroxyquinolate derivative.

In a further preferred embodiment, the electron-transport layer comprises at least one compound of the formula (VIII), 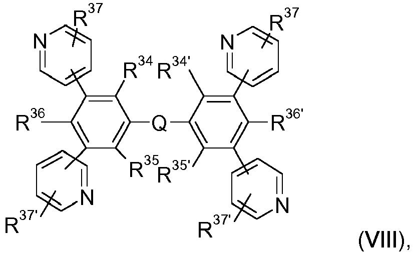 in which
R³⁴, R³⁵, R³⁶, R³⁷, R^{34'}, R^{35'}, R^{36'} and R^{37'} are each independently H, C₁-C₁₈-alkyl, C₁-C₁₈-alkyl which is substituted by E and/or interrupted by D, C₆-C₂₄-aryl, C₆-C₂₄-aryl which is substituted by G, C₂-C₂₀-heteroaryl or C₂-C₂₀-heteroaryl which is substituted by G,
Q is an arylene or heteroarylene group, each of which is optionally substituted by G;
D is -CO-; -COO-; -S-; -SO-; -SO₂-; -O-; -NR⁴⁰-; -SiR⁴⁵R⁴⁶-; -POR⁴⁷-; -CR³⁸=CR³⁹-; or -C≡C-; E is -OR⁴⁴; -SR⁴⁴; -NR⁴⁰R⁴¹; -COR⁴³; -COOR⁴²; -CONR⁴⁰R⁴¹; -CN; or F;
G is E, C₁-C₁₈-alkyl, C₁-C₁₈-alkyl which is interrupted by D , C₁-C₁₈-perfluoroalkyl, C₁-C₁₈-alkoxy, or C₁-C₁₈-alkoxy which is substituted by E and/or interrupted by D, in which R³⁸ and R³⁹ are each independently H, C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-;
R⁴⁰ and R⁴¹ are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-; or R⁴⁰ and R⁴¹ together form a 6-membered ring;
R⁴² and R⁴³ are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R⁴⁴ is C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R⁴⁵ and R⁴⁶ are each independently C₁-C₁₈-alkyl, C₆-C₁₈-aryl or C₆-C₁₈-aryl which is substituted by C₁-C₁₈-alkyl,
R⁴⁷ is C₁-C₁₈-alkyl, C₆-C₁₈-aryl or C₆-C₁₈-aryl which is substituted by C₁-C₁₈-alkyl.

Preferred compounds of the formula **(VIII)** are compounds of the formula (**VIIIa**) 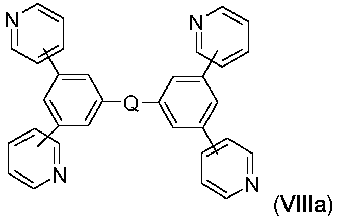 in which Q is: 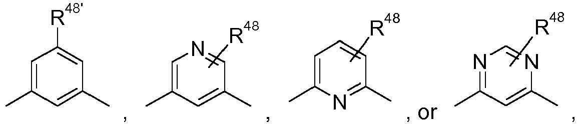 R⁴⁸ is H or C₁-C₁₈-alkyl and R^{48'} is H, C₁-C₁₈-alkyl or

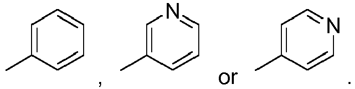

Particular preference is given to a compound of the formula

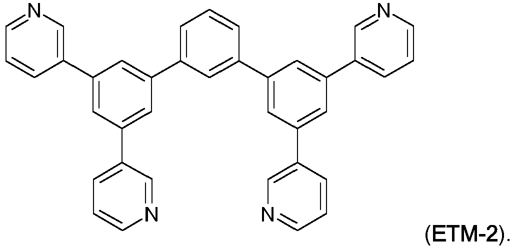

In a further, very particularly preferred embodiment, the electron-transport layer comprises a compound **Liq** and a compound **ETM-2.**

In a preferred embodiment, the electron-transport layer comprises the compound of the formula (VII) in an amount of 99 to 1% by weight, preferably 75 to 25% by weight, more preferably about 50% by weight, where the amount of the compounds of the formulae (VII) and the amount of the compounds of the formulae (VIII) adds up to a total of 100% by weight.

The preparation of the compounds of the formula (VIII) is described in J. Kido et al., Chem. Commun. (2008) 5821-5823, J. Kido et al., Chem. Mater. 20 (2008) 5951-5953 and JP2008/127326, or the compounds can be prepared analogously to the processes disclosed in the aforementioned documents.

It is likewise possible to use mixtures of alkali metal hydroxyquinolate complexes, preferably Liq, and dibenzofuran compounds in the electron-transport layer. Reference is made to WO2011/157790. Dibenzofuran compounds **A-1** to **A-36** and **B-1** to **B-22** described in WO2011/157790 are preferred, wherein dibenzofuran compound 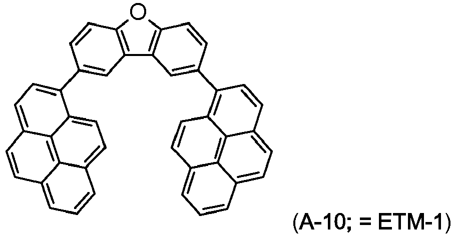 is most preferred.

In a preferred embodiment, the electron-transport layer comprises **Liq** in an amount of 99 to 1% by weight, preferably 75 to 25% by weight, more preferably about 50% by weight, where the amount of **Liq** and the amount of the dibenzofuran compound(s), especially **ETM-1**, adds up to a total of 100% by weight.

In a preferred embodiment, the electron-transport layer comprises at least one phenanthroline derivative and/or pyridine derivative.

In a further preferred embodiment, the electron-transport layer comprises at least one phenanthroline derivative and/or pyridine derivative and at least one alkali metal hydroxyquinolate complex.

In a further preferred embodiment, the electron-transport layer comprises at least one of the dibenzofuran compounds **A-1** to **A-36** and **B-1** to **B-22** described in WO2011/157790, especially **ETM-1.**

In a further preferred embodiment, the electron-transport layer comprises a compound described in WO2012/111462, WO2012/147397, WO2012014621, such as, for example, a compound of formula 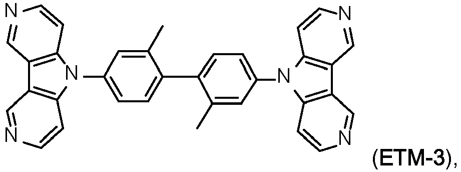 US2012/0261654 , such as, for example, a compound of formula 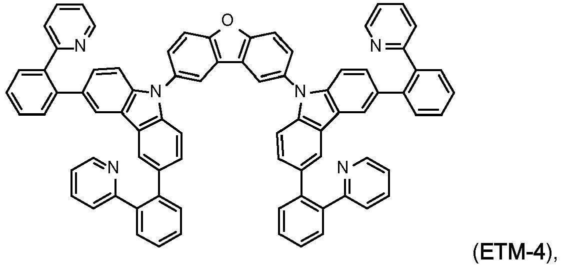 and WO2012/115034, such as for example, such as, for example, a compound of formula

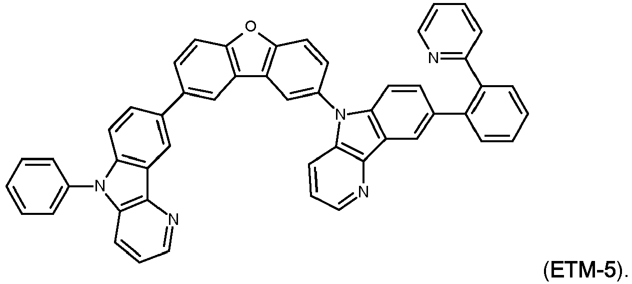

### Electron injection layer (h):

The electron injection layer may be any layer that improves the injection of electrons into an adjacent organic layer. Lithium-comprising organometallic compounds such as 8-hydroxyquinolatolithium (Liq), CsF, NaF, KF, Cs₂CO₃ or LiF may be applied between the electron transport layer (g) and the cathode (i) as an electron injection layer (h) in order to reduce the operating voltage.

### Cathode (i):

The cathode (i) is an electrode which serves to introduce electrons or negative charge carriers. The cathode may be any metal or nonmetal which has a lower work function than the anode. Suitable materials for the cathode are selected from the group consisting of alkali metals of group 1, for example Li, Cs, alkaline earth metals of group 2, metals of group 12 of the Periodic Table of the Elements, comprising the rare earth metals and the lanthanides and actinides. In addition, metals such as aluminum, indium, calcium, barium, samarium and magnesium, and combinations thereof, may be used.

In general, the different layers, if present, have the following thicknesses:
anode (a): 500 to 5000 Å (ångström), preferably 1000 to 2000 Å;
hole injection layer (b): 50 to 1000 Å, preferably 200 to 800 Å,
hole-transport layer (c): 50 to 1000 Å, preferably 100 to 800 Å,
electron/exciton blocking layer (d): 10 to 500 Å, preferably 50 to 100 Å,
light-emitting layer (e): 10 to 1000 Å, preferably 50 to 600 Å,
hole/ exciton blocking layer (f): 10 to 500 Å, preferably 50 to 100 Å,
electron-transport layer (g): 50 to 1000 Å, preferably 200 to 800 Å,
electron injection layer (h): 10 to 500 Å, preferably 20 to 100 Å,
cathode (i): 200 to 10 000 Å, preferably 300 to 5000 Å.

The person skilled in the art is aware (for example on the basis of electrochemical studies) of how suitable materials have to be selected. Suitable materials for the individual layers are known to those skilled in the art and are disclosed, for example, in WO 00/70655.

In addition, it is possible that some of the layers used in the inventive OLED have been surface-treated in order to increase the efficiency of charge carrier transport. The selection of the materials for each of the layers mentioned is preferably determined by obtaining an OLED with a high efficiency and lifetime.

The inventive OLED can be produced by methods known to those skilled in the art. In general, the inventive OLED is produced by successive vapor deposition of the individual layers onto a suitable substrate. Suitable substrates are, for example, glass, inorganic semiconductors or polymer films. For vapor deposition, it is possible to use customary techniques, such as thermal evaporation, chemical vapor deposition (CVD), physical vapor deposition (PVD) and others. In an alternative process, the organic layers of the OLED can be applied from solutions or dispersions in suitable solvents, employing coating techniques known to those skilled in the art.

Use of the compounds of the formula I in at least one layer of the OLED, preferably in the light-emitting layer (preferably as a matrix material), charge transport layer and/or in the charge/exciton blocking layer makes it possible to obtain OLEDs with high efficiency and with low use and operating voltage. Frequently, the OLEDs obtained by the use of the compounds of the formula I additionally have high lifetimes. The efficiency of the OLEDs can additionally be improved by optimizing the other layers of the OLEDs. For example, high-efficiency cathodes such as Ca or Ba, if appropriate in combination with an intermediate layer of LiF, can be used. Moreover, additional layers may be present in the OLEDs in order to adjust the energy level of the different layers and to facilitate electroluminescence.

The OLEDs may further comprise at least one second light-emitting layer. The overall emission of the OLEDs may be composed of the emission of the at least two light-emitting layers and may also comprise white light.

The OLEDs can be used in all apparatus in which electroluminescence is useful. Suitable devices are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cellphones, tablet PCs, laptops, digital cameras, MP3 players, vehicles and destination displays on buses and trains. Further devices in which the inventive OLEDs can be used are, for example, keyboards; items of clothing; furniture; wallpaper. In addition, the present invention relates to a device selected from the group consisting of stationary visual display units such as visual display units of computers, televisions, visual display units in printers, kitchen appliances and advertising panels, illuminations, information panels, and mobile visual display units such as visual display units in cellphones, tablet PCs, laptops, digital cameras, MP3 players, vehicles and destination displays on buses and trains; illumination units; keyboards; items of clothing; furniture; wallpaper, comprising at least one inventive organic light-emitting diode or at least one inventive light-emitting layer.

The following examples are included for illustrative purposes only and do not limit the scope of the claims. Unless otherwise stated, all parts and percentages are by weight.

### Examples

### Example 1

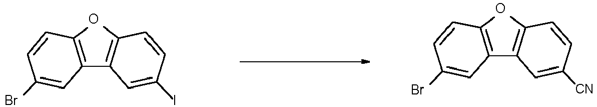

a) 50 g (0.134 mol) 2-Bromo-8-iodo-dibenzofuran and 12.0 g (0.134 mol) CuCN are added to 350 ml of DMF and the suspension is heated to 100°C internal temperature for 20 h under Nitrogen. The reaction mixture is filtered hot and the residue is washed twice with 30 ml of DMF each. The clear filtrate is poured on 3 l of water. The suspension is filtered and the residue is washed twice with water (500 ml each). The white solid is suspended in 1.5 l of 10% Ammonia in water, stirred for 45 minutes, filtered, washed three times with water (500 ml each) and dried at 80°C/125 mbar overnight. The resulting solid is dissolved in 600 ml of boiling THF, 60 g of silica are added, stirred for 1 h and then filtered hot. The filtrate is evaporated to 200 ml, cooled to 0°C, filtered, washed twice with cold THF (20 ml each) and dried at 80°C/125 mbar overnight to yield 27.1 g (74.4% of theory) of crude product. The crude product is purified by Soxhlet extraction using 200 ml of EtOAc to yield 23.5 g (64.6% of theory) as a white solid. The product is further purified by chromatography (solvent: Heptane/CH₂Cl₂ = 4:6) to yield 17.0 g (46.6% of theory) of product as a mixture of 8-Bromodibenzofuran-2-carbonitrile and 8-lododibenzofuran-2-carbonitrile (93:7) as a white solid.
   ¹H-NMR (400 MHz, CDCl3): δ 8.22 (d, J = 1.2 Hz, 1H), 8.09 (d, J = 8.2 Hz, 1H), 7.77 (dxd, J₁ = 8.4 Hz, J₂ = 1.6 Hz, 1H), 7.66 - 7.62 (m, 2H), 7.49 (d, J = 8.8 Hz. 1H). 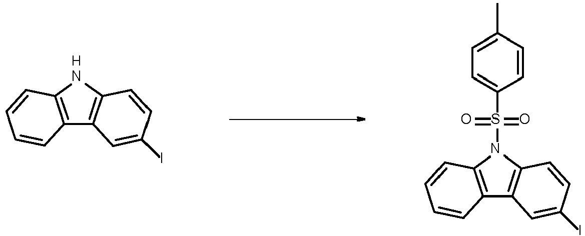
b) 100 g (0.341 mol) 3-lodo-9H-carbazole are added to 1.6 l Toluene, then 80.4 g (0.409 mol) 4-Methylbenzenesulfonyl chloride and 8.1 g (0.024 mol) Tetrabutylammonium hydrogen sulfate are added. To the stirred suspension 460 g 50% aqueous KOH solution are slowly added within 50 minutes while keeping the temperature below 25°C with a water bath. After stirring for additional 2 hours the reaction mixture is diluted with 250 ml of water, the phases are separated and the organic phase is washed four times with water (250 ml each), dried over Magnesium sulfate, filtered and evaporated to yield 148.1 g (97.1% of theory) of a white solid. The crude product is suspended in 300 ml of 2-Propanol, heated to reflux for 2 hours, cooled to RT filtered, washed three times with 2-Propanol (50 ml each) and dried at 80°C/125 mbar overnight to yield 138.2 g (90.6% of theory) 3-lodo-9-(p-tolylsulfonyl) carbazole as white solid.
   ¹H-NMR (400 MHz, CDCl₃): δ 8.31 (d, J = 8.4 Hz, 1H), 8.20 (d, J = 1.6 Hz, 1H), 8.10 (d, J = 8.4 Hz, 1H), 7.82 (d, J = 8.0 Hz, 1H), 7.75 (dxd, J₁ = 8.8 Hz, J₂ = 2.0 Hz, 1H), 7.67 (d, J = 8.4 Hz, 2H), 7.51 (t, J = 7.2 Hz, 1H), 7.36 (t, J = 7.2 Hz, 1H), 7.10 (d, J = 8.4 Hz, 2H), 2.25 (s, 3H). 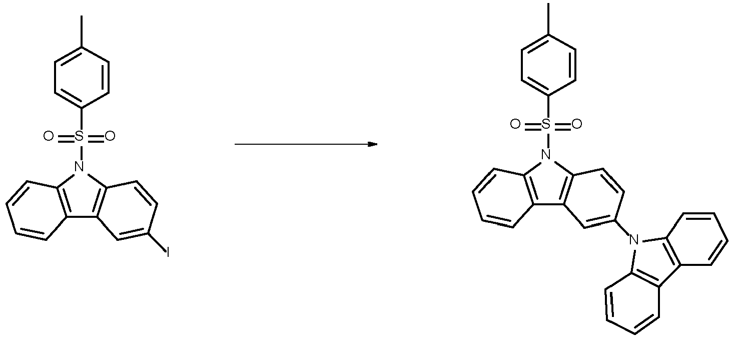
c) 54.8 g (0.137 mol) 3-lodo-9-(p-tolylsulfonyl) carbazole, 27.5 g (0.164 mol) 9H-Carbazole, 9.4 g (0.082 mol) trans-1,2-Diamino-cyclohexane, 183.2 g (0.863 mol) K₃PO₄ and 14.6 g (0.076 mol) Cul are added to 1 l Toluene and the grey suspension is heated to reflux for 48 hours. The reaction mixture is filtered hot through Hiflo and washed four times with Toluene (100 ml each). To the clear filtrate 100 g of silica are added and the suspension is heated to reflux for 1 hour, filtered hot and washed three times with Toluene (100 ml each). The filtrate is evaporated to yield 68.6 g (102.9% of theory) of a brown solid. To the crude product 600 ml of Cyclohexane/Toluene = 2:1 are added and heated to reflux to give a clear solution. Then 200 ml of Cyclohexane are added and the solution is cooled to RT and then to 0°C. The resulting suspension is filtered; the residue is washed three times with cold Cyclohexane (75 ml each) and dried at 80°C/125 mbar overnight to yield 39.0 g (58.5% of theory) 3-Carbazol-9-yl-9-(p-tolylsulfonyl)carbazole as white solid.
   ¹H-NMR (400 MHz, CDCl₃): δ 8.55 (d, J = 8.8 Hz, 1H), 8.40 (d, J = 8.4 Hz, 1H), 8.17 (d, J = 7.6 Hz, 2H), 8.09 (d, J = 2.0 Hz, 1H), 7.89 (d, J = 7.6 Hz, 1H), 7.83 (d, J = 8.4 Hz, 2H),7.67 (dxd, J₁ = 8.4 Hz, J₂ = 2.0 Hz, 1H), 7.56 (t, J = 8.4 Hz, 1H), 7.44 - 7.26 (m, 7H), 7.20 (d, J = 8.0 Hz, 2H), 2.36 (s, 3H). 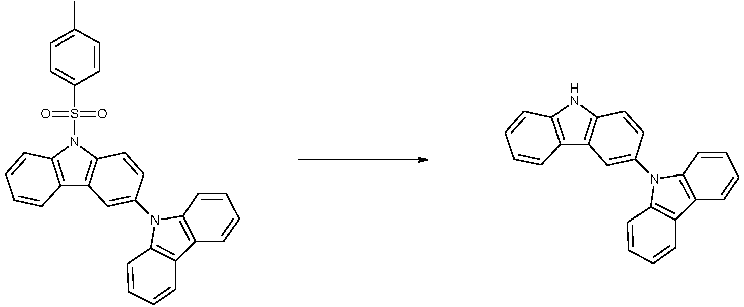
d) 10.6 g (160.3 mmol) KOH 85% are dissolved in 450 ml EtOH, then 39.0 g (80.1 mmol) 3-Carbazol-9-yl-9-(p-tolylsulfonyl)carbazole dissolved in 180 ml THF are added and the clear solution is heated to reflux for 6 hours. The reaction mixture is cooled to RT and then add-ed drop by drop to 1 l H₂O. The resulting suspension is filtered and the residue is washed four times with H₂O (200 ml each) and then dried at 80°C/125 mbar overnight to yield 25.6 g (96.1% of theory) of an off white solid. The crude product is suspended in 150 ml MeOH and heated to reflux for 2 hours. The suspension is cooled to RT and then to 0°C, filtered, washed twice with cold MeOH (20 ml each) and dried at 60°C /125 mbar overnight to yield 23.4 g (87.9%) of an off white solid. This procedure is repeated twice to yield 20.76 g (77.9% of theory) 3-Carbazol-9-yl-9H-carbazole as a white solid.
   ¹H-NMR (400 MHz, CDCl₃): δ 8.35 - 8.20 (m, 3H), 8.16 (br s, 1H), 8.08 (d, J = 7.6 Hz, 1H), 7.64 - 7.28 (m, 11H). 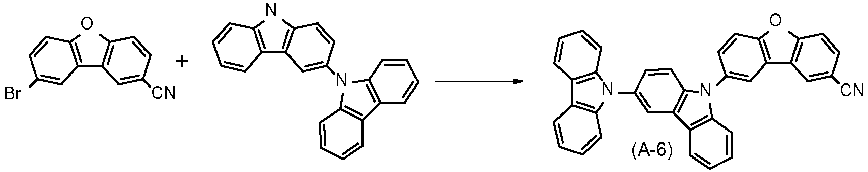
e) 7.78 g (23.4 mmol) 3-Carbazol-9-yl-9H-carbazole, 5.50 g (19.5 mmol) 8-Bromodibenzofuran-2-carbonitrile,1.11 g (5.8 mmol) CuI, 2.32 g (11.7 mmol) 1,10-Phenanthroline and 13.3 g (62.4 mmol) K₃PO₄ are suspended in 200 ml Mesitylene and heated to reflux for 48 hours. The resulting suspension is diluted with 50 ml Toluene, filtered hot through Hiflo and the residue is washed three times with Toluene (50 ml each). To the filtrate are added 30 g Silica, the suspension is heated to reflux for 1 hour, filtered hot and the residue is washed three times with Toluene (50 ml each). The filtrate is cooled to RT, dried with MgSO₄, filtered and evaporated to yield 14.1 g (138.7% of theory) of a slightly brown solid. To the crude product 100 ml MeOH are added and the suspension is heated to reflux for 1 hour, then cooled to 0°C, filtered, washed three times with MeOH (20 ml each) and dried at 60°C / 125 mbar overnight to yield 10.1 g (98.9% of theory) of a beige solid that is further purified by chromatography (solvent gradient: Heptane/CH₂Cl₂) to yield 5.81 g (56.9% of theory) 8-(3-carbazol-9-ylcarbazol-9-yl)dibenzofuran-2-carbonitrile as a white solid.
   ¹H-NMR (400 MHz, CDCl₃): δ 8.33 (d, J = 1.6 Hz, 2H), 8.26 (d, J = 2.0 Hz, 1H), 8.20 (d, J = 8.0 Hz, 2H), 8.16 (d, J = 7.6 Hz, 1H), 7.91 (d, J = 8.8 Hz, 1H), 7.83 (txd, J₁ = 8.8 Hz, J₂ = 1.6 Hz, 2H), 7.77 (d, J = 8.8 Hz, 1H), 7.59 - 7.29 (m, 11H).

### Example 2

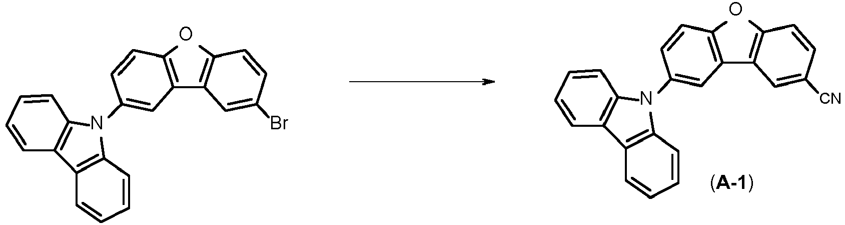

a) 5.0 g (12.12 mmol) 9-(8-Bromodibenzofuran-2-yl) carbazole (prepared according to WO2010079051, example 13), are dissolved in 25 ml DMF and 1.63 g (18.19 mmol) CuCN are added. The suspension is heated to reflux for 22 hours, filtered hot and the residue is washed twice with DMF (10 ml each). The filtrate is poured on 400 ml H₂O, stirred for 15 min. and the resulting suspension is filtered. The residue is washed three times with H₂O (100 ml each) and dried at 80°C /125 mbar overnight. To the crude product are added 500 ml Toluene and 15 g silica und the mixture is heated to reflux for 2 hours. The suspension is filtered hot and the residue is washed twice with Toluene (100 ml each). The filtrate is evaporated and the crude product is purified by chromatography (solvent: Heptane/CH₂Cl₂ = 3:7) to yield 2.68 g (61.7% of theory) 8-Carbazol-9-yldibenzofuran-2-carbonitrile as a white solid.
   ¹H-NMR (400 MHz, CDCl₃): 8.27 (d, J = 1.6 Hz, 1H), 8.18 (d, J = 7.6 Hz, 2H), 8.16 (d, J = 2.0 Hz, 1H), 7.85 (d, J = 8.4 Hz, 1H), 7.82 (dxd, J₁ = 8.4 Hz, J₂ = 1.6 Hz, 1H), 7.76 - 7.72 (m, 2H), 7.45 - 7.31 (m, 6H).

### Example 3

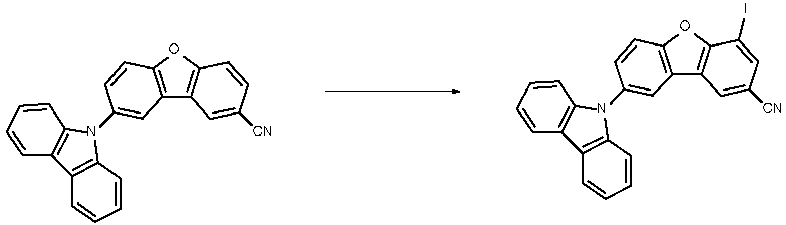

a) 6.97 g (68.9 mmol) Diisopropylamine are dissolved in 240 ml THF abs. and cooled to - 78°C with an Acetone/CO₂ bath. 28.1 ml (75.8 mmol) BuLi 2.7 M in Hexane are added, keeping the temperature below -70°C. The resulting clear solution is warmed to RT and then added slowly to a solution of 24.7 g (68.9 mmol) 8-Carbazol-9-yldibenzofuran-2-carbonitrile dissolved in 480 ml THF abs. cooled to -78°C with an Acetone/CO₂ bath keeping the temperature below -73°C. The reaction mixture is then stirred for another hour at - 78°C. A solution of 17.5 g (68.9 mmol) I₂ dissolved in 120 ml THF abs. is then added slowly keeping the temperature below -73°C. The reaction mixture is slowly warmed to RT, hydrolyzed with 500 ml of H₂O and then brought to pH = 7 by adding 4N HCI. The THF is then evaporated and the resulting mixture is extracted three times with EtOAc (500 ml each). The combined EtOAc extracts are then washed once with 300 ml H₂O and once with 100 ml sat. NaCl solution, dried with Na₂SO₄, filtered and evaporated to 90 g of a beige suspension, which is cooled to 0°C and filtered. The residue is washed once with cold EtOAc (10 ml) and twice with cold MeOH (10 ml each) and dried overnight at 80°C/125 mbar to yield 22.0 of crude product that is purified by chromatography (solvent: Heptane/CH₂Cl₂ = 3:7) to yield 12.8 g (38.3% of theory) 8-Carbazol-9-yl-4-iodo-dibenzofuran-2-carbonitrile as a white solid.
   ¹H-NMR (400 MHz, CDCl₃): 8.22 (d, J = 1.6 Hz, 1H), 8.18 (d, J = 7.6 Hz, 2H), 8.16 (d, J = 1.6 Hz, 1H), 8.14 (d, J = 2.0 Hz, 1H), 7.93 (d, J = 8.8 Hz, 1H), 7.77 (dxd, J₁ = 8.4 Hz, J₂ = 2.0 Hz, 1H), 7.45 - 7.31 (m, 6H). 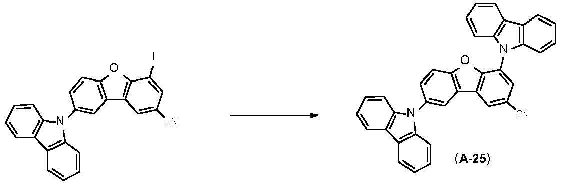
b) 12.8g (26.4 mmol) 8-Carbazol-9-yl-4-iodo-dibenzofuran-2-carbonitrile, 6.6 g (39.5 mmol) 9H-Carbazole, 0.9 g (7.9 mmol) trans-1,2-Diaminocyclohexane, 1.51 g (7.9 mmol) CuI and 35.2 g (166.0 mmol) K₃PO₄ are added to 300ml Toluene and the suspension is heated to reflux 5 hours. The reaction mixture is diluted with 100 ml Toluene, filtered hot and the residue is washed tree times with Toluene (100 ml each). 30 g Silica are added to the filtrate and the suspension is heated to reflux for 30 minutes, filtered and the residue is washed three times with Toluene (100 ml each). The filtrate is evaporated to 65 g of a suspension, which is cooled to RT and filtered. The residue is washed once with cold Toluene (10 ml) and dried overnight at 80°C/125 mbar to yield 8.1 of crude product that is purified by chromatography (solvent: Heptane/CH₂Cl₂ = 4:6) to yield 1.17 g (8.4% of theory) 4,8-Di(carbazol-9-yl)dibenzofuran-2-carbonitrile as a white solid.
   ¹H-NMR (400 MHz, CDCl₃): 8.04 (d, J = 1.6 Hz, 1H), 8.127 (s, 1H), 8.23 (d, J = 8.0 Hz, 2H), 8.19 (d, J = 7.6 Hz, 2H), 8.07 (d, J = 1.6 Hz, 1H), 7.80 - 7.65 (m, 2H), 7.50 - 7.27 (m, 12H).

### Example 4

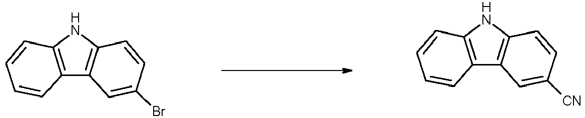

a) 100 g (0.406 mol) 3-Bromo-9H-carbazole and 58.2 g (0.650 mol) CuCN are added to 800 ml of DMF and the suspension is heated to reflux for 20 h under Nitrogen. The resulting solution is cooled to room temperature and then poured on 4 l of water. The suspension is filtered and the residue is washed three times with water (1 l each). The white solid is suspended in 1.5 l of 10% Ammonia in water, stirred for three hours, filtered, washed three times with water (1 l each) and dried at 80°C/125 mbar overnight. The resulting solid is dissolved in 1 l of boiling THF, 100 g of silica is added, stirred for 1 h and then filtered hot. The filtrate is evaporated to 400 ml, cooled to room temperature, filtered, washed three times with cold THF (50 ml each) and dried at 80°C/125 mbar overnight to yield 43.3 g (55.6% of theory) 9H-Carbazole-3-carbonitrile as a white solid.
   ¹H-NMR (400 MHz, CDCl₃): δ 8.39 (br s, 2H), 8.10 (d, J = 8.0 Hz, 1H), 7.67 (dxd, J₁ = 8.4 Hz, J₂= 1.6 Hz, 1H), 7.54 - 7.47 (m, 3H), 7.35 - 7.31 (m, 1H). 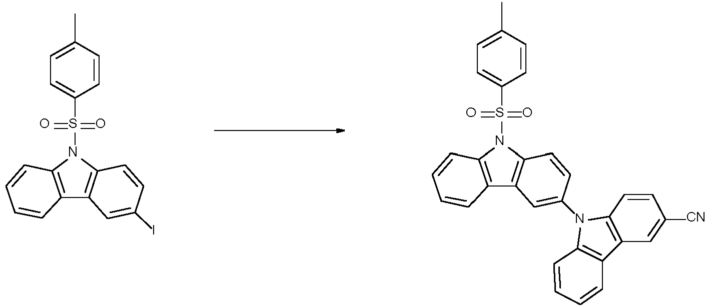
b) 58.4 g (0.131 mol) 3-lodo-9-(p-tolylsulfonyl) carbazole, 30.0 g (0.156 mol) 9H-Carbazole-3-carbonitrile, 8.9 g (0.078 mol) trans-1,2-Diamino-cyclohexane, 173.9 g (0.822 mol) K₃PO₄ and 13.9 g (0.073 mol) CuI are added to 900 ml 1,4-Dioxane and the grey suspension is heated to reflux for 27 hours. The reaction mixture is filtered hot through Hiflo and washed three times with 1,4-Dioxane (200 ml each). The filtrate is evaporated and the residue is dissolved in 750 ml EtOAc. To the clear solution 50 g of silica are added and the suspension is stirred for 1 hour, filtered and washed three times with EtOAc (100 ml each). The filtrate is evaporated to yield 140 g of a suspension, which is cooled to 0°C and filtered. The residue is washed once with 20 ml cold EtOAc and twice with cold MeOH (20 ml each) and dried at 80°C / 125 mbar overnight to yield 36.8 g crude product. 250 ml MeOH are added to the crude product and the suspension is heated to reflux for three hours, then cooled to RT and filtered. The residue is washed three times with MeOH (25 ml each) and dried at 80°C / 125 mbar overnight to yield 35.6 g 9-[9-(p-Tolylsulfonyl)carbazol-3-yl]carbazole-3-carbonitrile as an off white solid.
   ¹H-NMR (400 MHz, CDCl₃): δ 8.58 (d, J = 8.8 Hz, 1H), 8.48 (s, 1H), 8.40 (d, J = 8.8 Hz, 1H), 8.18 (d, J = 7.2 Hz, 2H), 8.05 (d, J = 2.0 Hz, 1H), 7.90 (d, J = 7.6 Hz, 1H), 7.83 (d, J = 8.4 Hz, 2H), 7.66 - 7.56 (m, 3H), 7.50 (t, J = 8.4 Hz, 1H), 7.43 - 7.36 (m, 4H), 7.22 (d, J = 8.0 Hz, 2H), 2.35 (s, 3H). 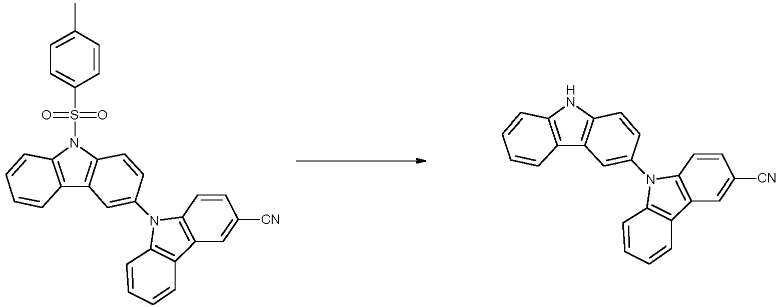
c) 9.2 g (139.3 mmol) KOH 85% are dissolved in 290 ml EtOH, then 35.6 g (69.6 mmol) 9-[9-(p-Tolylsulfonyl)carbazol-3-yl]carbazole-3-carbonitrile dissolved in 590 ml THF are added and the clear solution is stirred for 6 hours and then added drop by drop to 500 ml H₂O. The solution is brought to pH=8 by the addition of 4N HCI and the organic solvents are evaporated. The residue is extracted three times with EtOAc (250 ml each) and the combined organic phases are washed once with 100 ml of H₂O and once with 100 ml of brine, dried with Na₂SO₄, filtered and evaporated to yield 40.6 g of crude product as a grey solid. To the crude product 250 ml MeOH are added and the suspension is heated to reflux for 3 hours, cooled to RT and filtered. The residue is washed three times with MeOH (20 ml each) and dried at 80°C / 125 mbar. This procedure was repeated once to yield 18.2 g (73.0% of theory) 9-(9H-Carbazol-3-yl)carbazole-3-carbonitrile as a white solid.
   ¹H-NMR (400 MHz, CDCl₃): δ 8.48 (s, 1H), 8.41 (br s, 1H), 8.20 - 8.16 (m, 2H), 8.04 (d, J = 8.0 Hz, 1H), 7.66 - 7.28 (m, 10H). 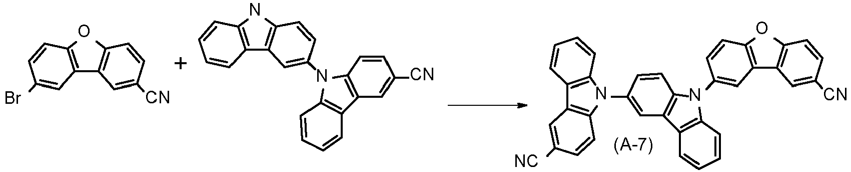 d ) 8.6 g (31.6 mmol) 8-Bromodibenzofuran-2-carbonitrile, 13.6 g (37.9 mmol) 9-(9H-Carbazol-3-yl)carbazole-3-carbonitrile, 1.81 g (9.5 mmol) CuI, 3.76 g (19.0 mmol) 1,10-Phenanthroline and 21.5 g (101.1 mmol) K₃PO₄ are suspended in 400 ml Mesitylene and heated to reflux for 56 hours. The resulting suspension is cooled to RT, filtered and washed three times with Heptane (200 ml each) and three times with MeOH (200 ml each). 1 l H₂O is added to the grey residue and stirred for 1 hour at RT. The suspension is filtered, washed three times with H₂O (200 ml each), three times with MeOH (200 ml each) and dried at 80°C / 125 mbar overnight to yield 22.7 g crude product as a grey solid. The crude product was purified by Soxhlet extraction using 300 ml MEK as solvent to yield 6.53 g (37.7% of theory) 9-[9-(8-Cyanodibenzofuran-2-yl)carbazol-3-yl]carbazole-3-carbonitrile as white solid.
   ¹H-NMR (400 MHz, CDCl₃): δ 8.50 (d, J = 0.8 Hz, 1H), 8.33 (d, J = 1.6 Hz, 1H), 8.30 (d, J = 1.6 Hz, 1H), 8.25 (d, J = 2.0 Hz, 1H), 8.20 (d, J = 7.6 Hz, 1H), 8.16 (d, J = 8.0 Hz, 1H), 7.93 (d, J = 8.4 Hz, 1H), 7.87 - 7.77 (m, 3H), 7.66 (dxd, J₁ = 8.4 Hz, J₂ = 1.6 Hz, 1H), 7.58 - 7.36 (m, 9H).

### Comparative Application Example 1

A glass substrate with 120 nm-thick indium-tin-oxide (ITO) transparent electrode used as an anode is first cleaned with isopropanol in an ultrasonic bath for 10 min. To eliminate any possible organic residues, the substrate is exposed to an ultraviolet light and ozone for further 30 min. This treatment also improves the hole injection properties of the ITO. The cleaned substrate is mounted on a substrate holder and loaded into a vacuum chamber. Thereafter, the organic materials specified below are applied by vapor deposition to the ITO substrate at a rate of approx. 0.2-1 Å/sec at about 10⁻⁶ -10⁻⁸ mbar. As a hole injection layer, compound 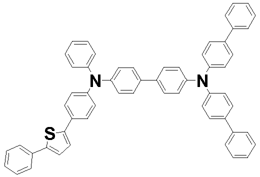 with 30 nm thickness is applied. Then compound 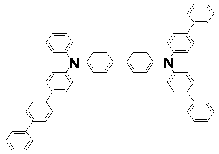 with 60 nm thickness is applied as a hole transporting layer. As an exciton and electron blocker, compound 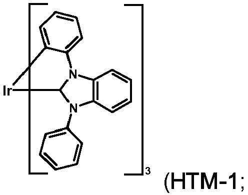 for preparation, see Ir complex (7) in the application WO2005/019373) is then applied with a thickness of 10 nm.

Subsequently, a mixture of 20% by weight of emitter compound, 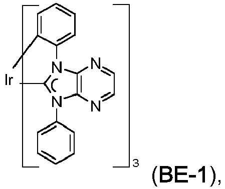 15% by weight of compound (**HTM-1**) and 65% by weight of host 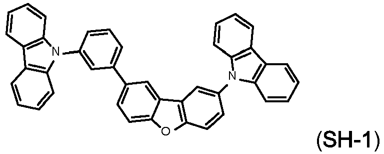 are applied to form a 40 nm-thick emitting layer. On the emitting layer, 5 nm-thick material (**SH-1**) is applied as an exciton blocker. Thereafter, compound 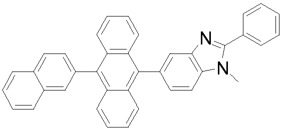 with 20 nm thickness is deposited as an electron transport layer. Finally, 1 nm-thick LiF is deposited as an electron injection layer and 80 nm-thick Al is then deposited as a cathode to complete the device. The device is sealed with a glass lid and a getter in an inert nitrogen atmosphere with less than 1 ppm of water and oxygen.

### OLED characterization

To characterize the OLED, electroluminescence spectra are recorded at various currents and voltages. In addition, the current-voltage characteristic is measured in combination with the luminance to determine luminous efficiency and external quantum efficiency (EQE). Driving voltage U and EQE are given at luminance (L) = 1000 cd/m² except otherwise stated.

### Application Example 1

Comparative Application Example 1 is repeated except that the exciton blocker (**SH-1**) is replaced by compound 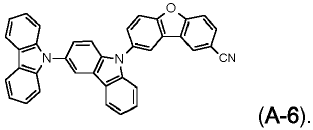 The device results are shown in Table 1.

### Application Example 2

Comparative Application Example 1 is repeated except that the exciton blocker (**SH-1**) is replaced by compound 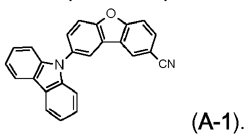 The device results are shown in Table 1.

### Application Example 3

Comparative Application Example 1 is repeated except that both the host (**SH-1**) and exciton blocker (**SH-1**) are replaced by compound 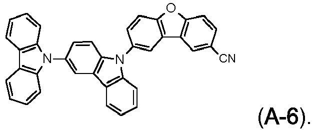 The device results are shown in Table 1.

### Application Example 4

Comparative Application Example 1 is repeated except that both the host (**SH-1**) and exciton blocker (**SH-1**) are replaced by compound 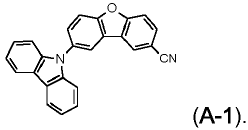 The device results are shown in Table 1.

**Table 1**

| **Appl. Ex.** | **Host** | **Exciton blocker** | **U [V]** | **EQE [%]** |
|---|---|---|---|---|
| **Comp. Appl. Ex. 1** | **(SH-1)** | **(SH-1)** | **5.42** | **17.1** |
| **Appl. Ex. 1** | **(SH-1)** | **(A-6)** | **4.47** | **17.2** |
| **Appl. Ex. 2** | **(SH-1)** | **(A-1)** | **4.50** | **17.5** |
| **Appl. Ex. 3** | **(A-6)** | **(A-6)** | **4.09** | **18.0** |
| **Appl. Ex. 4** | **(A-1)** | **(A-1)** | **4.02** | **18.5** |

The results shown in Table 1 demonstrate that the driving voltage U is significantly reduced with keeping EQE high enough when compounds (**A-6)** and (**A-1**) are used as the exciton blocker instead of reference compound (**SH-1**). Furthermore, when compounds (**A-6**) and (**A-1**) are used as both the host and exciton blocker at the same time, the driving voltage U is further reduced and EQE is also improved.

### Comparative Application Example 2

A glass substrate with 120 nm-thick ITO is cleaned and treated in the same manner as comparative application example 1. As a hole injection layer, compound 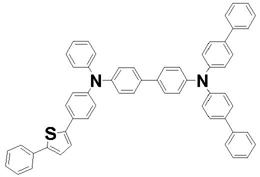 with 30 nm thickness is applied by vapor deposition. Then 60 nm of compound (**SH-1**) doped with MoOx (∼10%) is deposited as hole transporting layer. MoOx is used to improve the hole conductivity of **SH-1.** As an exciton and electron blocker, compound (**SH-1**) is applied with a thickness of 10 nm. Subsequently, a mixture of 20% by weight of emitter compound (**BE-1**) and 80% by weight of host (**SH-1**) are applied to form a 40 nm of emitting layer. On the emitting layer, 5 nm of material (**SH-1**) is applied as an exciton blocker. Thereafter, compound 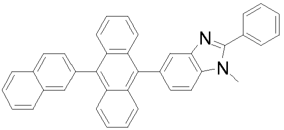 with 20 nm thickness is deposited as an electron transport layer. Finally, 1 nm of LiF is deposited as an electron injection layer and 80 nm of Al is then deposited as a cathode to complete the device. The device is sealed with a glass lid and a getter in an inert nitrogen atmosphere with less than 1 ppm of water and oxygen.

### Application Examples 5

Comparative Application Example 2 is repeated except that both the host (**SH-1**) and exciton blocker (**SH-1**) is replaced by compound 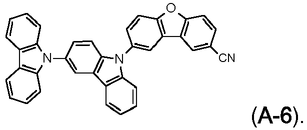 The device results are shown in Table 2.

### Application Examples 6

Comparative Application Example 2 is repeated except that both the host **(SH-1)** and exciton blocker (**SH-1**) is replaced by compound 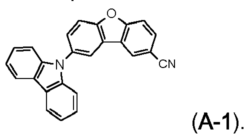 The device results are shown in Table 2.

**Table 2**

| **Appl. Ex.** | **Host** | **Exciton blocker** | **U [V]** | **EQE [%]** |
|---|---|---|---|---|
| **Comp. Appl. Ex. 2** | **(SH-1)** | **(SH-1)** | **5.54** | **2.0** |
| **Appl. Ex. 5** | **(A-6)** | **(A-6)** | **3.32** | **14.7** |
| **Appl. Ex. 6** | **(A-1)** | **(A-1)** | **3.58** | **19.8** |

The results shown in Table 2 demonstrate that the driving voltage and EQE are improved when compounds (**A-6**) and (**A-1**) are used as host and exciton blocker instead of reference compound (**SH-1**).

## Claims

1. A compound of the formula 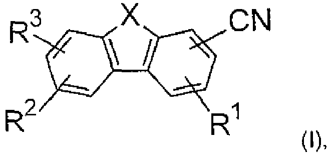 wherein
X is O, or S,
R¹ is H, Si(R²⁴)(R²⁵)(R²⁶), P(O)(R²⁷)(R²⁸), a C₁-C₂₅alkyl group, which can optionally be substituted by E, and or interrupted by D, a C₂-C₂₅alkenyl group, a C₂-C₂₅alkynyl group, -OR³², -SR³³, -COR³⁴, -COOR³⁵, a C₃-C₁₈cycloalkyl group, which can optionally be substituted by G; a group of formula 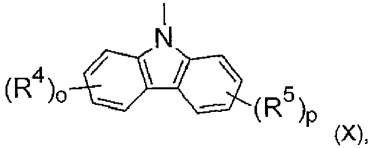 or 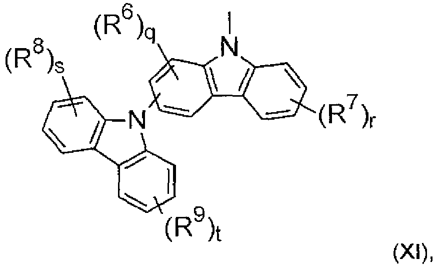
R² is H, Si(R²⁴)(R²⁵)(R²⁶), P(O)(R²⁷)(R²⁸), a C₁-C₂₅alkyl group, which can optionally be substituted by E, and or interrupted by D, a C₂-C₂₅alkenyl group, a C₂-C₂₅alkynyl group, -OR³², -SR³³, -COR³⁴, -COOR³⁵, a C₃-C₁₈cycloalkyl group, which can optionally be substituted by G; or a group of formula (X), or (XI),
R³ is H, Si(R²⁴)(R²⁵)(R²⁶), P(O)(R²⁷)(R²⁸), a C₁-C₂₅alkyl group, which can optionally be substituted by E, and or interrupted by D, a C₂-C₂₅alkenyl group, a C₂-C₂₅alkynyl group, -OR³², -SR³³, -COR³⁴, -COOR³⁵, a C₃-C₁₈cycloalkyl group, which can optionally be substituted by G; or a group of formula (X), or (XI),
o, p, r, s and t are independently of each other 0, or an integer 1 to 4,
q is 0, or an integer 1 to 3,
R⁴ and R⁵ may be the same or different in each occurence and are independently of each other H, CN, Si(R²⁴)(R²⁵)(R²⁶), P(O)(R²⁷)(R²⁸), N(R³⁰)(R³¹), a C₁-C₂₅alkyl group, which can optionally be substituted by E, and or interrupted by D, a C₂-C₂₅alkenyl group, a C₂-C₂₅alkynyl group, -OR³², -SR³³, -COR³⁴, -COOR³⁵, a C₃-C₁₈cycloalkyl group, or a group of formula 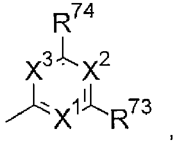 wherein
X¹, X² and X³ are independently of each other N, or CH,
R⁶, R⁷, R⁸ and R⁹ may be the same or different in each occurence and are independently of each other H, CN, Si(R²⁴)(R²⁵)(R²⁶), P(O)(R²⁷)(R²⁸), N(R³⁰)(R³¹), a C₁-C₂₅alkyl group, which can optionally be substituted by E, and or interrupted by D, a C₂-C₂₅alkenyl group, a C₂-C₂₅alkynyl group, -OR³², -SR³³, -COR³⁴, -COOR³⁵, a C₃-C₁₈cycloalkyl group, which can optionally be substituted by G; a C₆-C₂₄aryl group, which can optionally be substituted by G, or a C₂-C₃₀heteroaryl group, which can optionally be substituted by G;
R²⁴, R²⁵, R²⁶, R²⁷ and R²⁸ are independently of each other a C₁-C₂₅alkyl group, a C₆-C₂₄aryl group, which can optionally be substituted by G, or a C₂-C₃₀heteroaryl group, which can optionally be substituted by G;
R³⁰ and R³¹ are independently of each other a C₆-C₂₄aryl group, which can optionally be substituted by G, or a C₂-C₃₀heteroaryl group, which can optionally be substituted by G;
R³² and R³³ are independently of each other a C₆-C₁₈aryl group; a C₈-C₁₈aryl group, which is substituted by C₁-C₁₈alkyl group, or C₁-C₁₈alkoxy group; a C₁-C₁₈alkyl group; or a C₁-C₁₈alkyl group, which is interrupted by -O-;
R³⁴ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by - O-;
R³⁵ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-; D is -CO-, -COO-, -S-, -SO-, -SO₂-, -O-, -NR⁶⁵-, -SiR⁷⁰R⁷¹-, -POR⁷²-, -CR⁶³=CR⁶⁴-, or -C≡C-,
E is -OR⁶⁹, -SR⁶⁹, -NR⁶⁵R⁶⁶, -COR⁶⁸, -COOR⁵⁷, -CONR⁶⁵R⁶⁶, -CN, F, or NO₂;
G is E, or a C₁-C₁₈alkyl group, a C₃-C₁₈cycloalkyl group, a C₆-C₂₄aryl group, a C₆-C₂₄aryl group, which is substituted by F, -CN, C₁-C₁₈alkyl, or C₁-C₁₈alkyl which is interrupted by O; a C₂-C₅₀heteroaryl group, or a C₂-C₅₀heteroaryl group, which is substituted by F, -CN, C₁-C₁₈alkyl, C₁C₁₈alkyl which is interrupted by O;
R⁶³ and R⁶⁴ are independently of each other H, C₆-C₁₈aryl; C₆-C₁₈aryl which is substituted by C₁-C₁₈alkyl, or C₁-C₁₈alkoxy,- C₁-C₁₈alkyl; or C₁-C₁₈alkyl which is interrupted by -O-;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ together form a five or six membered ring;
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-;
R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by - O-;
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-;
R⁷⁰ and R⁷¹ are independently of each other a C₁-C₁₈alkyl group, a C₅-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl; and
R⁷³ and R⁷⁴ are independently of each other H, or a phenyl group.
with the proviso that one of R¹, R² and R³ is a group of formula (X), or (XI).

2. The compound according to claim 1, which is a compound of formula 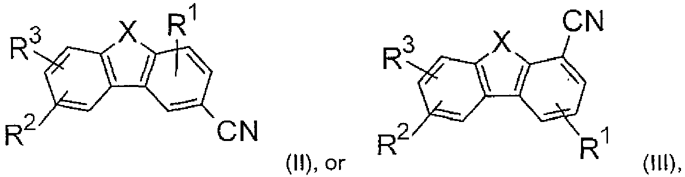 wherein X, R¹, R² and R³ are defined in claim 1.

3. The compound according to claim 1, which is a compound of formula 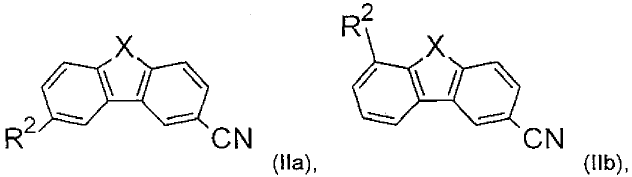 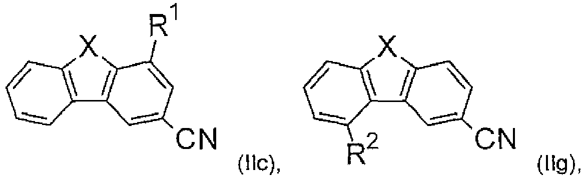 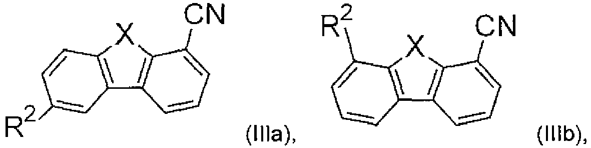 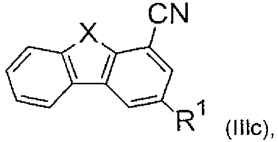 or 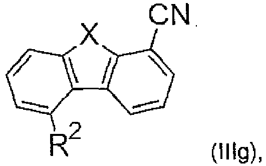 wherein X, R¹, R² and R³ are defined in claim 1.

4. The compound according to any of claims 1 to 3, wherein
R¹ is H, a group of formula (X), or (XI),
R² is H, a group of formula (X), or (XI), and
R³ is H, a group of formula (X), or (XI), wherein the group of formula (X), or (XI) is defined in claim 1.

5. The compound according to any of claims 1 to 4, wherein the group of formula (X) is a group of formula 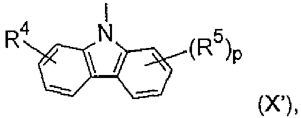
wherein p is 0, 1, or 2,
R⁴ and R⁵ are independently of each other H, CN, a C₁-C₂₅alkyl group, a group of formula 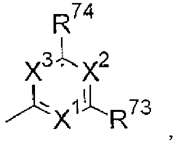 wherein
X¹, X² and X³ are independently of each other N, or CH, and
R⁷³ and R⁷⁴ are independently of each other H, or a phenyl group.

6. The compound according to claim 5, wherein the group of formula (X) is a group of formula 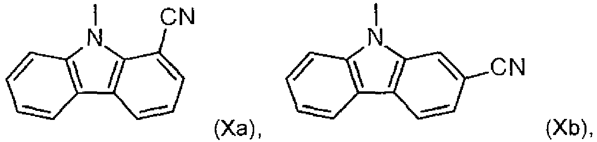 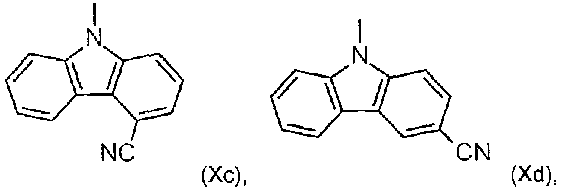 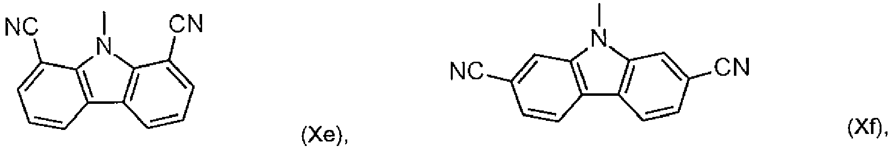 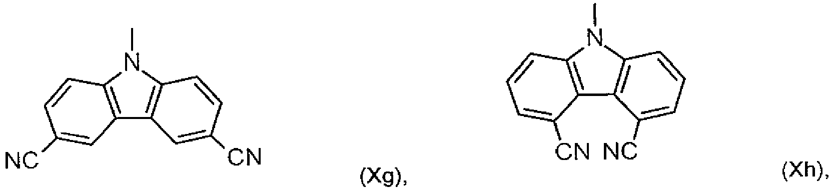 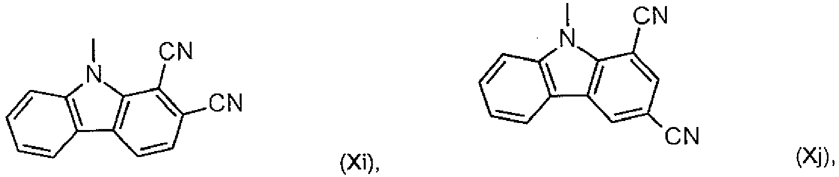 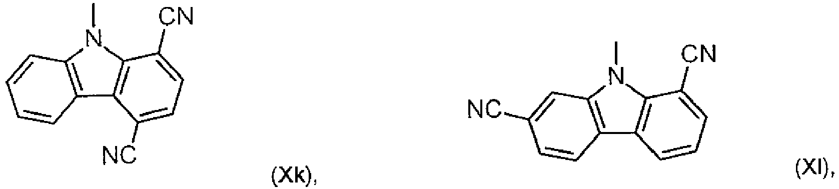 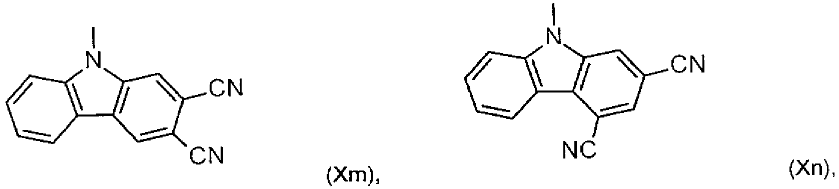 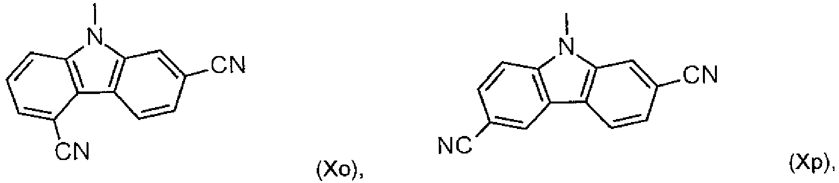 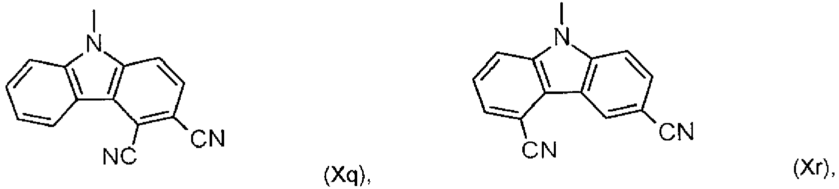 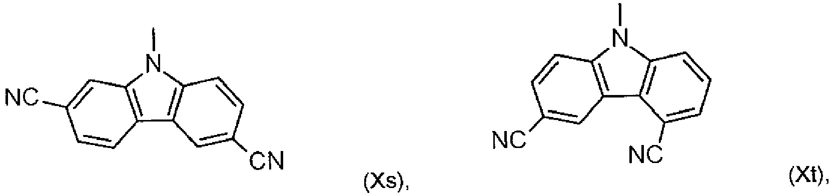 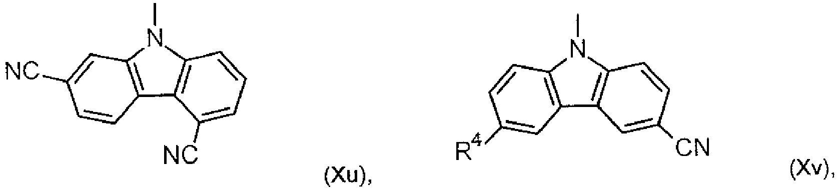 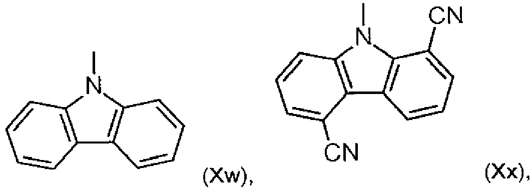 or 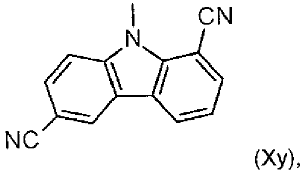 wherein R⁴ is a C₁-C₂₅alkyl group.

7. The compound according to any of claims 1 to 4, wherein the group of formula (XI) is a group of formula 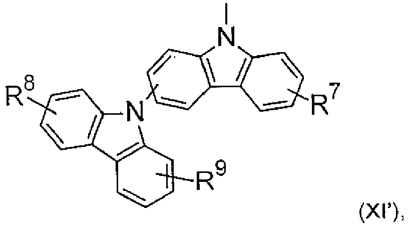 wherein
R⁷ is H, CN, or a C₁-C₂₅alkyl group;
R⁸ is H, CN, or a C₁-C₂₅alkyl group; and
R⁹ is H, CN, or a C₁-C₂₅alkyl group;

8. The compound according to claim 7, wherein the group of formula (XI) is a group of formula 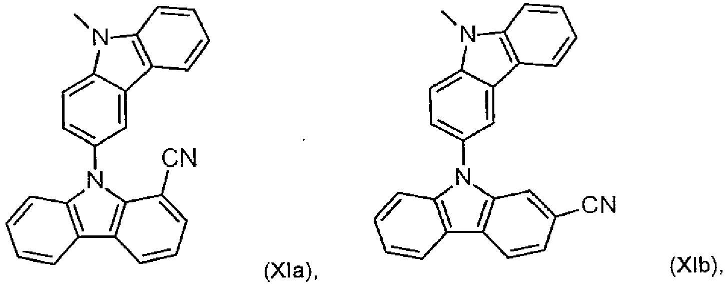 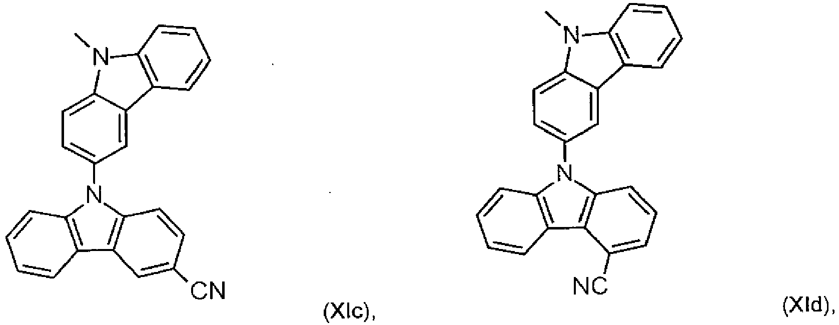 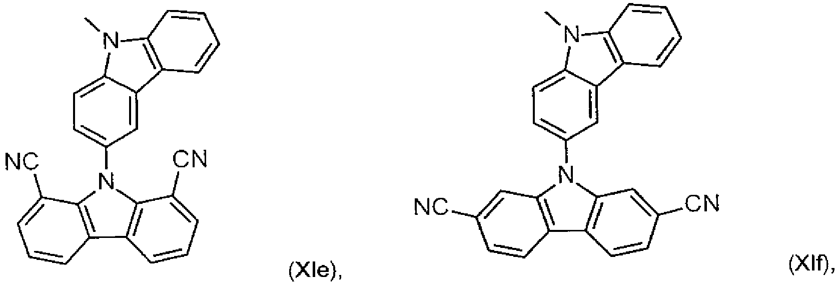 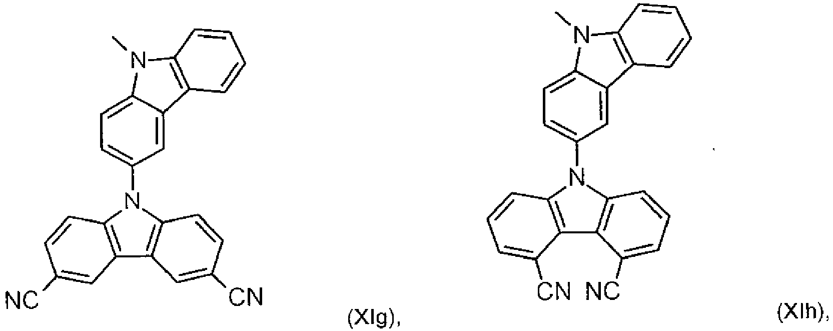 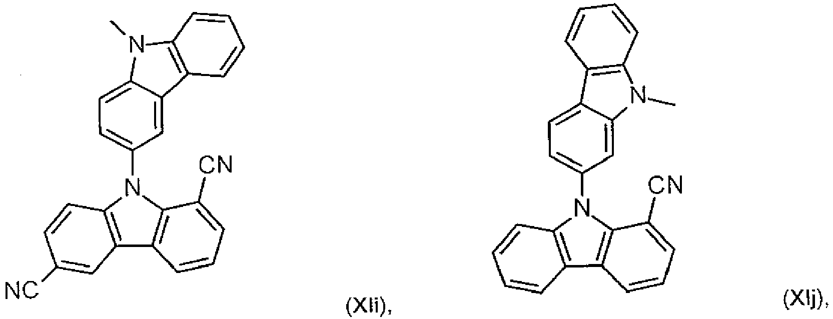 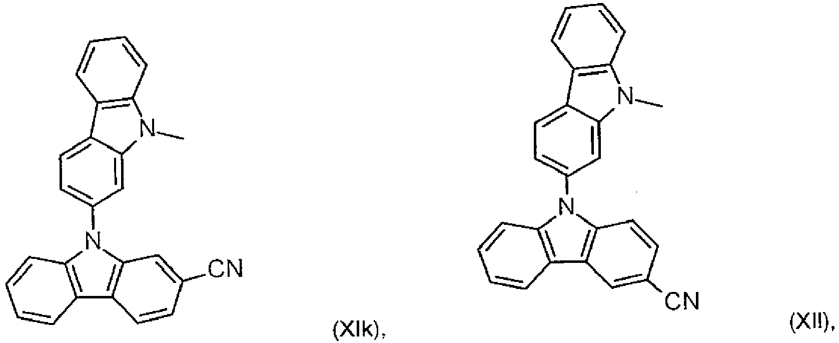 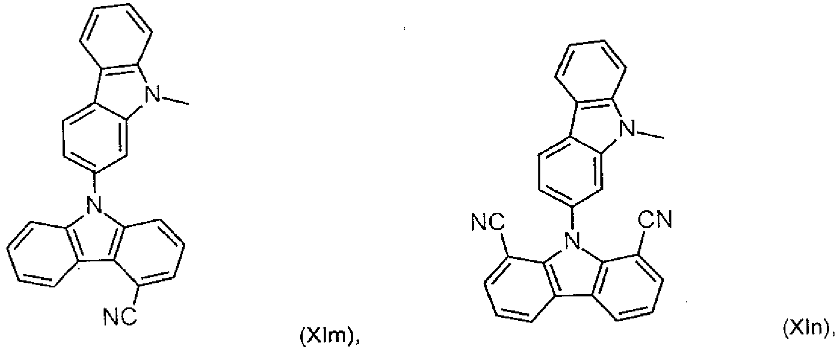 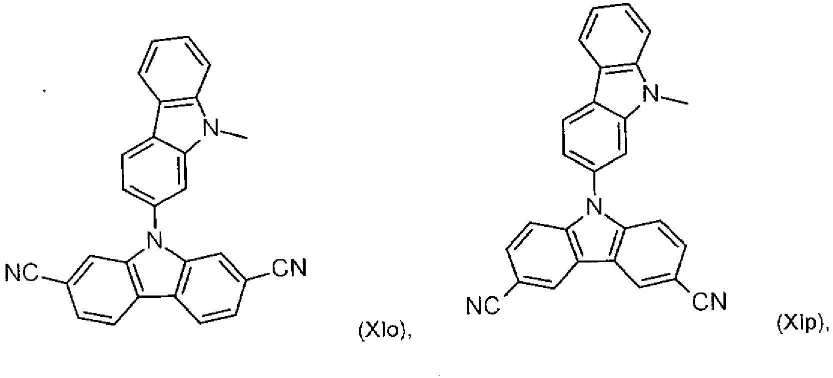 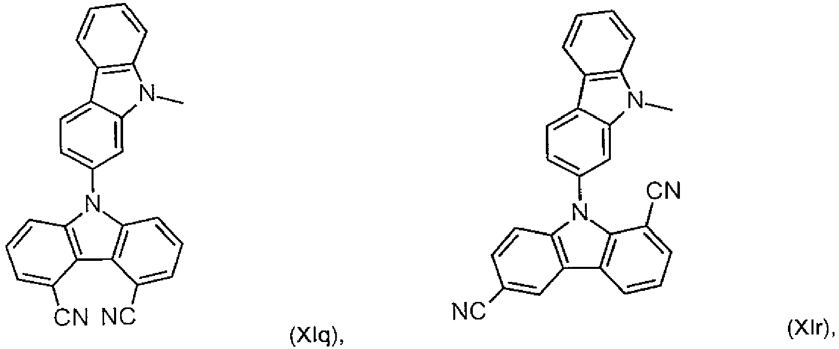 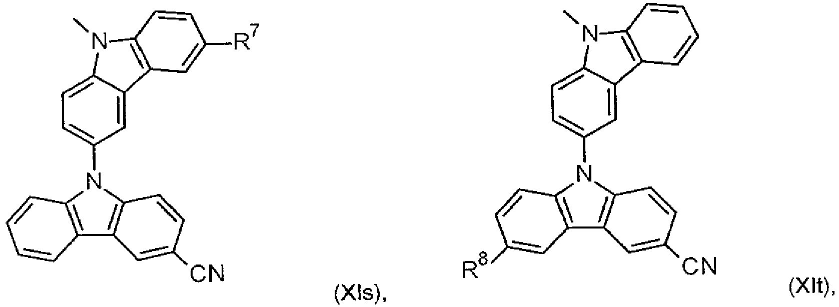 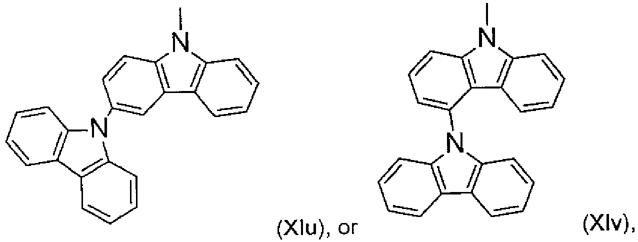 wherein R⁷ is a C₁-C₂₅alkyl group and R⁸ is a C₁-C₂₅alkyl group.

9. The compound according to any of claims 1 to 8, wherein X is O.

10. The compound according to any of claims 1 to 9, which is a compound of formula 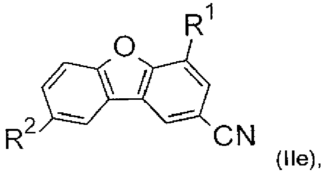 wherein
R¹ is H, and R² is a group of formula (Xa), (Xb), (Xc), (Xd), (Xf), (Xg), (Xv), or (Xw) as defined in claim 7; or a group of formula (XIa), (XIb), (XIc), (XId), (XIf), (XIg), (XIs), (XIt), (XIu), or (XIv) as defined in claim 9; or
R² is H, and R¹ is a group of formula (Xa), (Xb), (Xc), (Xd), (Xf), (Xg), (Xv), or (Xw) as defined in claim 7; or a group of formula (XIa), (XIb), (XIc), (XId), (XIf), (XIg), (XIs), (XIt), (XIu), or (XIv) as defined in claim 9.

11. An electronic device, comprising a compound according to any of claims 1 to 10.

12. The electronic device according to claim 11, which is an electroluminescent device.

13. An electron transport layer, hole blocking layer, or an emitting layer comprising a compound according to any of claims 1 to 10.

14. The emitting layer according to claim 13, comprising a compound according to any of claims 1 to 11 as host material in combination with a phosphorescent emitter.

15. An apparatus selected from the group consisting of stationary visual display units; mobile visual display units; illumination units; keyboards; items of clothing; furniture; wallpaper, comprising the organic electronic device according to claim 11, or 12, or the electron transport layer, hole blocking layer and/or the emitting layer according to claim 13.

16. Use of the compounds of formula I according to any of claims 1 to 10 for electrophotographic photoreceptors, photoelectric converters, organic solar cells, switching elements, organic light emitting field effect transistors, image sensors, dye lasers, electroluminescent devices, as transport material in hole blocking layer and/or emissive layer and/or as electron blocking layer.

## Patentansprüche

1. Verbindung der Formel 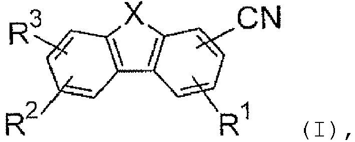 wobei
X für O oder S steht,
R¹ für H, Si (R²⁴) (R²⁵) (R²⁶), P(O)(R²⁷)(R²⁸), eine C₁-C₂₅-Alkylgruppe, die gegebenenfalls durch E substituiert und/oder durch D unterbrochen sein kann, eine C₂-C₂₅-Alkenylgruppe, eine C₂-C₂₅-Alkinylgruppe, -OR³², -SR³³, -COR³⁴, -COOR³⁵, eine C₃-C₁₈-Cycloalkylgruppe, die gegebenenfalls durch G substituiert sein kann, oder eine Gruppe der Formel 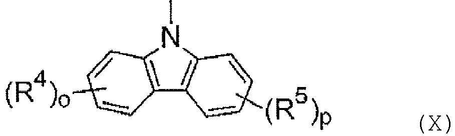 oder 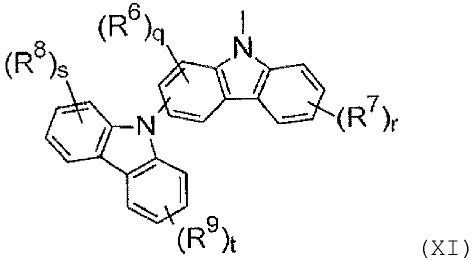 steht,
R² für H, Si (R²⁴) (R²⁵) (R²⁶) , P(O)(R²⁷)(R²⁸), eine C₁-C₂₅-Alkylgruppe, die gegebenenfalls durch E substituiert und/oder durch D unterbrochen sein kann, eine C₂-C₂₅-Alkenylgruppe, eine C₂-C₂₅-Alkinylgruppe, -OR³², -SR³³, -COR³⁴, -COOR³⁵, eine C₃-C₁₈-Cycloalkylgruppe, die gegebenenfalls durch G substituiert sein kann, oder eine Gruppe der Formel (X) oder (XI) steht,
R³ für H, Si(R²⁴) (R²⁵) (R²⁶), P(O)(R²⁷)(R²⁸), eine C₁-C₂₅-Alkylgruppe, die gegebenenfalls durch E substituiert und/oder durch D unterbrochen sein kann, eine C₂-C₂₅-Alkenylgruppe, eine C₂-C₂₅-Alkinylgruppe, -OR³², -SR³³, -COR³⁴, -COOR³⁵, eine C₃-C₁₈-Cycloalkylgruppe, die gegebenenfalls durch G substituiert sein kann, oder eine Gruppe der Formel (X) oder (XI) steht,
o, p, r, s und t unabhängig voneinander für 0 oder eine ganze Zahl von 1 bis 4 stehen,
q für 0 oder eine ganze Zahl von 1 bis 3 steht,
R⁴ und R⁵ bei jedem Auftreten gleich oder verschieden sein können und unabhängig voneinander für H, CN, Si (R²⁴) (R²⁵) (R²⁶), P(O)(R²⁷)(R²⁸), N(R³⁰)(R³¹), eine C₁-C₂₅-Alkylgruppe, die gegebenenfalls durch E substituiert und/oder durch D unterbrochen sein kann, eine C₂-C₂₅-Alkenylgruppe, eine C₂-C₂₅-Alkinylgruppe, -OR³², -SR³³, -COR³⁴ -COOR³⁵, eine C₃-C₁₈-Cycloalkylgruppe oder eine Gruppe der Formel 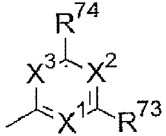 steht, wobei
X¹, X² und X³ unabhängig voneinander für N oder CH stehen,
R⁶, R⁷, R⁸ und R⁹ bei jedem Auftreten gleich oder verschieden sein können und unabhängig voneinander für H, CN, Si(R²⁴)(R²⁵)(R²⁶), P(O)(R²⁷)(R²⁸), N(R³⁰)(R³¹), eine C₁-C₂₅-Alkylgruppe, die gegebenenfalls durch E substituiert und/oder durch D unterbrochen sein kann, eine C₂-C₂₅-Alkenylgruppe, eine C₂-C₂₅-Alkinylgruppe, -OR³², -SR³³, -COR³⁴, -COOR³⁵, eine C₃-C₁₈-Cycloalkylgruppe, die gegebenenfalls durch G substituiert sein kann, eine C₆-C₂₄-Arylgruppe, die gegebenenfalls durch G substituiert sein kann, oder eine C₂-C₃₀-Heteroarylgruppe, die gegebenenfalls durch G substituiert sein kann, stehen,
R²⁴, R²⁵, R²⁶, R²⁷ und R²⁸ unabhängig voneinander für eine C₁-C₂₅-Alkylgruppe, eine C₆-C₂₄-Arylgruppe, die gegebenenfalls durch G substituiert sein kann, oder eine C₂-C₃₀-Heteroarylgruppe, die gegebenenfalls durch G substituiert sein kann, stehen,
R³⁰ und R³¹ unabhängig voneinander für eine C₆-C₂₄-Arylgruppe, die gegebenenfalls durch G substituiert sein kann, oder eine C₂-C₃₀-Heteroaryl-gruppe, die gegebenenfalls durch G substituiert sein kann, stehen,
R³² und R³³ unabhängig voneinander für eine C₆-C₁₈-Arylgruppe, eine C₆-C₁₈-Arylgruppe, die durch eine C₁-C₁₈-Alkylgruppe oder C₁-C₁₈-Alkoxygruppe substituiert ist, eine C₁-C₁₈-Alkylgruppe oder eine C₁-C₁₈-Alkylgruppe, die durch -O- unterbrochen ist, stehen,
R³⁴ für H, eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O-unterbrochen ist, steht,
R³⁵ für eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O-unterbrochen ist, steht,
D für -CO-, -COO-, -S-, -SO-, -SO₂-, -O-, -NR⁶⁵--SiR⁷⁰R⁷¹-, POR⁷²-, -CR⁶³=CR⁶⁴- oder -C≡C- steht,
E für -OR⁶⁹, -SR⁶⁹, -NR⁶⁵R⁶⁶, -COR⁶⁸, -COOR⁶⁷, -CONR⁶⁵R⁶⁶, -CN, F oder NO₂ steht,
G für E oder eine C₁-C₁₈-Alkylgruppe, eine C₃-C₁₈-Cycloalkylgruppe, eine C₆-C₂₄-Arylgruppe, eine C₆-C₂₄-Arylgruppe, die durch F, -CN, C₁-C₁₈-Alkyl oder C₁-C₁₈-Alkyl, das durch O unterbrochen ist, substituiert ist, eine C₂-C₅₀-Heteroarylgruppe oder eine C₂-C₅₀-Heteroarylgruppe, die durch F, -CN, C₁-C₁₈-Alkyl oder C₁-C₁₈-Alkyl, das durch O unterbrochen ist, substituiert ist, steht,
R⁶³ und R⁶⁴ unabhängig voneinander für H, C₆-C₁₈-Aryl, ein C₆-C₁₈-Aryl, das durch C₁-C₁₈-Alkyl oder C₁-C₁₈-Alkoxy substituiert ist, C₁-C₁₈-Alkyl oder C₁-C₁₈-Alkyl, das durch -O- unterbrochen ist, stehen, R⁶⁵ und R⁶⁶ unabhängig voneinander für eine C₆-C₁₈-Arylgruppe, ein C₆-C₁₈-Aryl, das durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, stehen oder
R⁶⁵ und R⁶⁶ zusammen einen fünf- oder sechsgliedrigen Ring bilden,
R⁶⁷ für eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht,
R⁶⁸ für H, eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht,
R⁶⁹ für ein C₆-C₁₈-Aryl, ein C₆-C₁₈-Aryl, das durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht,
R⁷⁰ und R⁷¹ unabhängig voneinander für eine C₁-C₁₈-Alkylgruppe, eine C₆-C₁₈-Arylgruppe oder eine C₆-C₁₈-Arylgruppe, die durch C₁-C₁₈-Alkyl substituiert ist, stehen und
R⁷² für eine C₁-C₁₈-Alkylgruppe, eine C₆-C₁₈-Arylgruppe oder eine C₆-C₁₈-Arylgruppe, die durch C₁-C₁₈-Alkyl substituiert ist, steht und
R⁷³ und R⁷⁴ unabhängig voneinander für H oder eine Phenylgruppe stehen,
mit der Maßgabe, dass mindestens eine der Variablen R¹, R² und R³ für eine Gruppe der Formel (X) oder (XI) steht.

2. Verbindung nach Anspruch 1, bei der es sich um eine Verbindung der Formel 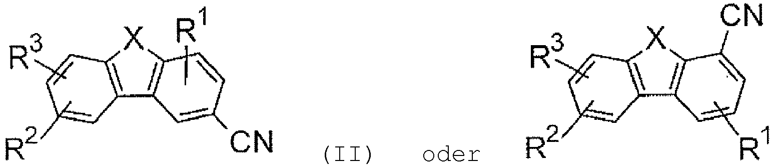 (III) handelt, wobei X, R¹, R² und R³ wie in Anspruch 1 definiert sind.

3. Verbindung nach Anspruch 1, bei der es sich um eine Verbindung der Formel 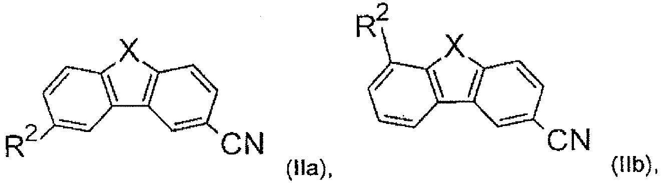 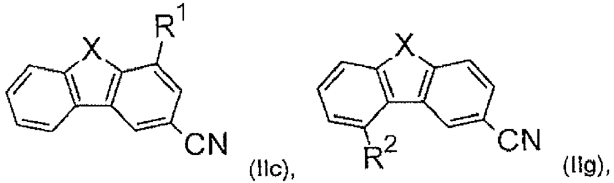 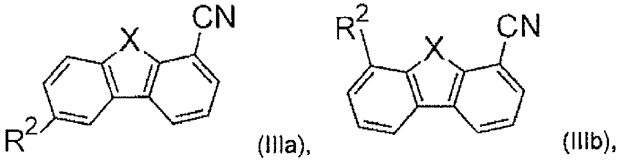 oder 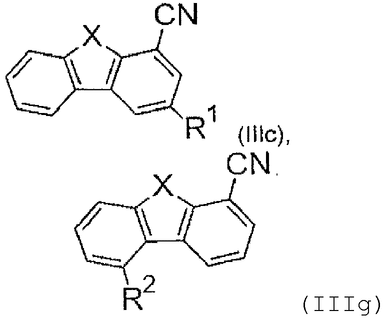 handelt, wobei X, R¹, R² und R³ wie in Anspruch 1 definiert sind.

4. Verbindung nach einem der Ansprüche 1 bis 3, wobei
R¹ für H oder eine Gruppe der Formel (X) oder (XI) steht,
R² für H oder eine Gruppe der Formel (X) oder (XI) steht und
R³ für H oder eine Gruppe der Formel (X) oder (XI) steht, wobei die Gruppe der Formel (X) oder (XI) in Anspruch 1 definiert ist.

5. Verbindung nach einem der Ansprüche 1 bis 4, wobei es sich bei der Gruppe der Formel (X) um eine Gruppe der Formel 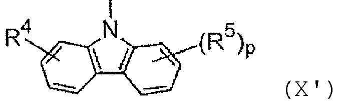 handelt,
wobei p für 0, 1 oder 2 steht, R⁴ und R⁵ unabhängig voneinander für H, CN, eine C₁-C₂₅-Alkylgruppe oder eine Gruppe der Formel 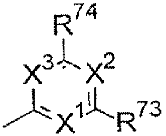 stehen, wobei
X¹, X² und X³ unabhängig voneinander für N oder CH stehen und
R⁷³ und R⁷⁴ unabhängig voneinander für H oder eine Phenylgruppe stehen.

6. Verbindung nach 5, wobei es sich bei der Gruppe der Formel (X) um eine Gruppe der Formel 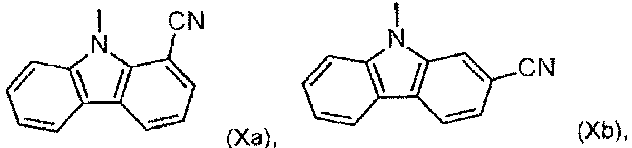 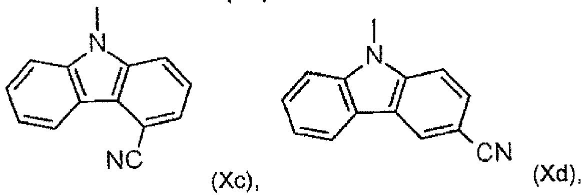 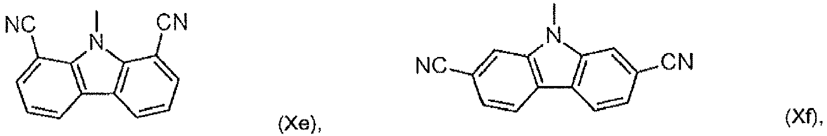 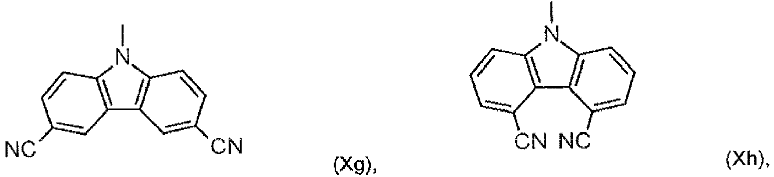 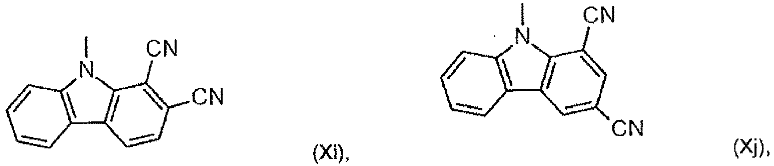 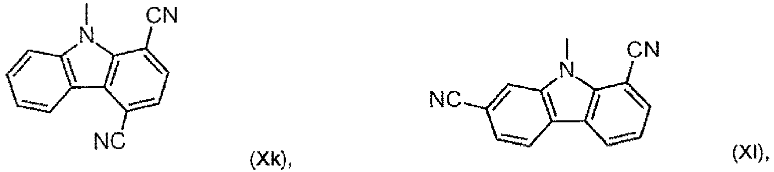 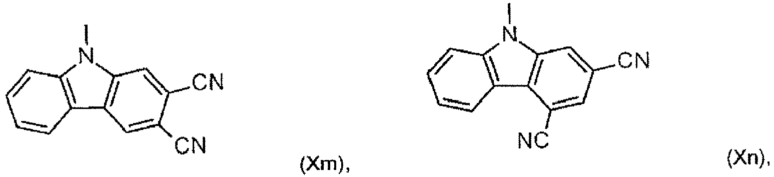 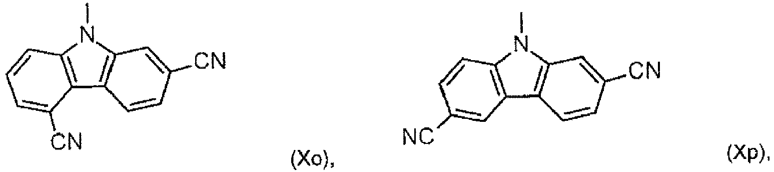 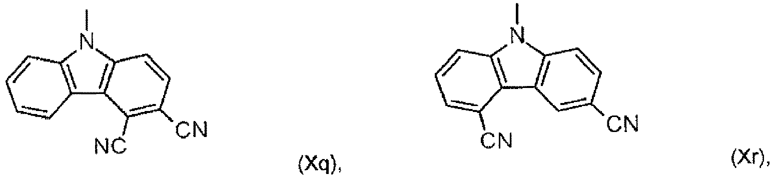 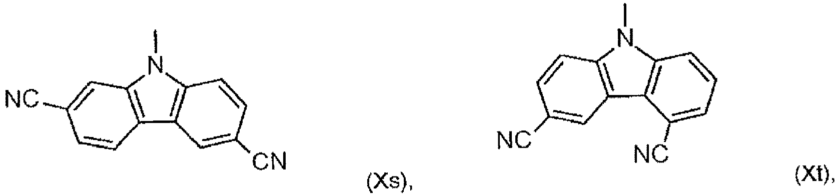 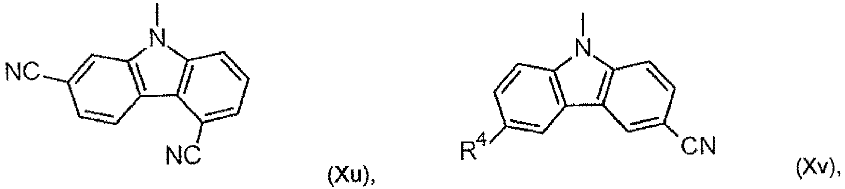 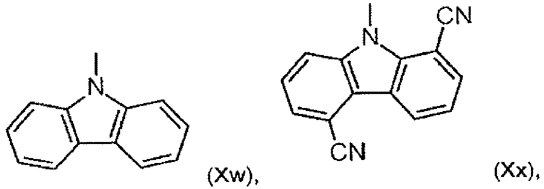 oder 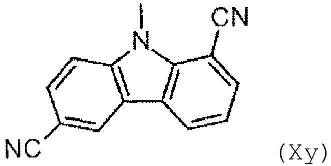 handelt, wobei R⁴ für eine C₁-C₂₅-Alkylgruppe steht.

7. Verbindung nach einem der Ansprüche 1 bis 4, wobei es sich bei der Gruppe der Formel (XI) um eine Gruppe der Formel 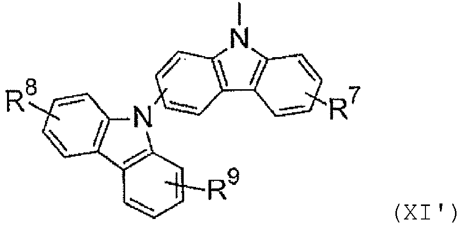 handelt, wobei
R⁷ für H, CN oder eine C₁-C₂₅-Alkylgruppe steht,
R⁸ für H, CN oder eine C₁-C₂₅-Alkylgruppe steht und
R⁹ für H, CN oder eine C₁-C₂₅-Alkylgruppe steht.

8. Verbindung nach Anspruch 7, wobei es sich bei der Gruppe der Formel (XI) um eine Gruppe der Formel 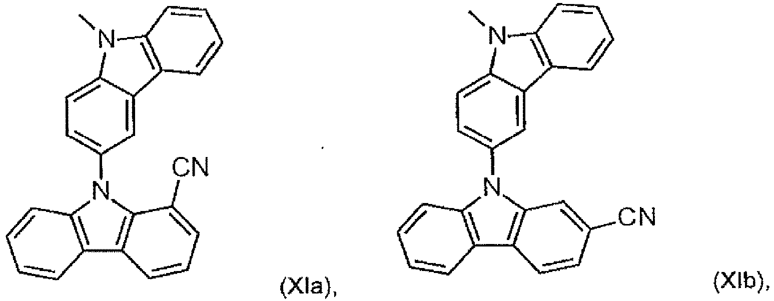 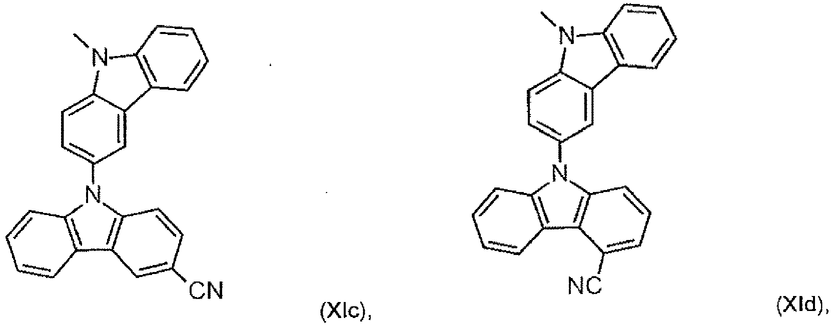 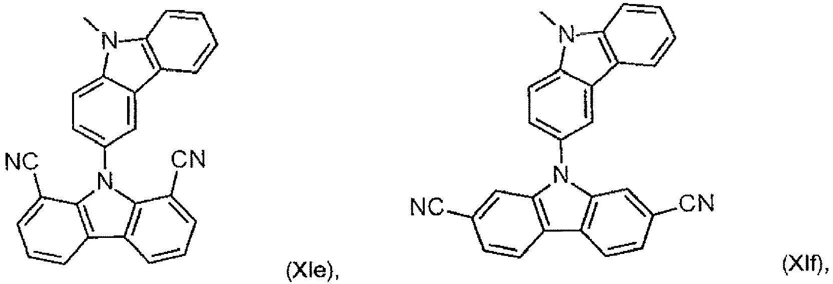 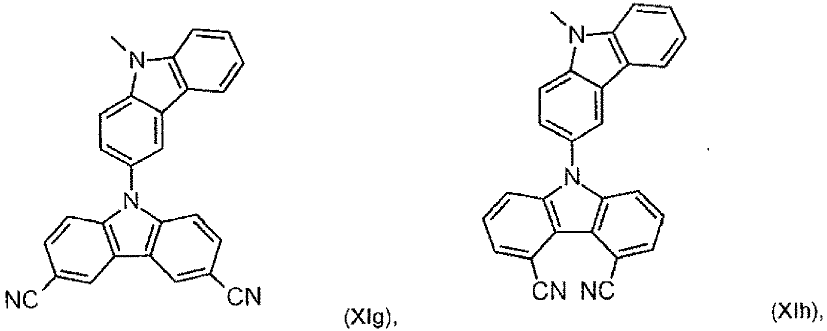 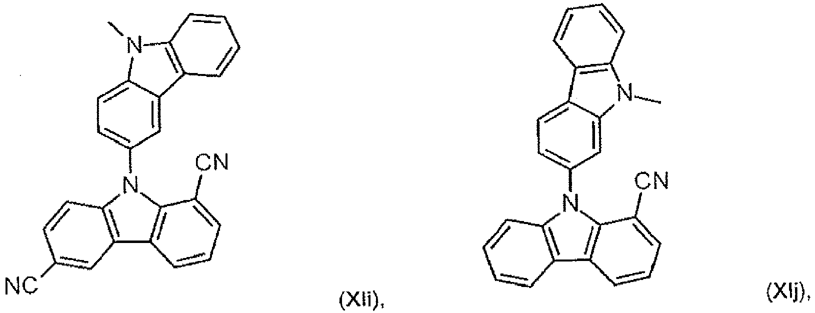 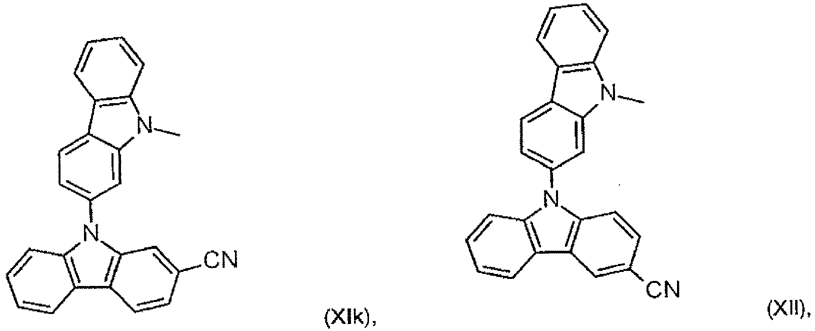 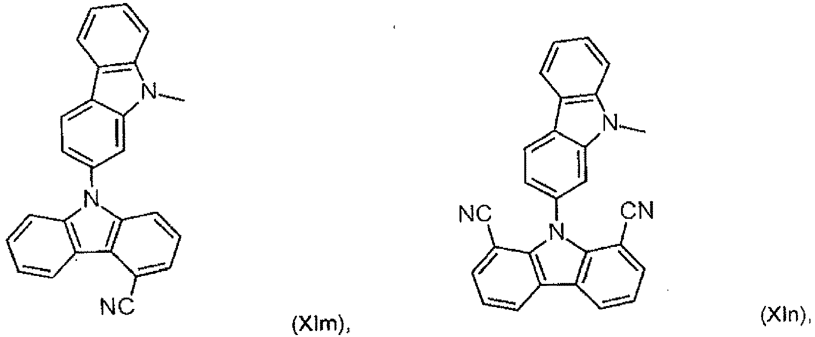 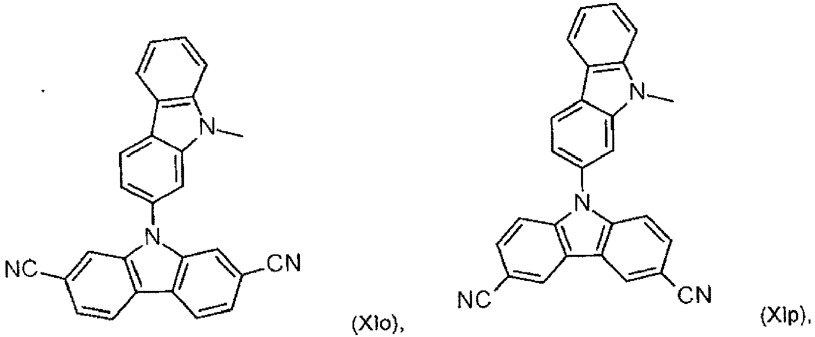 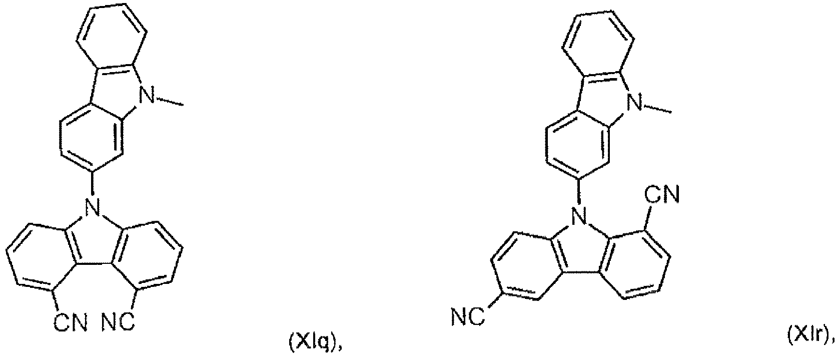 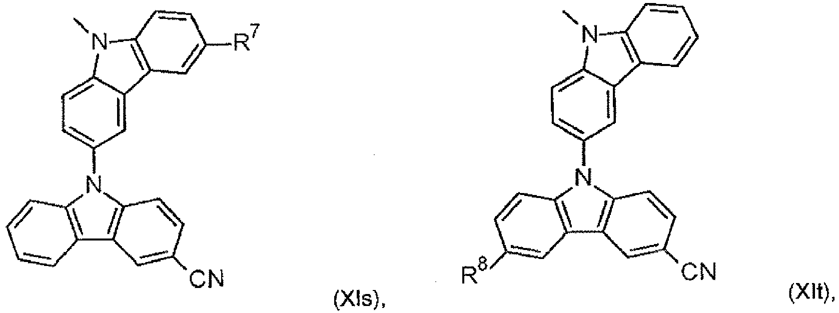 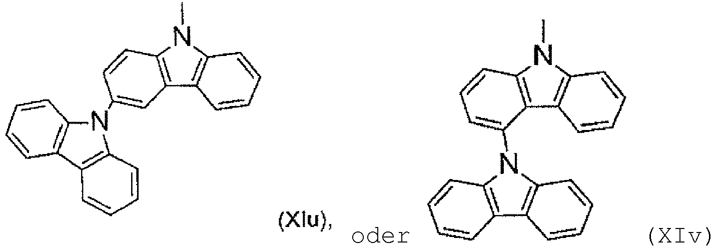 handelt, wobei R⁷ für eine C₁-C₂₅-Alkylgruppe steht und R⁸ für eine C₁-C₂₅-Alkylgruppe steht.

9. Verbindung nach einem der Ansprüche 1 bis 8, wobei X für O steht.

10. Verbindung nach einem der Ansprüche 1 bis 9, bei der es sich um eine Verbindung der Formel 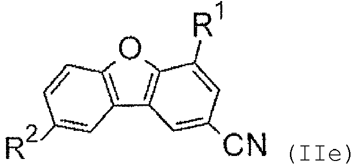 handelt, wobei
R¹ für H steht und R² für eine Gruppe der Formel (Xa), (Xb), (Xc), (Xd), (Xf), (Xg), (Xv) oder (Xw) gemäß Anspruch 7 oder eine Gruppe der Formel (XIa), (XIb), (XIc), (XId), (XIf), (XIg), (XIs), (XIt), (XIu) oder (XIv) gemäß Anspruch 9 steht oder
R² für H steht und R¹ für eine Gruppe der Formel (Xa), (Xb), (Xc), (Xd), (Xf), (Xg), (Xv) oder (Xw) gemäß Anspruch 7 oder eine Gruppe der Formel (XIa), (XIb), (XIc), (XId), (XIf), (XIg), (XIs), (XIt), (XIu) oder (XIv) gemäß Anspruch 9 steht.

11. Elektronische Vorrichtung, umfassend eine Verbindung nach einem der Ansprüche 1 bis 10.

12. Elektronische Vorrichtung nach Anspruch 11, bei der es sich um eine Elektrolumineszenzvorrichtung handelt.

13. Elektronentransportschicht, Lochblockierungsschicht oder Emissionsschicht, umfassend eine Verbindung nach einem der Ansprüche 1 bis 10.

14. Emissionsschicht nach Anspruch 13, umfassend eine Verbindung nach einem der Ansprüche 1 bis 11 als Wirtsmaterial in Kombination mit einem phosphoreszierenden Emitter.

15. Apparatur, ausgewählt aus der Gruppe bestehend aus stationären Bildschirmen, mobilen Bildschirmen, Beleuchtungseinheiten, Tastaturen, Kleidungsstücken, Möbeln und Tapeten, umfassend die organische elektronische Vorrichtung nach Anspruch 11 oder 12 oder die Elektronentransportschicht, die Lochblockierungsschicht und/oder die Emissionsschicht nach Anspruch 13.

16. Verwendung der Verbindungen der Formel I nach einem der Ansprüche 1 bis 10 für elektrophotographische Photorezeptoren, photoelektrische Umwandler, organische Solarzellen, Schaltelemente, organische lichtemittierende Feldeffekttransistoren, Bildsensoren, Farbstofflaser und Elektrolumineszenzvorrichtungen, als Transportmaterial in einer Lochblockierungsschicht und/oder Emissionsschicht und/oder als Elektronenblockierungsschicht.

## Revendications

1. Composé de la formule
X étant O, ou S, 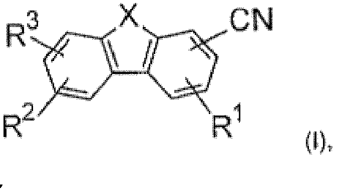
R¹ étant H, Si (R²⁴)(R²⁵)(R²⁶), P(O)(R²⁷)(R²⁸), un groupe C₁₋₂₅-alkyle, qui peut éventuellement être substitué par E, et/ou interrompu par D, un groupe C₂₋₂₅-alcényle, un groupe C₂₋₂₅-alcynyle, -OR³², - SR³³, -COR³⁴, -COOR³⁵, un groupe C₃₋₁₈-cycloalkyle, qui peut éventuellement être substitué par G ; un groupe de formule 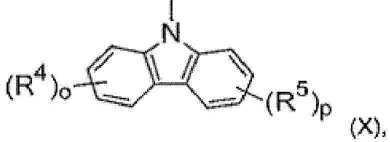 ou 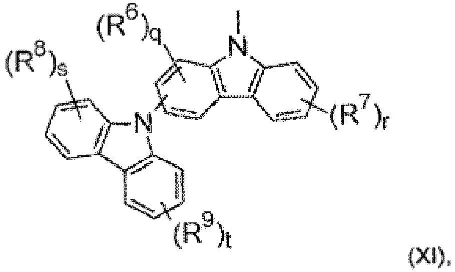
R² étant H, Si(R²⁴)(R²⁵)(R²⁶), P(O)(R²⁷)(R²⁸), un groupe C₁₋₂₅-alkyle, qui peut éventuellement être substitué par E, et/ou interrompu par D, un groupe C₂₋₂₅-alcényle, un groupe C₂₋₂₅-alcynyle, -OR³², - SR³³, -COR³⁴, -COOR³⁵, un groupe C₃₋₁₈-cycloalkyle, qui peut éventuellement être substitué par G ; ou un groupe de formule (X), ou (XI),
R² étant H, Si(R²⁴)(R²⁵)(R²⁶), P(O)(R²⁷)(R²⁸), un groupe C₁₋₂₅-alkyle, qui peut éventuellement être substitué par E, et/ou interrompu par D, un groupe C₂₋₂₅-alcényle, un groupe C₂₋₂₅-alcynyle, -OR³², - SR³³, -COR³⁴, -COOR³⁵, un groupe C₃₋₁₈-cycloalkyle, qui peut éventuellement être substitué par G ; ou un groupe de formule (X), ou (XI),
o, p, r, s et t étant indépendamment l'un de l'autre 0, ou un entier de 1 à 4,
q étant 0, ou un entier de 1 à 3,
R⁴ et R⁵ pouvant être identiques ou différents en chaque occurrence et étant indépendamment l'un de l'autre H, CN, Si(R²⁴)(R²⁵)(R²⁶), P(O)(R²⁷)(R²⁸), N(R³⁰)(R³¹), un groupe C₁₋₂₅-alkyle, qui peut éventuellement être substitué par E, et/ou interrompu par D, un groupe C₂₋₂₅-alcényle, un groupe C₂₋₂₅-alcynyle, -OR³², -SR³³, -COR³⁴, -COOR³⁵, un groupe C₃₋₁₈-cycloalkyle, ou un groupe de formule 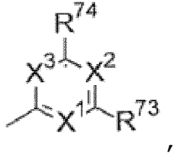
X¹, X² et X³ étant indépendamment l'un de l'autre N ou CH,
R⁶, R⁷, R⁸ et R⁹ pouvant être identiques ou différents en chaque occurrence et étant indépendamment les uns des autres H, CN, Si(R²⁴)(R²⁵)(R²⁶), P(O)(R²⁷)(R²⁸), N(R³⁰)(R³¹), un groupe C₁₋₂₅-alkyle, qui peut éventuellement être substitué par E, et/ou interrompu par D, un groupe C₂₋₂₅-alcényle, un groupe C₂₋₂₅-alcynyle, -OR³², - SR³³, -COR³⁴, -COOR³⁵, un groupe C₃₋₁₈-cycloalkyle, qui peut éventuellement être substitué par G ; un groupe C₆₋₂₄-aryle, qui peut éventuellement être substitué par G, ou un groupe C₂₋₃₀-hétéroaryle, qui peut éventuellement être substitué par G ;
R²⁴, R²⁵, R²⁶, R²⁷ et R²⁸ étant indépendamment l'un de l'autre un groupe C₁₋₂₅-alkyle, un groupe C₆₋₂₄-aryle, qui peut éventuellement être substitué par G, ou un groupe C₂₋₃₀-hétéroaryle, qui peut éventuellement être substitué par G ;
R³⁰ et R³¹ étant indépendamment l'un de l'autre un groupe C₆₋₂₄-aryle, qui peut éventuellement être substitué par G, ou un groupe C₂₋₃₀-hétéroaryle, qui peut éventuellement être substitué par G ;
R³² et R³³ étant indépendamment l'un de l'autre un groupe C₆₋₁₈-aryle ; un groupe C₆₋₁₈-aryle, qui est substitué par un groupe C₁₋₁₈-alkyle, ou par un groupe C₁₋₁₈-alcoxyle ; un groupe C₁₋₁₈-alkyle ; ou un groupe C₁₋₁₈-alkyle, qui est interrompu par -O- ; R³⁴ étant H ; un groupe C₆₋₁₈-aryle, qui est substitué par C₁₋₁₈-alkyle, ou par C₁₋₁₈-alcoxyle ; un groupe C₁₋₁₈-alkyle ; ou un groupe C₁₋₁₈-alkyle, qui est interrompu par -O- ;
R³⁵ étant un groupe C₆₋₁₈-aryle ; un groupe C₆₋₁₈-aryle, qui est substitué par C₁₋₁₈-alkyle, ou par C₁₋₁₈-alcoxyle ; un groupe C₁₋₁₈-alkyle ; ou un groupe C₁₋₁₈-alkyle, qui est interrompu par -O- ;
D étant -CO-, -COO-, -S-, -SO-, -SO₂-, -O-, -NR⁶⁵-, -SiR⁷⁰R⁷¹, -POR⁷²-, -CR⁶³=CR⁶⁴-, ou -C≡C-,
E étant -OR⁶⁹, -SR⁶⁹, -NR⁶⁵R⁶⁶, -COR⁶⁸, -COOR⁶⁷, - CONR⁶⁵R⁶⁶, -CN, F ou NO₂ ;
G étant E, ou un groupe C₁₋₁₈-alkyle, un groupe C₃-₁₈-cycloalkyle, un groupe C₆₋₂₄-aryle, un groupe C₆₋₂₄-aryle, qui est substitué par F, -CN, C₁₋₁₈-alkyle, ou C₁₋₁₈-alkyle qui est interrompu par O ; un groupe C₂₋₅₀-hétéroaryle, ou un groupe C₂₋₅₀-hétéroaryle, qui est substitué par F, -CN, C₁₋₁₈-alkyle, ou C₁₋₁₈-alkyle qui est interrompu par O ; R⁶³ et R⁶⁴ étant indépendamment l'un de l'autre H, C₆₋₁₈-aryle ; C₆₋₁₈-aryle qui est substitué par C₁₋₁₈-alkyle, ou C₁₋₁₈-alcoxyle ; C₁₋₁₈-alkyle ; ou C₁₋₁₈-alkyle qui est interrompu par -O- ;
R⁶⁵ et R⁶⁶ étant indépendamment l'un de l'autre un groupe C₆₋₁₈-aryle ; un C₆₋₁₈-aryle, qui est substitué par C₁₋₁₈-alkyle, ou C₁₋₁₈-alcoxyle ; un groupe C₁₋₁₈-alkyle ; ou un groupe C₁₋₁₈-alkyle, qui est interrompu par -O- ; ou
R⁶⁵ et R⁶⁶ formant ensemble un cycle à cinq ou six chaînons ;
R⁶⁷ étant un groupe C₆₋₁₈-aryle ; un groupe C₆₋₁₈-aryle, qui est substitué par C₁₋₁₈-alkyle, ou C₁₋₁₈-alcoxyle ; un groupe C₁₋₁₈-alkyle ; ou un groupe C₁₋₁₈-alkyle, qui est interrompu par -O- ;
R⁶⁸ étant H ; un groupe C₆₋₁₈-aryle ; un groupe C₆₋₁₈-aryle, qui est substitué par C₁₋₁₈-alkyle, ou C₁₋₁₈-alcoxyle ; un groupe C₁₋₁₈-alkyle ; ou un groupe C₁₋₁₈-alkyle, qui est interrompu par -O- ;
R⁶⁹ étant un C₆₋₁₈-aryle ; un C₆₋₁₈-aryle, qui est substitué par C₁₋₁₈-alkyle, ou C₁₋₁₈-alcoxyle ; un groupe C₁₋₁₈-alkyle ; ou un groupe C₁₋₁₈-alkyle, qui est interrompu par -O- ;
R⁷⁰ et R⁷¹ étant indépendamment l'un de l'autre un groupe C₁₋₁₈-alkyle, un groupe C₆₋₁₈-aryle ou un C₆₋₁₈-aryle, qui est substitué par C₁₋₁₈-alkyle, et
R⁷² étant un groupe C₁₋₁₈-alkyle, un groupe C₆₋₁₈-aryle ou un C₆₋₁₈-aryle, qui est substitué par C₁₋₁₈-alkyle ; et
R⁷³ et R⁷⁴ étant indépendamment l'un de l'autre H, ou un groupe phényle,
étant entendu qu'un parmi R¹, R² et R³ est un groupe de formule (X), ou (XI).

2. Composé selon la revendication 1, qui est un composé de formule 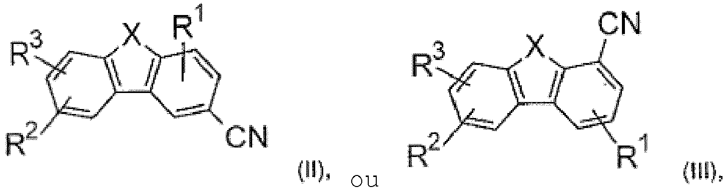 X, R¹, R² et R³ étant définis dans la revendication 1.

3. Composé selon la revendication 1, qui est un composé de formule 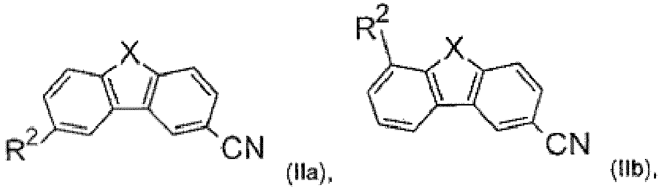 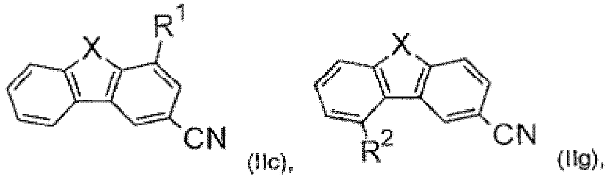 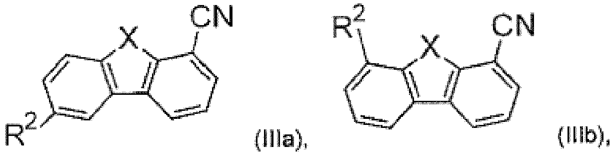 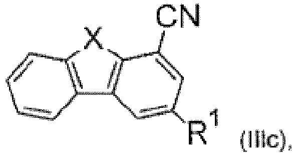 ou 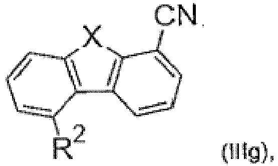 X, R¹, R² et R³ étant définis dans la revendication 1.

4. Composé selon l'une quelconque des revendications 1 à 3,
R¹ étant H, un groupe de formule (X), ou (XI),
R² étant H, un groupe de formule (X), ou (XI), et
R³ étant H, un groupe de formule (X), ou (XI), le groupe de formule (X), ou (XI) étant défini dans la revendication 1.

5. Composé selon l'une quelconque des revendications 1 à 4, le groupe de formule (X) étant un groupe de formule 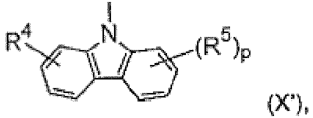
p étant 0, 1 ou 2,
R⁴ et R⁵ étant indépendamment l'un de l'autre H, CN un groupe C₁₋₂₅-alkyle, un groupe de formule 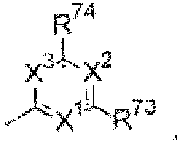
X¹, X² et X³ étant indépendamment l'un de l'autre N, ou CH, et
R⁷³ et R⁷⁴ étant indépendamment l'un de l'autre H, ou un groupe phényle.

6. Composé selon la revendication 5, le groupe de formule (X) étant un groupe de formule 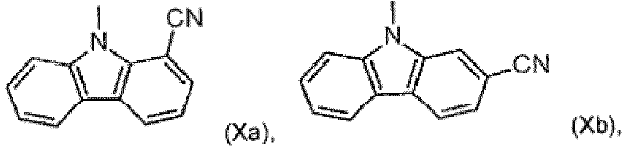 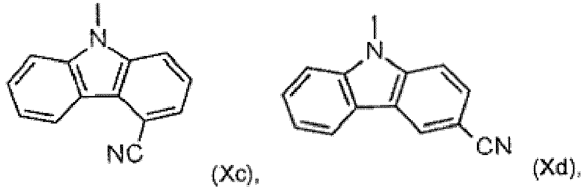 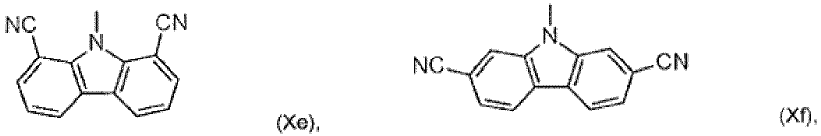 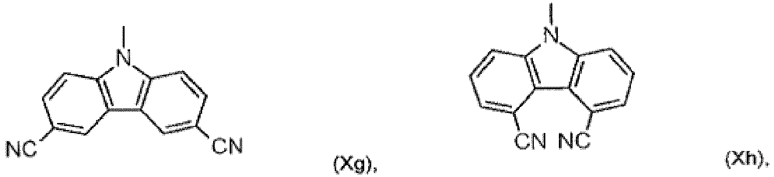 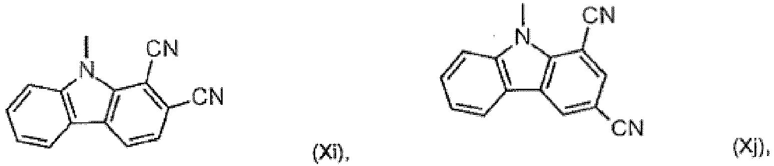 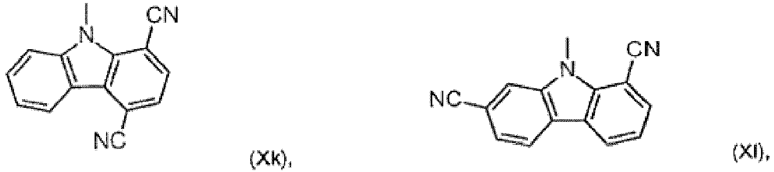 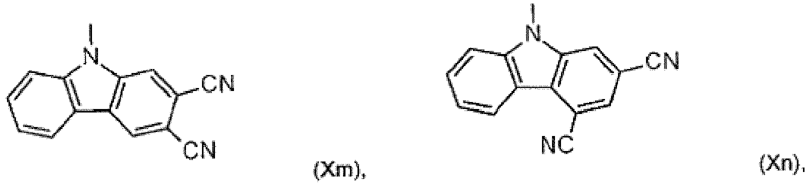 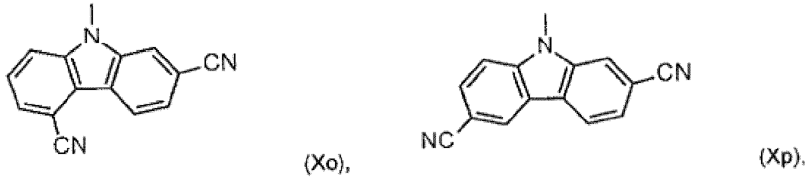 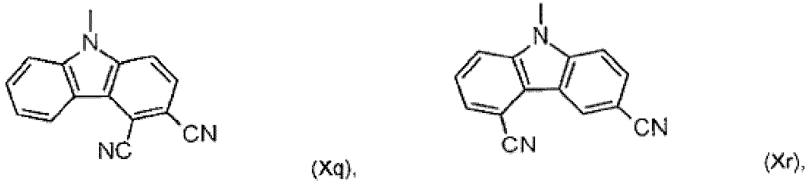 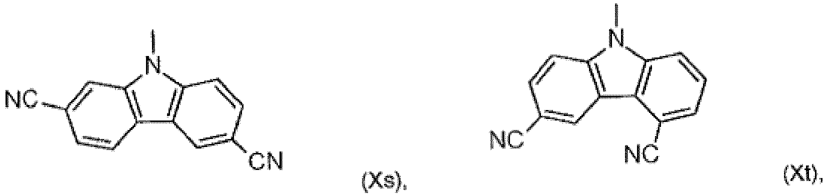 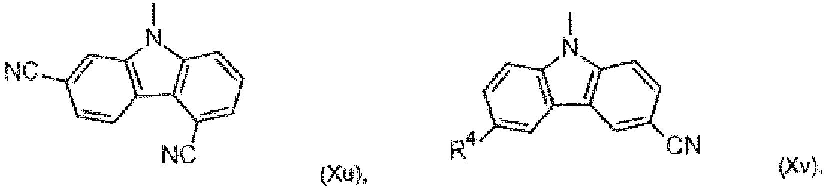 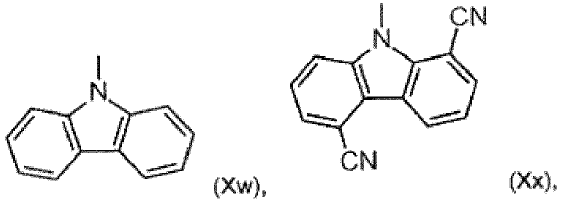 ou 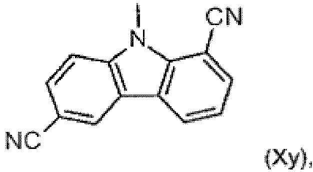 R⁴ étant un groupe C₁₋₂₅-alkyle.

7. Composé selon l'une quelconque des revendications 1 à 4, le groupe de formule (XI) étant un groupe de formule 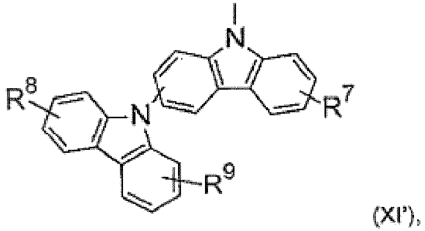
R⁷ étant H, CN, ou un groupe C₁₋₂₅-alkyle ;
R⁸ étant H, CN, ou un groupe C₁₋₂₅-alkyle ; et
R⁹ étant H, CN, ou un groupe C₁₋₂₅-alkyle.

8. Composé selon la revendication 7, le groupe de formule (XI) étant un groupe de formule 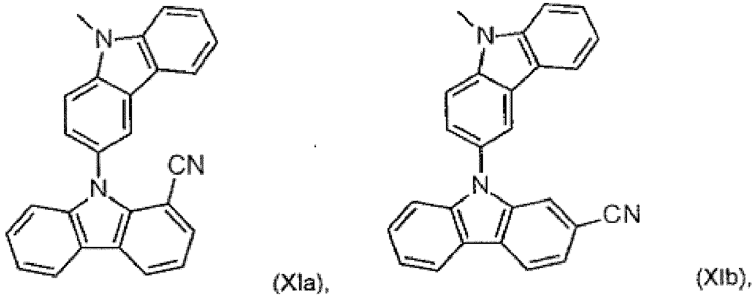 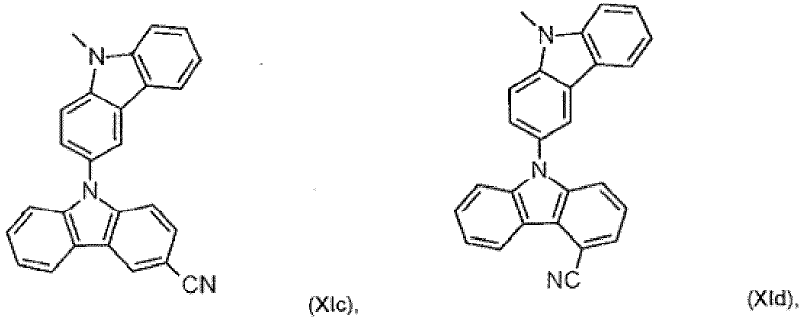 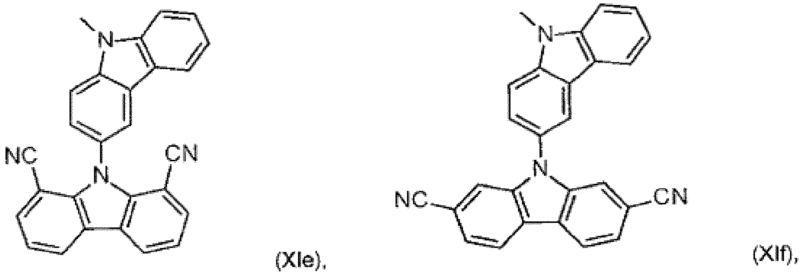 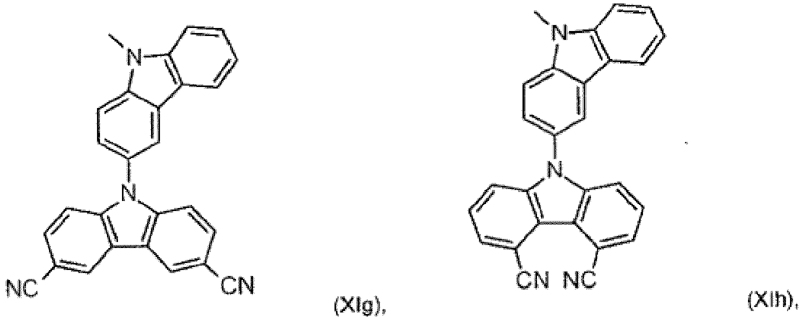 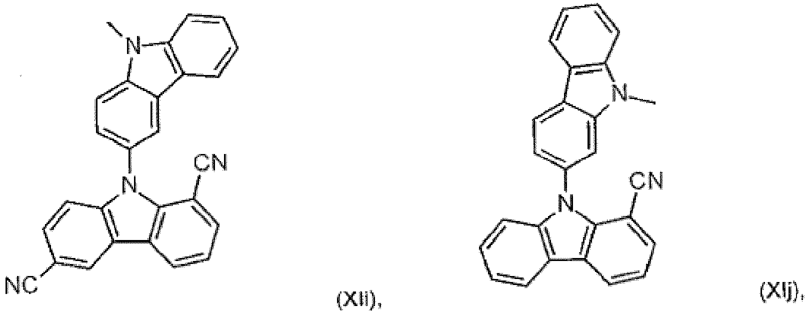 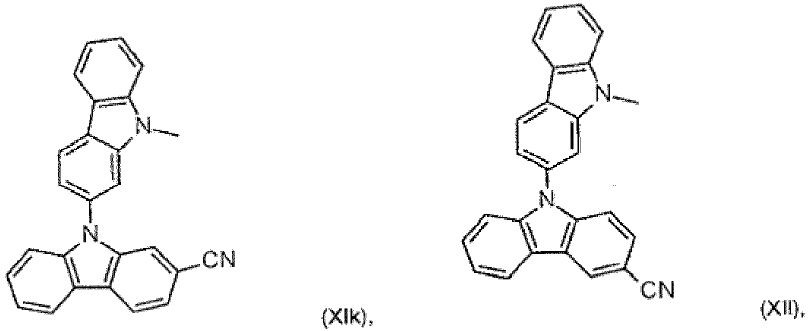 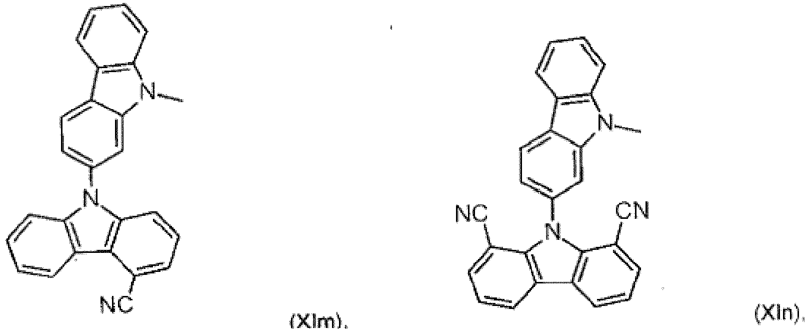 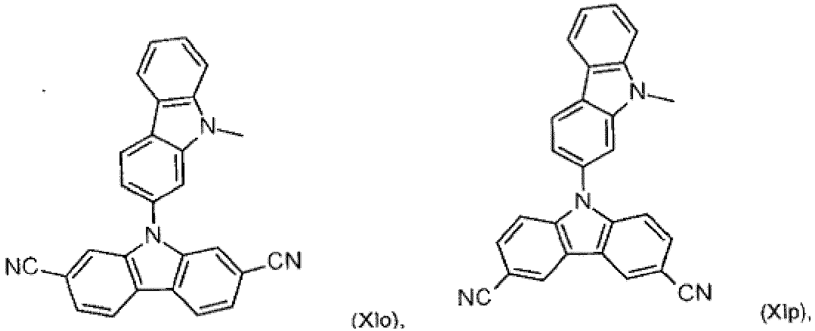 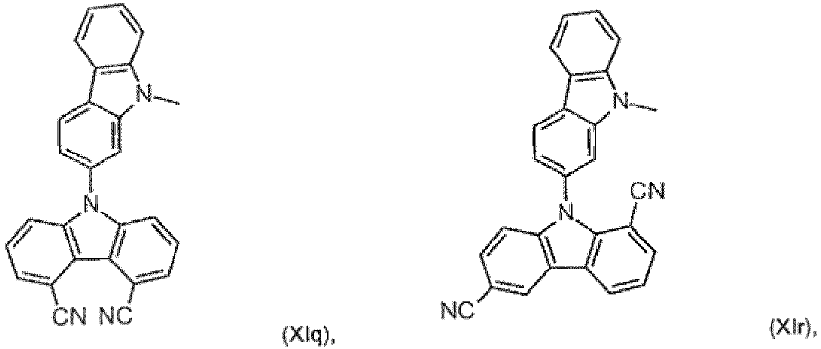 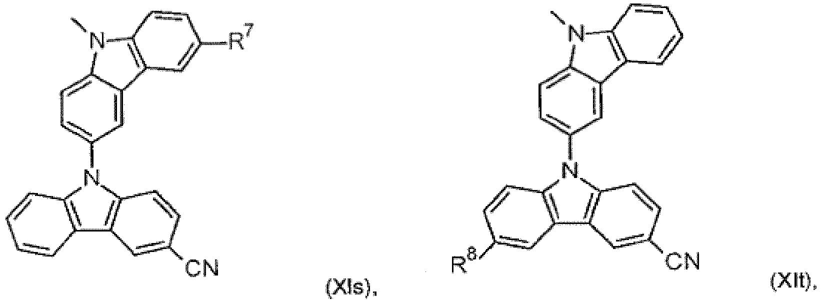 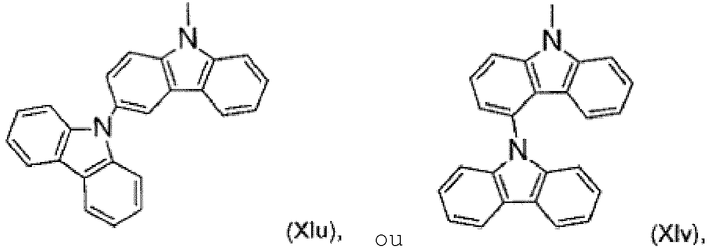 R⁷ étant un groupe C₁₋₂₅-alkyle et R⁸ étant un groupe C₁₋₂₅-alkyle.

9. Composé selon l'une quelconque des revendications 1 à 8, X étant O.

10. Composé selon l'une quelconque des revendications 1 à 9, qui est un composé de formule 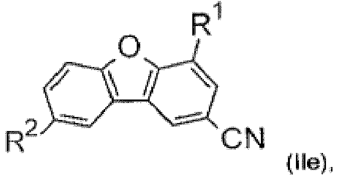
R¹ étant H, et R² étant un groupe de formule (Xa), (Xb), (Xc), (Xd), (Xf), (Xg), (Xv) ou (Xw) tel que défini dans la revendication 7 ; ou un groupe de formule (XIa), (XIb), (XIc), (XId), (XIf), (XIg), (XIs), (XIt), (XIu) ou (XIv) tel que défini dans la revendication 9 ; ou
R² étant H, et R¹ étant un groupe de formule (Xa), (Xb), (Xc), (Xd), (Xf), (Xg), (Xv) ou (Xw) tel que défini dans la revendication 7 ; ou un groupe de formule (XIa), (XIb), (XIc), (XId), (XIf), (XIg), (XIs), (XIt), (XIu) ou (XIv) tel que défini dans la revendication 9.

11. Dispositif électronique, comprenant un composé selon l'une quelconque des revendications 1 à 10.

12. Dispositif électronique selon la revendication 11, qui est un dispositif électroluminescent.

13. Couche de transport d'électrons, couche de blocage de trous, ou couche émettrice comprenant un composé selon l'une quelconque des revendications 1 à 10.

14. Couche émettrice selon la revendication 13, comprenant un composé selon l'une quelconque des revendications 1 à 11 en tant que matériau hôte en combinaison avec un émetteur phosphorescent.

15. Appareil choisi dans le groupe constitué par des unités stationnaires d'affichage visuel ; des unités mobiles d'affichage visuel ; des unités d'éclairage ; des claviers ; des articles d'habillement ; du mobilier ; de la tapisserie, comprenant le dispositif électronique selon la revendication 11, ou 12, ou la couche de transport d'électrons, la couche de blocage de trous et/ou la couche émettrice selon la revendication 13.

16. Utilisation des composés de formule I selon l'une quelconque des revendications 1 à 10 pour des photorécepteurs électrophotographiques, des convertisseurs photoélectriques, des cellules solaires organiques, des éléments de commutation, des transistors organiques électroluminescents à effet de champ, des capteurs d'image, des lasers à colorant, des dispositifs électroluminescents, en tant que matériau de transport dans une couche de blocage de trous et/ou une couche émissive et/ou en tant que couche de blocage d'électrons.
